# EUROPEAN PATENT APPLICATION

(11) **EP 4 339 210 A1**
(43) Date of publication of application: **20.03.2024**
(21) Application number: 22807907.5
(22) Date of filing: 13.05.2022
(51) Int. Cl.: C07K 16/28, A61P 35/00, G01N 33/574, G01N 33/68, A61K 45/06, A61K 39/00

(54) **COMBINED THERAPY USING ANTI-CD300C ANTIBODY**

(30) Priority: 13.05.2021 KR 20210062311; 13.05.2021 KR 20210062312; 13.05.2021 KR 20210062313; 27.08.2021 KR 20210114297; 06.04.2022 KR 20220042680
(71) Applicant: CentricsBio, Inc., Seoul 05836 (KR)
(72) Inventor: JEON, Jae-Won, Suwon-si, Gyeonggi-do 16512 (KR); LEE, Suin, Gunpo-si, Gyeonggi-do 15801 (KR); KIM, Haneul, Namyangju-si, Gyeonggi-do 12221 (KR); LIM, Chang Ki, Hwaseong-si, Gyeonggi-do 18496 (KR)
(74) Representative: Regimbeau
(86) International application number: PCT/KR2022/006938
(87) International publication number: WO 2022/240260

(57) **Abstract**

Disclosed are an anti-CD300c antibody and a combined therapy thereof and, more specifically, a pharmaceutical composition, a kit, and a method for the prevention or treatment of cancer, each of which comprises, as active ingredients, an anti-CD300c monoclonal antibody and at least one additional anticancer agent.

## Description

### [Technical Field]

The present disclosure relates to an anti-CD300c antibody and a combined therapy using an anti-CD300c antibody. More specifically, the present disclosure relates to a composition, a kit, and a combined therapy for the prevention or treatment of cancer, each of which includes, as active ingredients, an anti-CD300c monoclonal antibody and at least one additional anticancer agent.

### [Background Art]

In recent years, immunotherapies that activate the immune system of the human body to help fight cancer cells are receiving attention as cancer treatments. Previous cancer therapeutic agents have focused on killing rapidly dividing cells, which is the characteristic of cancer cells, so that there are side effects of acting on not only cancer cells but also rapidly dividing cells among normal cells. However, it is known that immunotherapeutic agents use the immune system of a cancer patient to affect cancer cells, so there are few typical side effects of existing anticancer agents. In addition, immunotherapeutic agents utilize the immune system of the body and thus can be applied to a variety of cancer types, unlike targeted therapeutic agents targeting specific mutations or signals of cancer. An immune checkpoint inhibitor such as Keytruda^{®} is receiving attention as a type of immunotherapeutic agent.

Meanwhile, CD300 antigen-like family member C (CD300c) protein, which is a protein encoded by the CD300c gene in humans, exists on the surface of various cancer cells. The inhibition of activity or expression of the CD300c protein can activate T cells to reduce the proliferation of cancer (Korean Patent Publication No. 10-2019-0136949).

It is also known that many types of tumors often express multiple immune checkpoint proteins simultaneously. Therefore, despite the promising clinical activity of immune checkpoint inhibitors such as Keytruda^{®}, increasing therapeutic activity, reducing toxicity, or increasing therapeutic effects by both is still a key goal in the development of immunotherapeutic agents.

### [Prior Art Document]

### [Patent Document]

(Patent Document 1) Korean Patent Publication No. 10-2019-0136949

### [Disclosure]

### [Technical Problem]

An aspect of the present disclosure is to solve all of the above-mentioned problems.

Another aspect of the present disclosure is to provide a combined therapy using an anti-CD300c antibody for preventing or treating cancer.

Still another aspect of the present disclosure is to provide a pharmaceutical composition for a combined therapy using an anti-CD300c antibody for preventing or treating cancer.

Still another aspect of the present disclosure is to provide a kit used in a combined therapy using an anti-CD300c antibody for preventing or treating cancer.

Still another aspect of the present disclosure is to provide a diagnostic or treatment method based on a change in marker protein according to the expression level of an anti-CD300c antibody or the administration of an anti-CD300c antibody.

The aspect of the present disclosure is not limited to ones as mentioned above. The aspect of the present disclosure will become clearer from the following description, and will be realized by the means as described in the claims and combinations thereof.

### [Technical Solution]

Representative features of the present disclosure for achieving the above-mentioned objects are as follows.

In accordance with an aspect of the present disclosure, there are provided an anti-CD300c antibody or antigen-binding fragment thereof and use of the anti-CD300c antibody or antigen-binding fragment thereof for preventing or treating cancer.

In accordance with another aspect of the present disclosure, there is provided a combined use of an anti-CD300c antibody or antigen-binding fragment thereof for the prevention or treatment of cancer and at least one additional anticancer agent.

In accordance with still another aspect of the present disclosure, there is provided a pharmaceutical composition for the prevention or treatment of cancer, the pharmaceutical composition containing, as active ingredients, an anti-CD300c antibody or antigen-binding fragment thereof and at least one additional anticancer agent
In accordance with still another aspect of the present disclosure, there is provided a method for preventing or treating cancer, the method including administering, to a subject in need of the prevention or treatment of cancer, an anti-CD300c (CD300 antigen-like family member C) antibody or antigen-binding fragment thereof and the at least one additional anticancer agent.

In accordance with still another aspect of the present disclosure, there is provided a kit for preventing or treating cancer, the kit including: a composition containing an anti-CD300c antibody or antigen-binding fragment thereof at an effective amount; and instructions directing the combined use of the antibody or antigen-binding fragment thereof and at least one additional anticancer agent.

In accordance with still another aspect of the present disclosure, there is provided a method for providing information needed for the prediction of the therapeutic responsiveness of an anti-CD300c antibody or antigen-binding fragment thereof, the method including determining the expression level of a marker for predicting the therapeutic responsiveness on the basis of a biological sample or data obtained from a subject.

In accordance with still another aspect of the present disclosure, there is provided a method for providing information for the prevention or treatment of cancer, the method including measuring the expression level of a CD300c protein on the basis of a biological sample or data obtained from a subject in need of the prevention or treatment of cancer.

### [Advantageous Effects]

It was confirmed both *in vivo* and *in vitro* that the combined therapy of an anti-CD300c antibody according to the present disclosure and an additional anticancer agent can inhibit the growth, proliferation, metastasis, and the like of cancer cells effectively and synergistically compared with the use of the anti-CD300c antibody or the additional anticancer agent alone. Therefore, the combined use of an anti-CD300c antibody and an additional anticancer agent can be advantageously employed in the treatment or prevention of various types of cancers.

It was also revealed that there is a significant relevance between the expression level of a CD300c protein and the survival period in various cancer patients, indicating that the expression level of the CD300c protein can be used to predict the prognosis of cancer patients or the possibility of treatment using anti-CD300c antibodies. Furthermore, it was confirmed that marker proteins with respect to immune checkpoint proteins, immune cell activation factors, and tumor microenvironments were changed after the administration of anti-CD300c antibodies, indicating that these marker proteins can be utilized to identify the therapeutic responsiveness of a patient to anti-CD300c antibodies or effectively administer anti-CD300c antibodies in combination with other anticancer agents.

### [Brief Description of Drawings]

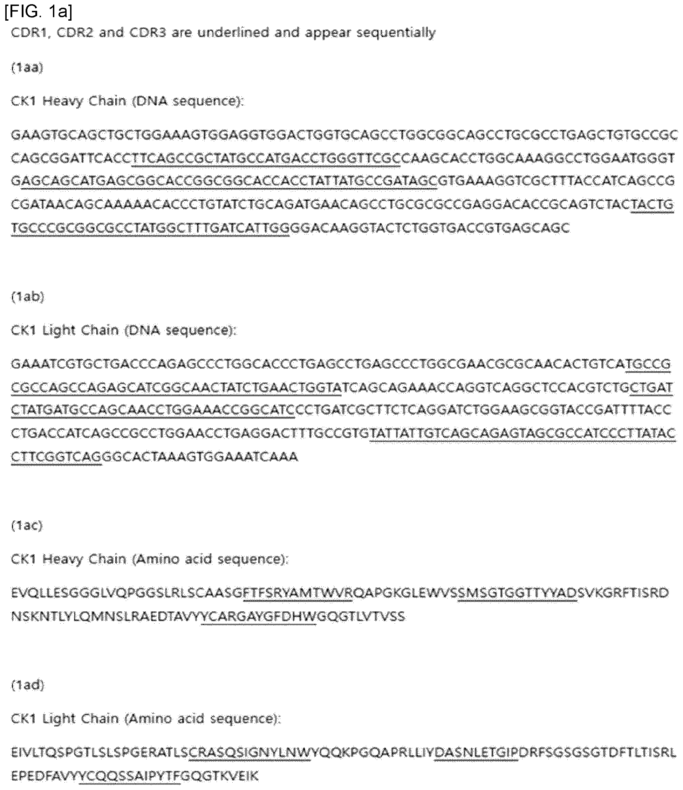

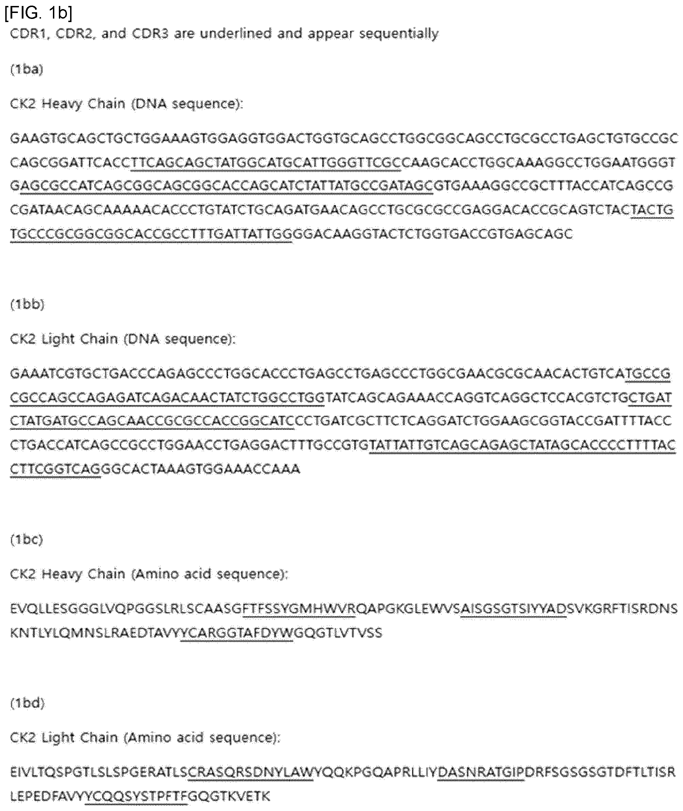

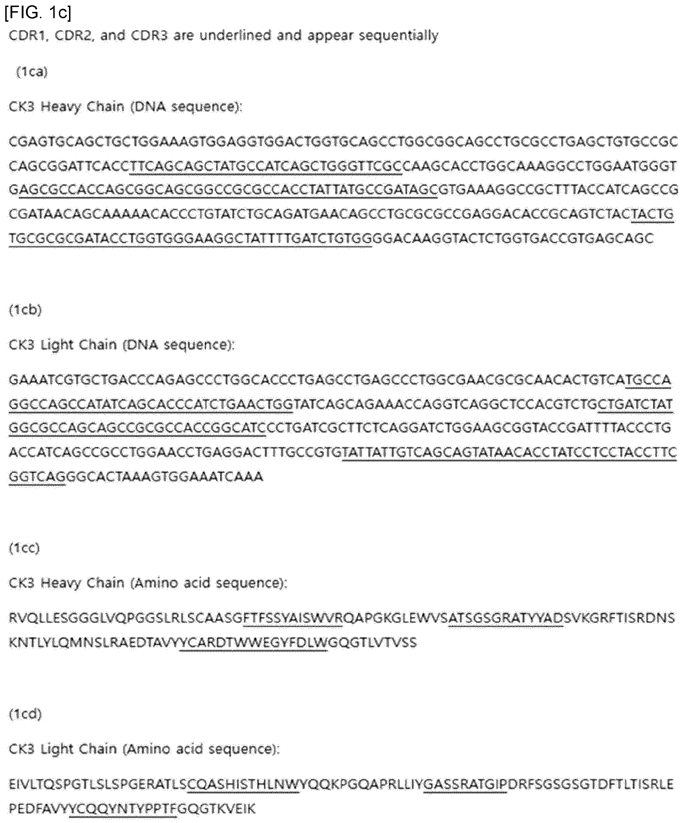

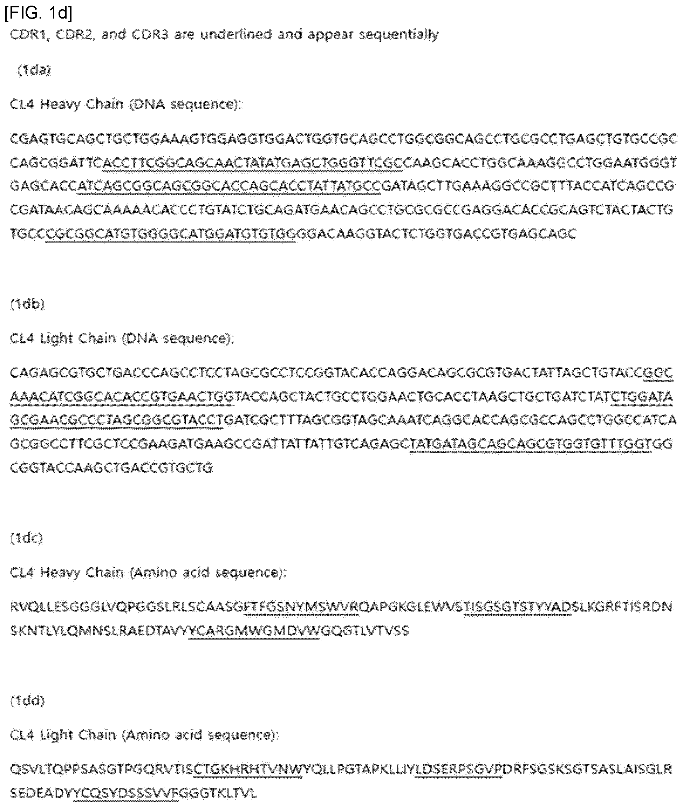

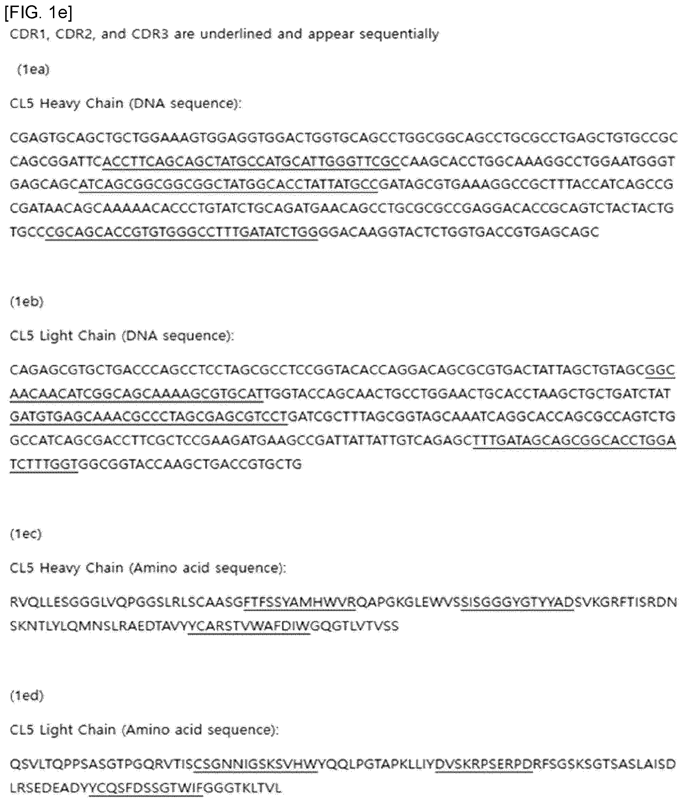

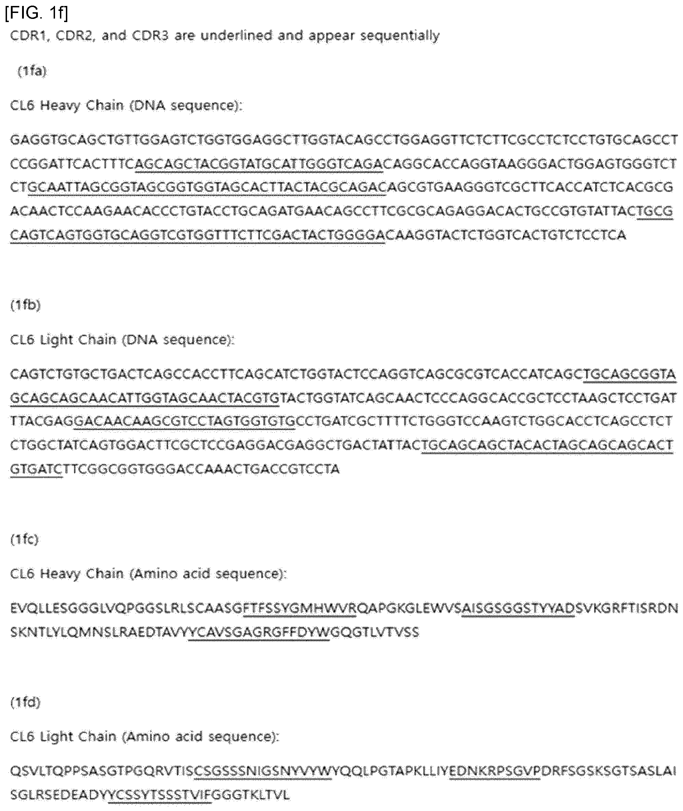

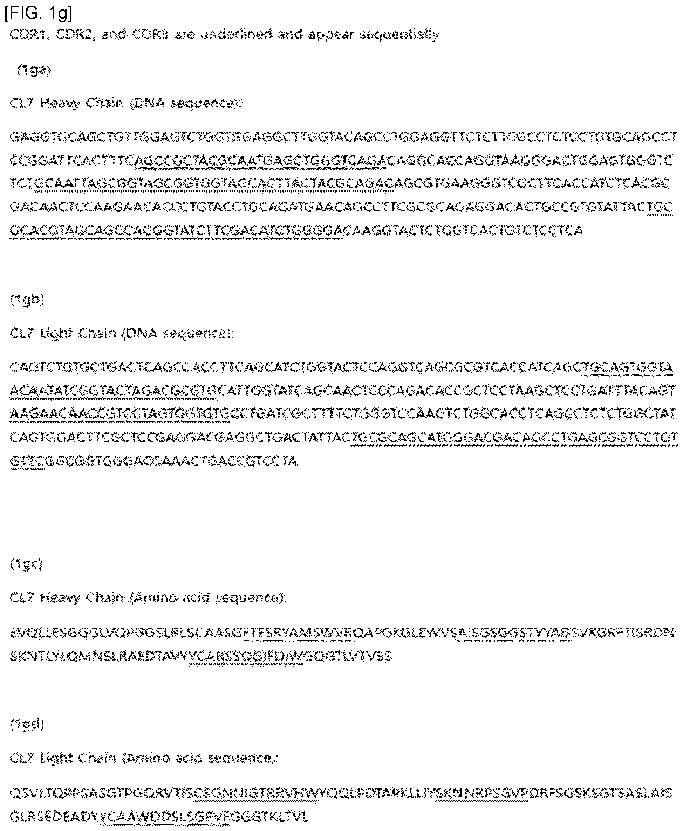

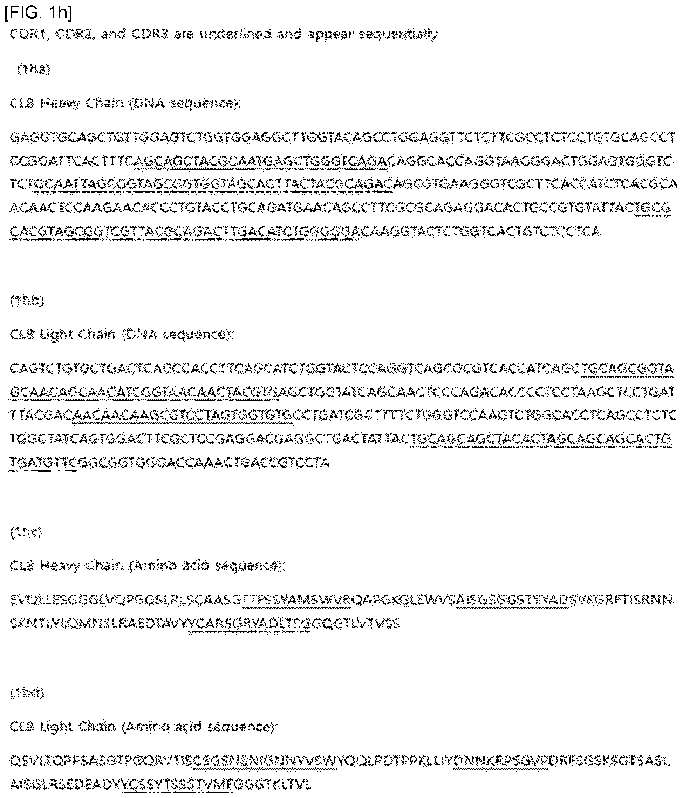

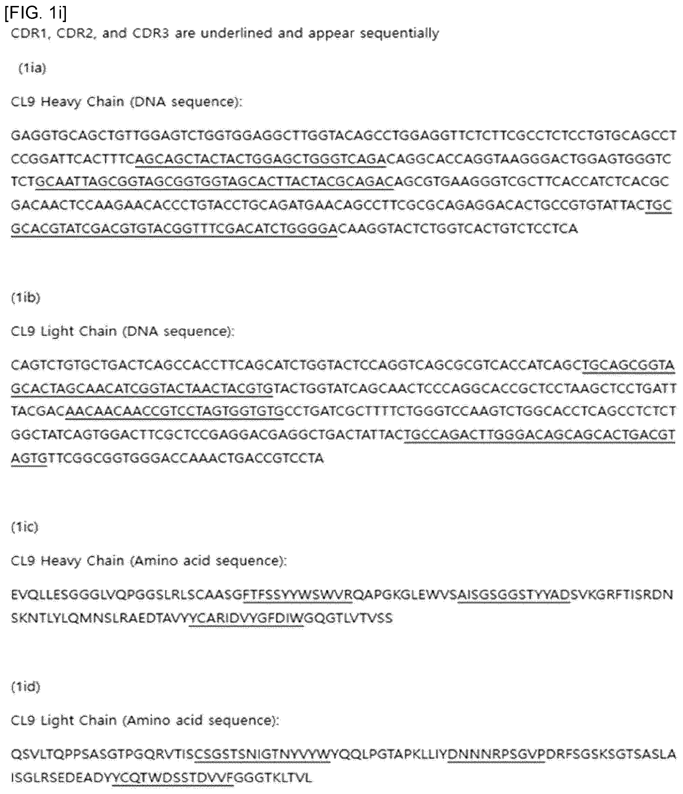

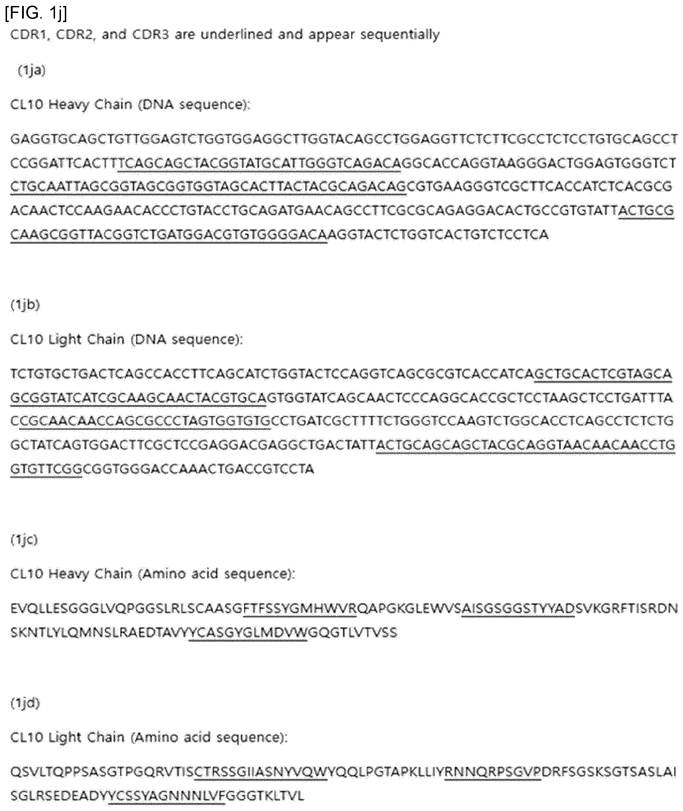

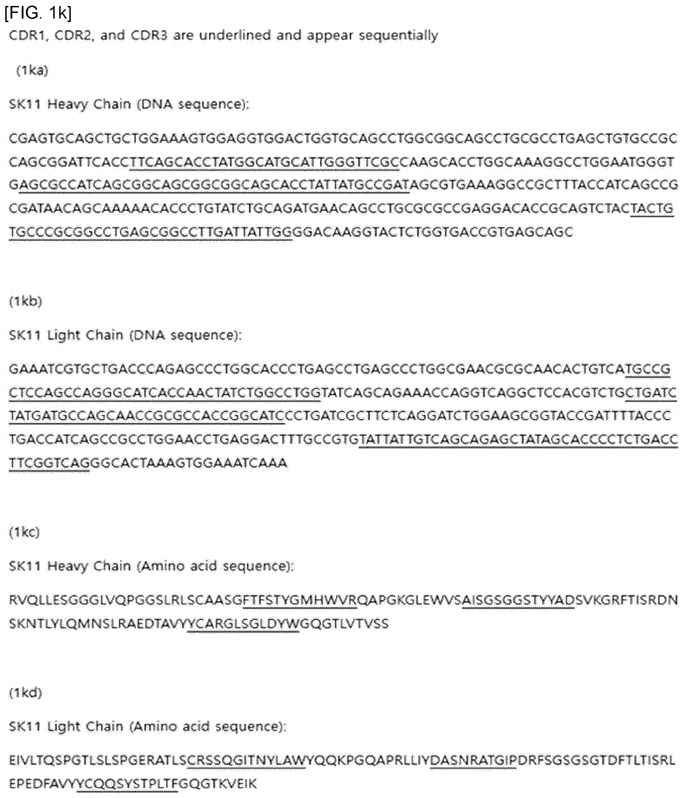

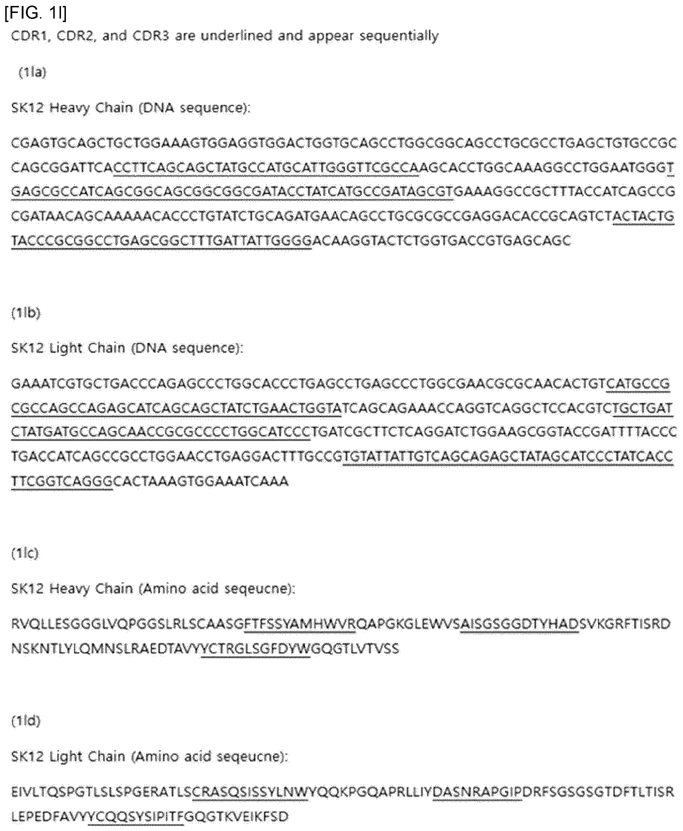

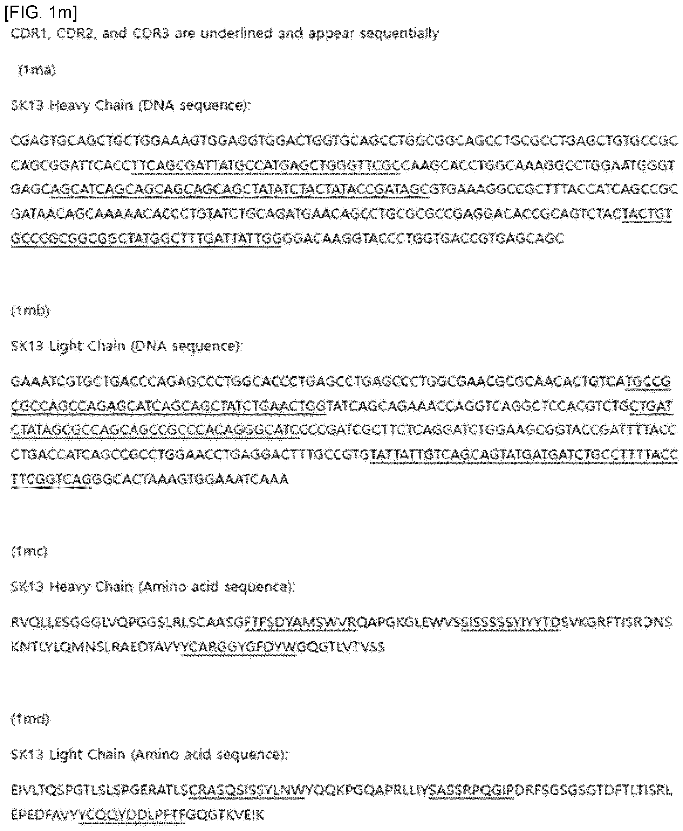

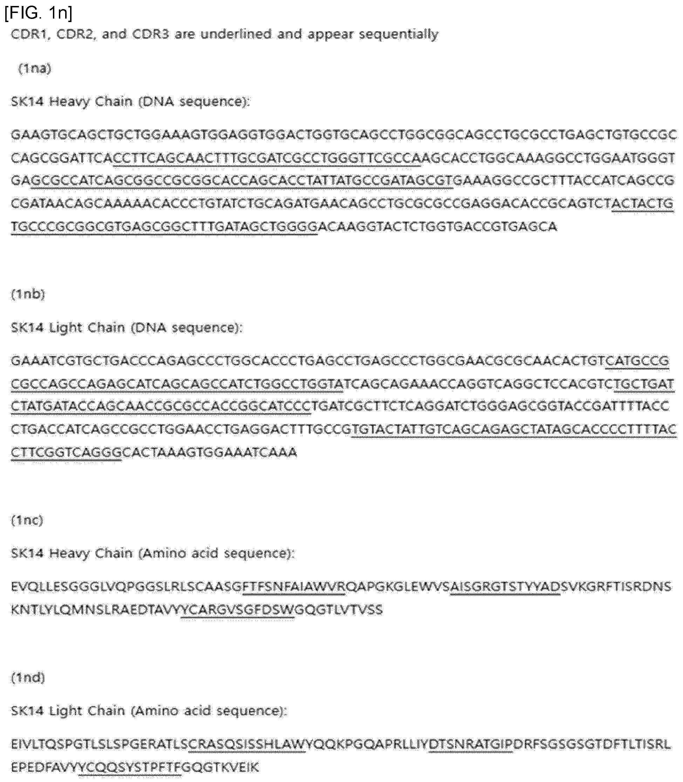

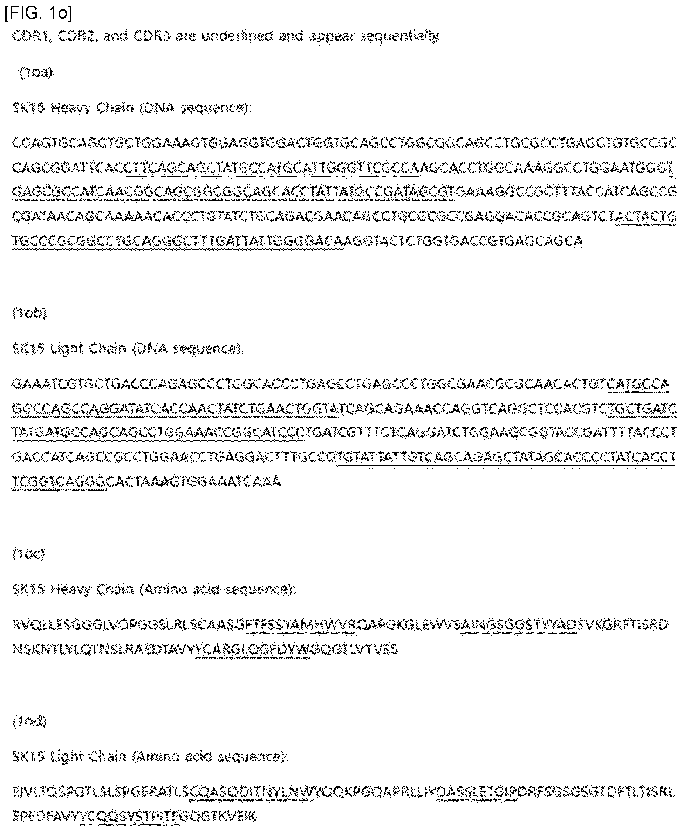

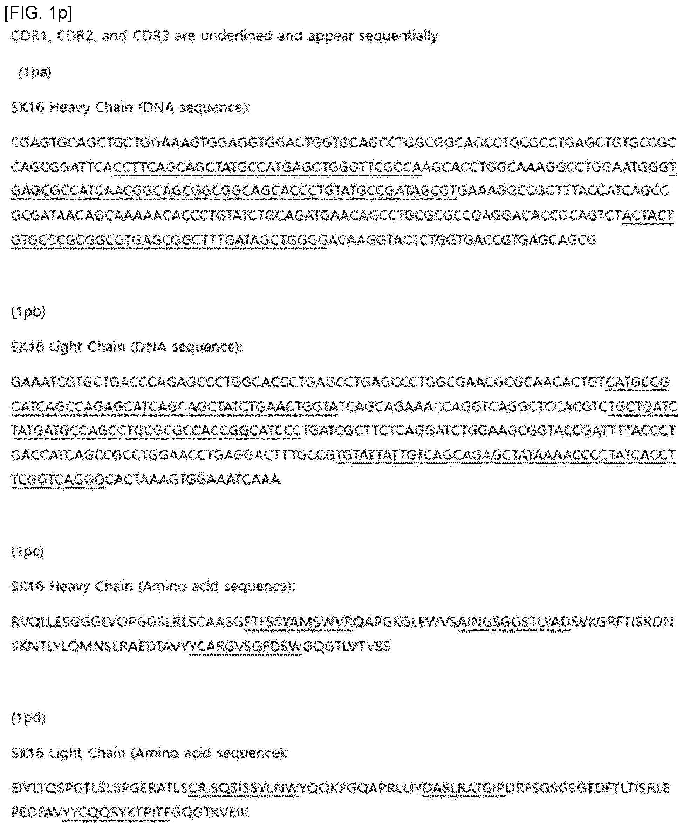

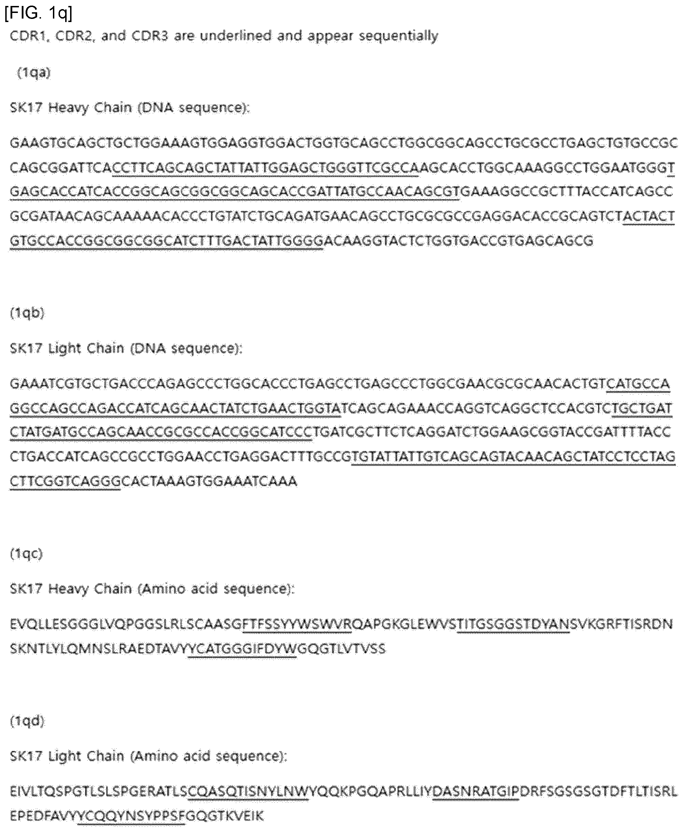

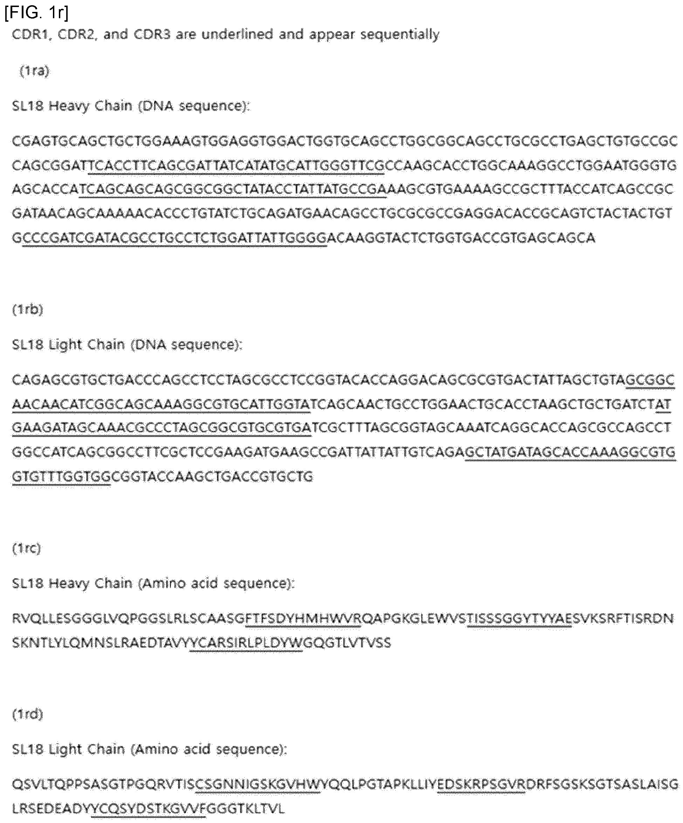

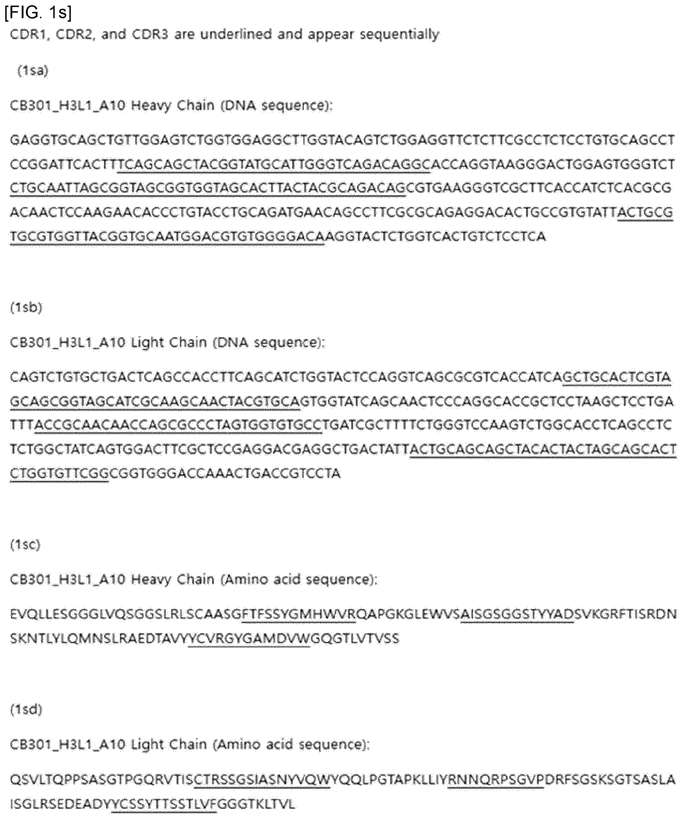

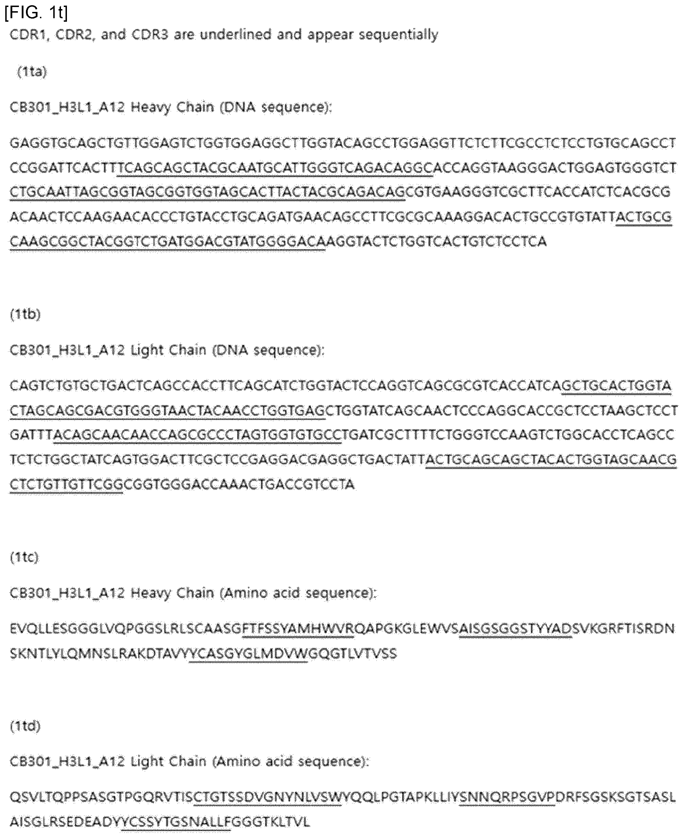

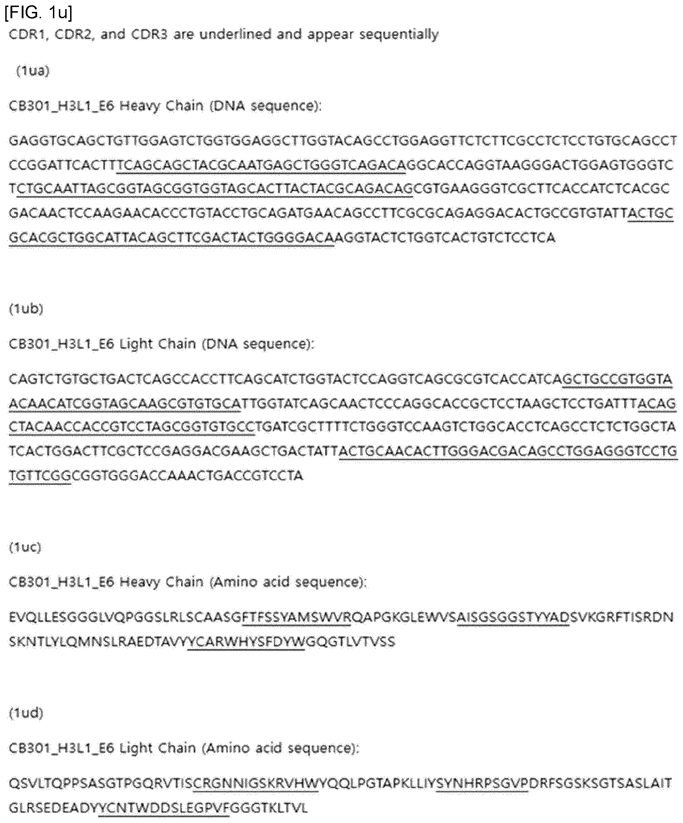

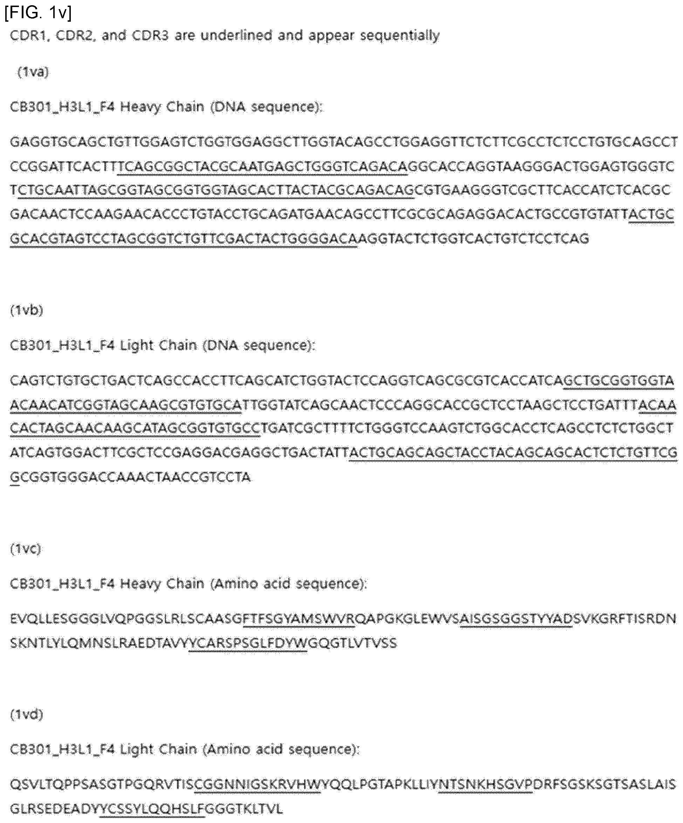

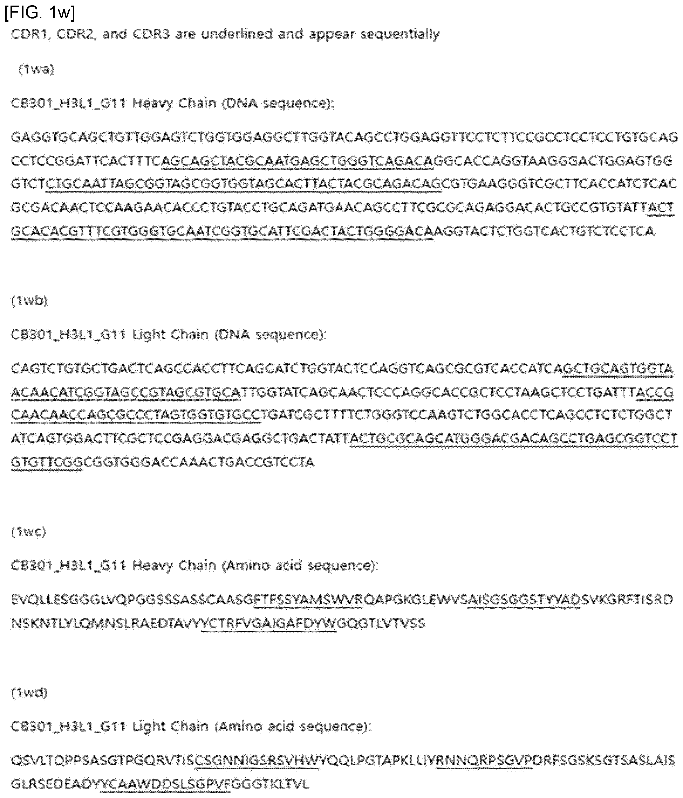

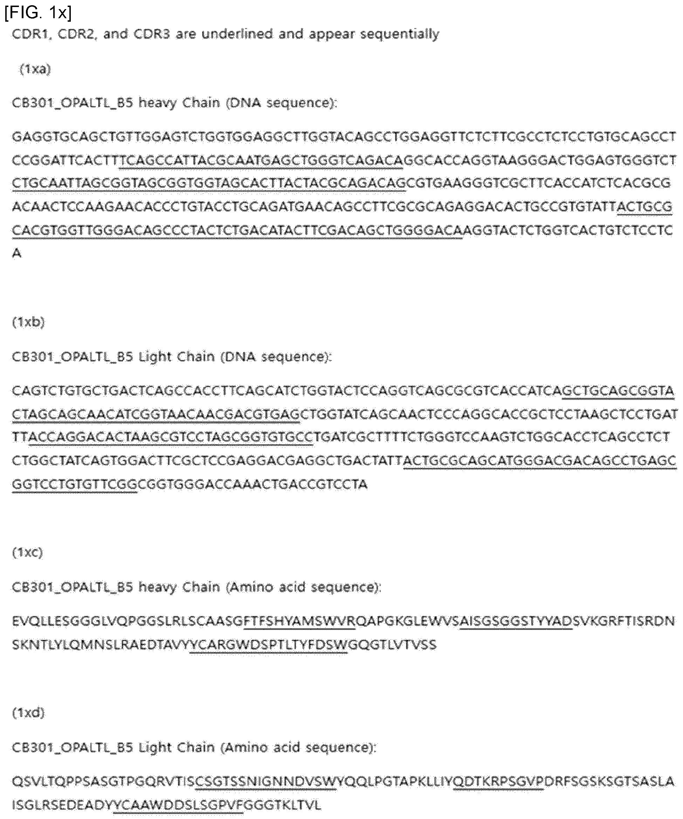

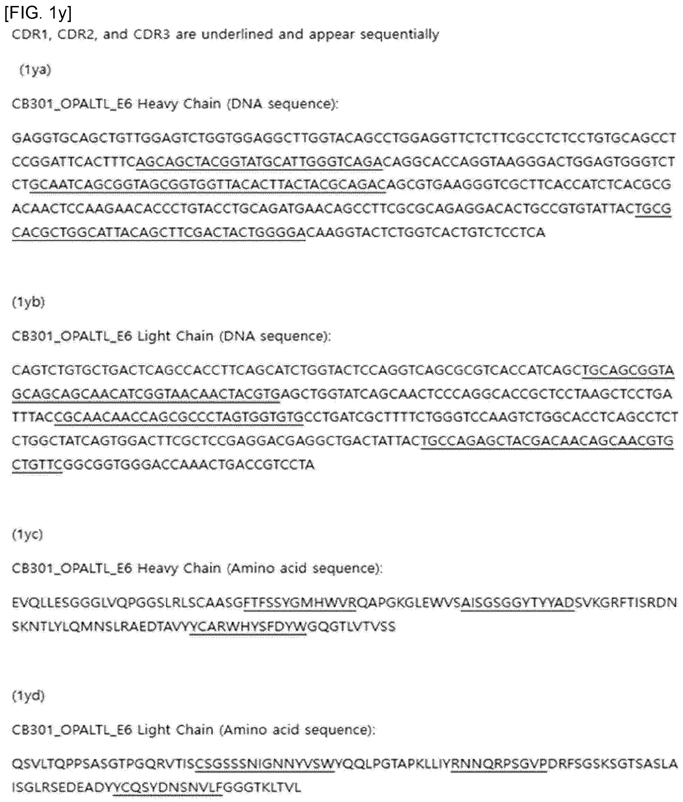

FIGS. 1a to 1y illustrate heavy chain and light chain variable region sequences (nucleic acid and amino acid sequences) of 25 types of anti-CD300c monoclonal antibodies, respectively, according to the present disclosure. In each drawing, the CDR regions (CDR1, CDR2, and CDR3) are sequentially indicated.
FIG. 2 illustrates the results obtained by performing SDS-PAGE on anti-CD300c monoclonal antibodies under a non-reducing condition, according to Example 1.4.
FIG. 3 illustrates the results obtained by performing SDS-PAGE on anti-CD300c monoclonal antibodies under a reducing condition, according to Example 1.4.
FIG. 4 illustrates the results obtained by identifying the expression of CD300c in normal cells, immune cells, and a cancer cell line, according to Experimental Example 1.1.
FIGS. 5a and 5b illustrate the results obtained by identifying the expression of CD300c in cancer tissue (FIG. 5a) and immune cells (FIG. 5b), according to Experimental Example 1.2.
FIGS. 6a and 6b illustrate the results obtained by identifying the expression of CD300c in tonsil tissue (FIG. 6a) and cancer tissue (FIG. 6b), according to Experimental Example 1.3.
FIG. 7 illustrates the results obtained by identifying the binding affinity, to a CD300c antigen, of an anti-CD300c monoclonal antibody, according to Experimental Example 2.1.
FIG. 8 illustrates sigmoidal curves according to the results of FACS binding in Experimental Example 2.2.
FIG. 9 illustrates the results of the binding ELISA, according to Experimental Example 2.3.
FIG. 10 illustrates the results of the surface plasmon resonance, according to Experimental Example 2.4.
FIG. 11 illustrates the results of the binding ELISA, according to Experimental Example 2.5.
FIG. 12 illustrates the results of the binding ELISA, according to Experimental Example 2.6.
FIG. 13 illustrates the results obtained by identifying whether an anti-CD300c monoclonal antibody promotes the differentiation into M1 macrophages in mouse macrophages, according to Experimental Example 3.1.
FIG. 14 illustrate the results obtained by identifying the effect of an anti-CD300c monoclonal antibody on tumor-associated macrophages, according to Experimental Example 3.2.
FIG. 15 illustrates the results obtained by identifying *in vivo* effects of an anti-CD300c monoclonal antibody on CD8+ T cells, according to Experimental Example 3.3.
FIG. 16 illustrates the results obtained by identifying whether an anti-CD300c monoclonal antibody increases the number of CD8+ T cells in a tumor-specific manner, according to Experimental Example 3.4.
FIG. 17 illustrates the results obtained by identifying *in vivo* effects of an anti-CD300c monoclonal antibody on an increase in activity of CD8+ T cells, according to Experimental Example 3.5.
FIG. 18 illustrates the results obtained by identifying *in vivo* effects of an anti-CD300c monoclonal antibody on an increase of cytotoxic T cells relative to regulatory T cells, according to Experimental Example 3.6.
FIG. 19 illustrates the results obtained by identifying effects of an anti-CD300c monoclonal antibody on cytotoxic T cells, regulatory T cells, and tumor-associated macrophages, according to Experimental Example 3.7.
FIG. 20 illustrates the results obtained by identifying whether an anti-CD300c monoclonal antibody exhibits anticancer effects, according to Experimental Example 3.8.
FIG. 21 illustrates the results obtained by identifying anticancer effects of an anti-CD300c monoclonal antibody under *in vivo* conditions, according to Experimental Example 3.9.
FIG. 22 illustrates the results obtained by identifying the overall survival periods depending on the expression level of CD300c in various cancer patients, according to Experimental Example 4.
FIG. 23 illustrates the Nanostring Immune profiling results obtained when a solid cancer model was treated with CL7, according to Example 2.1.
FIG. 24 illustrates changes in expression of various immune cell- and tumor microenvironment-related markers obtained when a solid cancer model was treated with CL7, according to Example 2.1. The symbol * indicates a marker whose expression level was statistically significantly changed compared with before treatment with CL7.
FIG. 25 illustrates changes in expression of immune checkpoint markers identified on the basis of the Nanostring Immune profiling results obtained in Example 2.1, according to Example 2.2. The symbol * indicates a marker whose expression level was statistically significantly changed compared with before treatment with CL7.
FIG. 26 illustrates the results of differentiation of monocytes into M1 macrophages (whether or not M1 macrophages are increased) by treatment with an anti-CD300c monoclonal antibody and the immunotherapeutic agent alone or in combination, according to Experimental Example 5.1.
FIG. 27 illustrates the results of differentiation of monocytes into M1 macrophages (which indicate whether or not M1 macrophage markers increase) by treatment with an anti-CD300c monoclonal antibody, according to Experimental Example 5.2.
FIG. 28 illustrates the results of differentiation of monocytes into M1 macrophages (which indicates whether or not M1 macrophage markers increase) by combined treatment with an anti-CD300c monoclonal antibody and an immunotherapeutic agent, according to Experimental Example 5.2.
FIGS. 29 to 31 illustrate the results obtained by identifying the signaling of MAPK (FIG. 29), NF-kB (FIG. 30), and IkB (FIG. 31), which are signals of M1 macrophage differentiation, by combined treatment with an anti-CD300c monoclonal antibody and an immunotherapeutic agent, according to Experimental Example 5.4.
FIG. 32 illustrates the results obtained by identifying changes in apoptosis signal by combined treatment with an anti-CD300c monoclonal antibody and an immunotherapeutic agent, according to Experimental Example 6.1.
FIGS. 33 and 34 illustrate the results obtained by identifying cancer cell growth inhibitory effects by combined treatment with an anti-CD300c monoclonal antibody and an immunotherapeutic agent, according to Experimental Example 6.2.
FIG. 35 schematically illustrates the experimental method used in Experimental Example 7.1.
FIG. 36 illustrates the *in vivo* cancer growth inhibitory effects observed when mice transplanted with a colon cancer cell line were administered an anti-CD300c monoclonal antibody and an anti-PD-1 antibody alone or in combination, according to Experimental Example 7.1.
FIG. 37 illustrates the results obtained by identifying whether an anti-CD300c monoclonal antibody increases M1 macrophages in cancer tissues of a mouse model, according to Experimental Example 7.3.
FIG. 38 illustrates the results obtained by identifying whether an anti-CD300c monoclonal antibody promotes CD8+ T cell immunity in a mouse tumor model, according to Experimental Example 7.4.
FIG. 39 schematically illustrates the experimental method used in Experimental Example 8.1.
FIG. 40 illustrates the results obtained by identifying whether an anti-CD300c monoclonal antibody is effective in other carcinomas in addition to a CT26 colon cancer mouse model, according to Experimental Example 8.1.
FIG. 41 illustrates the results obtained by identifying *in vivo* effects, of administration of an anti-CD300c monoclonal antibody and an immunotherapeutic agent alone or in combination (including double and triple combined administration), on CD8+ T cells in a B16F10 melanoma model, according to Experimental Example 8.2.
FIG. 42 illustrates the results obtained by identifying *in vivo* effects, of an anti-CD300c monoclonal antibody and an immunotherapeutic agent alone or in combination (including double and triple combined administration), on regulatory T cells in a B16F10 melanoma model, according to Experimental Example 8.3.
FIG. 43 illustrates the results obtained by identifying *in vivo* effects, of an anti-CD300c monoclonal antibody and an immunotherapeutic agent alone or in combination (including double and triple combined administration), on macrophages in a B16F10 melanoma model, according to Experimental Example 8.4.
FIGS. 44a and 44b illustrate the results obtained by identifying *in vivo* anticancer effects by combined administration of an anti-CD300c monoclonal antibody and an immunotherapeutic agent, according to Experimental Example 9. FIG. 44a illustrates tumor volume decrease rates, and FIG. 44b illustrates complete remission rates.
FIG. 45 illustrates the results obtained by identifying effects of improving the long-term survival rate by combined administration of an anti-CD300c monoclonal antibody and an immunotherapeutic agent, according to Experimental Example 10.
FIG. 46 illustrates the results obtained by identifying *in vivo* effects of preventing cancer recurrence by combined administration of an anti-CD300c monoclonal antibody and an immunotherapeutic agent, according to Experimental Example 11.
FIG. 47 illustrates the results obtained by identifying immune memory effects by combined administration of an anti-CD300c monoclonal antibody and an immunotherapeutic agent, according to Experimental Example 12.
FIGS. 48a and 48b illustrate the results obtained by identifying whether administration of an anti-CD300c monoclonal antibody and an immunotherapeutic agent alone or in combination (including double and triple combined administration) can promote differentiation from monocytes to M1 macrophages, according to Experimental Example 14.
FIGS. 49a and 49b illustrate the results obtained by identifying whether an anti-CD300c monoclonal antibody can inhibit cancer cell growth by combined administration with an immunotherapeutic agent, according to Experimental Example 15.
FIGS 50 to 52 illustrates graphs selecting optimal treatment concentrations of sonafenib (FIG. 50), gemcitabine (FIG. 51), and paclitaxel (FIG. 52) for combined treatment with an anti-CD300c monoclonal antibody, respectively.
FIGS 53 to 55 illustrates the results obtained by identifying the cancer cell growth inhibitory effects of sonafenib (FIG. 53), gemcitabine (FIG. 54), and paclitaxel (FIG. 55) used for combined treatment with an anti-CD300c monoclonal antibody, respectively.

### [Best Mode for Carrying Out the Invention]

The following detailed description of the present disclosure will be described, with reference to specific drawings, for specific embodiments in which the present disclosure may be practiced. However, the present disclosure is not limited thereto, and the scope of the present disclosure is defined only by the appended claims, appropriately interpreted, along with the full range of equivalents to which the claims are entitled. It is to be understood that the various embodiments of the present disclosure, although different from each other, are not necessarily mutually exclusive. For example, a particular feature, structure, or characteristic described herein may vary from one embodiment to another or be implemented as a combination of embodiments without departing from the spirit and scope of the present disclosure. Technical and scientific terms used herein have the same meanings as commonly used in the art to which the present disclosure belongs, unless otherwise defined. For the purpose of interpreting this specification, the following definitions will apply, and the singular forms "a," "an," and "the" include plural referents and *vice versa* unless the context clearly dictates otherwise.

### Definition

The term "about" as used herein refers to an acceptable error range for each value which is known to one of ordinary skill in the art.

The term "antibody" as used herein is used broadly and includes monoclonal antibodies (including full length antibodies) of any isotype such as IgG, IgM, IgA, IgD, and IgE, polyclonal antibodies, multispecific antibodies (for example, bispecific antibodies), antibody fusions (for example, a fusion of an antibody with a (poly)peptide or a fusion of an antibody with a compound), and antibody fragments (including antigen-binding fragments). As used herein, the prefix "anti-", when in conjunction with an antigen, indicates that the given antibody is reactive with the given antigen. An antibody reactive with a specific antigen can be generated, without limitation, by synthetic and/or recombinant methods such as selection of libraries of recombinant antibodies in phage or similar vectors, or by immunizing an animal with the antigen or an antigen-encoding nucleic acid. A typical IgG antibody is composed of two identical heavy chains and two identical light chains that are joined by disulfide bonds. Each heavy and light chain contains a constant region and a variable region. Heavy chain variable regions (HVRs) and light chain variable regions (LVRs) contain three segments, referred to as "complementarity determining regions" ("CDRs") or "hypervariable regions", respectively, which are primarily responsible for binding an epitope of an antigen. They are usually referred to as CDR1, CDR2, and CDR3, numbered sequentially from the N-terminus. The more highly conserved portions of the variable regions outside of the CDRs are called the "framework regions" ("FRs"). An antibody herein may be, for example, an animal antibody, a chimeric antibody, a humanized antibody, or a human antibody.

The term "humanization" (also called reshaping or CDR-grafting) includes a well-established technique for reducing the immunogenicity of monoclonal antibodies from xenogeneic sources (commonly rodent) and for improving their affinity or effector function (ADCC, complement activation, C1q binding).

The term "monoclonal antibody" as used herein refers to an antibody obtained from a population of substantially homogeneous antibodies, that is, the individual antibodies including the population are identical except for possible naturally occurring mutations and/or post-translation modifications (for example, isomerizations, amidations) that may be present in small amounts. Monoclonal antibodies are highly specific, being directed against a single antigenic site. The monoclonal antibody is obtained from a substantially homogeneous population of antibodies, displays the nature of an antibody, and is not to be construed as requiring production of the antibody by any particular method. For example, monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci.

The term "antigen-binding fragment" refers to a portion of an antibody having specific binding ability to an antigen or a polypeptide including the same. The terms "antibody" and "antigen-binding fragment" may be used interchangeably except for a case where it is understood in the context that the "antibody" specifically excludes the "antigen-binding fragment," and the "antibody" may be interpreted as including the "antigen-binding fragment." Examples of the antigen-binding fragment include, but are not limited to, Fv, Fab, Fab', Fab'-SH, F(ab')2, diabodies, triabodies, tetrabodies, cross-Fab fragments, linear antibodies, single chain antibody molecules (for example, scFv), and multispecific antibodies formed of antibody fragments and single domain antibodies.

The term "anticancer agent" collectively refers to known medications used in conventional cancer treatment that act on various metabolic pathways of cells and exhibit cytotoxic or cytostatic effects on cancer cells. The anticancer agent largely includes chemotherapeutic agents, targeted therapeutic agents, and immunotherapeutic agents.

The term "immunotherapeutic agent" refers to a medication that activates immune cells to kill cancer cells.

The term "chemotherapeutic agent" refers to a cancer therapeutic agent also called cytotoxic anticancer agent or chemical drug anticancer agent.

The term "subject" is used interchangeably with "patient" and may be a mammal who is in need of prevention or treatment of cancer, such as primates (for example, humans), companion animals (for example, dogs and cats), livestock (for example, cows, pigs, horses, sheep, and goats), and laboratory animals (for example, rats, mice, and guinea pigs). In an embodiment of the present disclosure, the subject is a human.

The term "treatment" generally means obtaining a desired pharmacological and/or physiological effect. The effect may be therapeutic in terms of partially or completely curing a disease and/or adverse effect attributed to the disease. Desirable therapeutic effects include, but are not limited to, prevention of onset or recurrence of disease, alleviation of symptoms, diminishment of any direct or indirect pathological consequences of the disease, prevention of metastasis, decreasing the rate of disease progression, amelioration or slowing of the disease state, and remission or improved prognosis. Preferably, the "treatment" may refer to medical intervention of a disease or disorder that has already developed.

The term "prevention" relates to a prophylactic treatment, that is, to a measure or procedure, the purpose of which is to prevent, rather than to cure a disease. "Prevention" means that a desired pharmacological and/or physiological effect is obtained which is prophylactic in terms of completely or partially preventing a disease or symptom thereof.

The term "administration" means a method of providing a substance (for example, an anti-CD300c antibody or antigen-binding fragment thereof or another anticancer agent) to a subject to achieve a prophylactic or therapeutic purpose (for example, prevention or treatment of cancer).

The term "biological sample" encompasses a variety of sample types obtained from a subject and may be used in diagnostic or monitoring assays. The biological sample includes, but is not limited to, blood and other liquid samples of biological origin, and solid tissue samples such as a biopsy specimen or tissue cultures or cells derived therefrom and the progeny thereof. Thus, the biological sample encompasses a clinical sample, and also includes cells in culture, cell supernatants, cell lysates, serum, plasma, biological fluid, and tissue samples, in particular, tumor samples. The term "biological data" refers to any analytical data obtained using the biological sample.

The term "expression level" may be determined by measuring the expression level of at least one of mRNA and protein of a corresponding marker. For the method for measuring the expression level of mRNA or protein, any method known in the art may be used. For example, an agent for measuring the expression level of mRNA may be a pair of primers or a probe specifically binding to the corresponding marker gene, and an agent for measuring the expression level of the protein may be an antibody, substrate, ligand, or cofactor specifically binding to the corresponding marker. An assay method for measuring the mRNA level includes, but is not limited to, reverse transcriptase polymerase chain reaction, competitive reverse transcriptase polymerase chain reaction, real-time reverse transcriptase polymerase chain reaction, RNase protection assay, Northern blotting, or DNA chip assay. An assay method for measuring the protein level includes, but is not limited to, Western blotting, ELISA, radioimmunoassay, radial immunodiffusion assay, Ouchterlony immunodiffusion, rocket immunoelectrophoresis, immunohistochemical staining, immunoprecipitation assay, complement fixation assay, FACS, and protein chip assay.

The term "therapeutic responsiveness" refers to whether an individual suffering from or suspected of having cancer responds favorably or unfavorably to treatment with a therapeutically active ingredient (such as a CD300c antibody or antigen-binding fragment thereof). The therapeutic responsiveness may be assessed by changes in immune system associated with tumor treatment which are observed after administration of the CD300c antibody or antigen-binding fragment thereof.

### Anti-CD300c Antibody

The anti-CD300c antibody or antigen-binding fragment thereof according to the present disclosure is an antigen-binding molecule specifically binding to a CD300c protein.

The term "CD300c protein" is used interchangeably with "CD300c" or "CD300c antigen" and is a protein encoded by CD300c gene. It is known that the CD300c protein exhibits significant sequence identity to B7 family proteins and is expressed on the membrane of antigen presenting cells. Inhibition of expression or activity of the CD300c protein may induce activation of T cells and/or promotion of differentiation into M1 macrophages.

The term "anti-CD300c antibody" may be used interchangeably with a polypeptide that binds to the CD300c protein. The term "polypeptide" is intended to mean any polymer of amino acids linked to one another by peptide bonds, irrespective of its length. That is, the polypeptide as used herein also encompasses peptides and proteins.

In an embodiment, the anti-CD300c antibody or antigen-binding fragment thereof is capable of specifically binding to an extracellular domain (ECD) of the CD300c protein. The extracellular domain of CD300c may be an extracellular domain of human CD300c protein, and may include the amino acid sequence as set forth in SEQ ID NO: 402.

In an embodiment, it was identified that the expression level of a CD300c protein exhibits a very high correlation with survival of various cancer patients. Specifically, it was identified that relative to the average CD300c expression level of cancer patients, cancer patients with a high CD300c expression level had shorter survival than cancer patients with a low CD300c expression level. This means that inhibition of expression or activity of CD300c using the anti-CD300c antibody or antigen-binding fragment thereof according to the present disclosure can result in a cancer therapeutic effect or an effect of increasing survival of cancer patients.

The anti-CD300c antibody or antigen-binding fragment thereof according to the present disclosure can specifically bind to CD300c expressed on the surface of various cancer cells, and thus exhibit an anticancer effect. The binding of the anti-CD300c antibody to CD300c can activate T cells and at the same time promote differentiation into M1 macrophages to effectively inhibit the proliferation of cancer cells, which allows the anti-CD300c antibody to be effectively used as an immunotherapeutic agent for various cancers. In addition, the anti-CD300c antibody according to the present disclosure can exhibit a further increased therapeutic effect through combined administration with a conventional immunotherapeutic agent, and also has inter-species cross-reactivity (for example, between human and mouse antigens) that allows the antibody to be widely applied to various mammals. In addition, it is expected that when resistant cancer cells showing the ability to resist apoptosis are treated with the anti-CD300c antibody of the present disclosure, this antibody is able to remarkably weaken resistance of the cancer cells, thereby showing excellent efficacy in preventing cancer recurrence. In addition, in general, cancer cells inhibit production of the proinflammatory cytokine IL-2 to evade the immune system. It was identified that the anti-CD300c antibody activates the immune system by restoring production of IL-2 blocked by these cancer cells, which induces cancer cell death. Thus, it is expected that the anti-CD300c antibody can be utilized as a more fundamental immunotherapeutic agent. For the details on the CD300c protein or the anti-CD300c antibody, reference may also be made to Korean Patent Publication No. 10-2019-0136949, which is incorporated herein by reference in its entirety for all purposes.

In an embodiment, the antibody or antigen-binding fragment thereof may include:
(i) a heavy chain variable region including: CDR1 including or consisting of an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295;
   CDR2 including or consisting of an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and
   CDR3 including or consisting of an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region including CDR1 including or consisting of an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298;
   CDR2 including or consisting of an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and
   CDR3 including or consisting of an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300.

In another embodiment, (i) the heavy chain variable region may include:
CDR1 including or consisting of an amino acid sequence selected from the group consisting of SEQ ID NO: 43, SEQ ID NO: 79, SEQ ID NO: 115, and SEQ ID NO: 211;
CDR2 including or consisting of an amino acid sequence selected from the group consisting of SEQ ID NO: 44, SEQ ID NO: 80, SEQ ID NO: 116, and SEQ ID NO: 212; and
CDR3 including or consisting of an amino acid sequence selected from the group consisting of SEQ ID NO: 45, SEQ ID NO: 81, SEQ ID NO: 117, and SEQ ID NO: 213; and
(ii) the light chain variable region may include:
CDR1 including or consisting of an amino acid sequence selected from the group consisting of SEQ ID NO: 46, SEQ ID NO: 82, SEQ ID NO: 118, and SEQ ID NO: 214;
CDR2 including or consisting of an amino acid sequence selected from the group consisting of SEQ ID NO: 47, SEQ ID NO: 83, SEQ ID NO: 119, and SEQ ID NO: 215; and
CDR3 including or consisting of an amino acid sequence selected from the group consisting of SEQ ID NO: 48, SEQ ID NO: 84, SEQ ID NO: 120, and SEQ ID NO: 216.

In still another embodiment, the antibody or antigen-binding fragment thereof may be any one selected from the following antibodies:
an antibody including: a heavy chain variable region including CDR1 including or consisting of the amino acid sequence as set forth in SEQ ID NO: 79, CDR2 including or consisting of the amino acid sequence as set forth in SEQ ID NO: 80, CDR3 including or consisting of the amino acid sequence as set forth in SEQ ID NO: 81; and a light chain variable region including CDR1 including or consisting of the amino acid sequence as set forth in SEQ ID NO: 82, CDR2 including or consisting of the amino acid sequence as set forth in SEQ ID NO: 83, CDR3 including or consisting of the amino acid sequence as set forth in SEQ ID NO: 84;
an antibody including: a heavy chain variable region including CDR1 including or consisting of the amino acid sequence as set forth in SEQ ID NO: 115, CDR2 including or consisting of the amino acid sequence as set forth in SEQ ID NO: 116, CDR3 including or consisting of the amino acid sequence as set forth in SEQ ID NO: 117; and a light chain variable region including CDR1 including or consisting of the amino acid sequence as set forth in SEQ ID NO: 118, CDR2 including or consisting of the amino acid sequence as set forth in SEQ ID NO: 119, CDR3 including or consisting of the amino acid sequence as set forth in SEQ ID NO: 120; and
an antibody or antigen-binding fragment thereof including: a heavy chain variable region including CDR1 including or consisting of the amino acid sequence as set forth in SEQ ID NO: 211, CDR2 including or consisting of the amino acid sequence as set forth in SEQ ID NO: 212, CDR3 including or consisting of the amino acid sequence as set forth in SEQ ID NO: 213; and a light chain variable region including CDR1 including or consisting of the amino acid sequence as set forth in SEQ ID NO: 214, CDR2 including or consisting of the amino acid sequence as set forth in SEQ ID NO: 215, CDR3 including or consisting of the amino acid sequence as set forth in SEQ ID NO: 216.

In still another embodiment, the antibody or antigen-binding fragment thereof may include: a heavy chain variable region including CDR1 including or consisting of the amino acid sequence as set forth in SEQ ID NO: 79, CDR2 including or consisting of the amino acid sequence as set forth in SEQ ID NO: 80, CDR3 including or consisting of the amino acid sequence as set forth in SEQ ID NO: 81; and a light chain variable region including CDR1 including or consisting of the amino acid sequence as set forth in SEQ ID NO: 82, CDR2 including or consisting of the amino acid sequence as set forth in SEQ ID NO: 83, CDR3 including or consisting of the amino acid sequence as set forth in SEQ ID NO: 84;
In still another embodiment, the antibody or antigen-binding fragment thereof may include: a heavy chain variable region including CDR1 including or consisting of the amino acid sequence as set forth in SEQ ID NO: 115, CDR2 including or consisting of the amino acid sequence as set forth in SEQ ID NO: 116, CDR3 including or consisting of the amino acid sequence as set forth in SEQ ID NO: 117; and a light chain variable region including CDR1 including or consisting of the amino acid sequence as set forth in SEQ ID NO: 118, CDR2 including or consisting of the amino acid sequence as set forth in SEQ ID NO: 119, CDR3 including or consisting of the amino acid sequence as set forth in SEQ ID NO: 120.

In still another embodiment, the antibody or antigen-binding fragment thereof may include: a heavy chain variable region including CDR1 including or consisting of the amino acid sequence as set forth in SEQ ID NO: 211, CDR2 including or consisting of the amino acid sequence as set forth in SEQ ID NO: 212, CDR3 including or consisting of the amino acid sequence as set forth in SEQ ID NO: 213; and a light chain variable region including CDR1 including or consisting of the amino acid sequence as set forth in SEQ ID NO: 214, CDR2 including or consisting of the amino acid sequence as set forth in SEQ ID NO: 215, CDR3 including or consisting of the amino acid sequence as set forth in SEQ ID NO: 216.

In still another embodiment, the heavy chain variable region may include an amino acid sequence selected from the group consisting of SEQ ID Nos: 303, 307, 311, 315, 319, 323, 327, 331, 335, 339, 343, 347, 351, 355, 359, 363, 367, 371, 375, 379, 383, 387, 391, 395, and 399; and the light chain variable region may include an amino acid sequence selected from the group consisting of SEQ ID Nos: 304, 308, 312, 316, 320, 324, 328, 332, 336, 340, 344, 348, 352, 356, 360, 364, 368, 372, 376, 380, 384, 388, 392, 396, and 400.

In still another embodiment, the heavy chain variable region may include an amino acid sequence selected from the group consisting of SEQ ID Nos: 315, 327, 339, and 371; and the light chain variable region may include an amino acid sequence selected from the group consisting of SEQ ID Nos: 316, 328, 340, and 372.

In still another embodiment, the heavy chain variable region may include: the amino acid sequence as set forth in SEQ ID No: 315 and the light chain variable region may include the amino acid sequence as set forth in of SEQ ID No: 316; the heavy chain variable region may include the amino acid sequence as set forth in SEQ ID No: 327 and the light chain variable region may include the amino acid sequence as set forth in of SEQ ID No: 328; the heavy chain variable region may include the amino acid sequence as set forth in SEQ ID No: 339 and the light chain variable region may include the amino acid sequence as set forth in of SEQ ID No: 340; or the heavy chain variable region may include the amino acid sequence as set forth in SEQ ID No: 371 and the light chain variable region may include the amino acid sequence as set forth in of SEQ ID No: 372.

In still another embodiment, the anti-CD300c antibody or antigen-binding fragment thereof may include: a heavy chain variable region including CDR1 to CDR3 including or consisting of the amino acid sequences represent by Formulas (1) to (3), respectively; and a light chain variable region including CDR1 to CDR3 including or consisting of the amino acid sequences represented by Formulas (4) to (6), respectively (each amino acid sequence is shown in N→C direction):
FTFX1X2X3X4MX5WVR (1) (SEQ ID NO: 403)
   wherein,
   X1= G or S
   X2= S, R, or D
   X3= N or Y
   X4= Y, A, G, or H
   X5= S or H
X1ISX2SGX3X4TYYAX5 (2) (SEQ ID NO: 404)
   wherein,
   X1= T or A
   X2= G or S
   X3= T or G
   X4= S or Y
   X5= D or E
YCAX1X2X3X4X5X6X7X8X9W (3) (SEQ ID NO: 405)
   wherein,
   X1= R or S
   X2= G or S
   X3= M, S, Y, or I
   X4= W, Q, G, or R
   X5= G or L
   X6= M, I, or P
   X7= D, F, or L
   X8= V or D
   X9= I, Y, or not present
CX1X2X3X4X5X6X7X8X9X10X11VX12W (4) (SEQ ID NO: 406)
   wherein,
   X1 = Tor S
   X2= G or R
   X3= K, N, or S
   X4= H, N, or S
   X5= R, I, or G
   X6= H, G, or I
   X7= T, I, or S
   X8= R, A, K, or not present
   X9= R, S, G, or not present
   X10= N or not present
   X11 = Y or not present
   X12= N, H, or Q
X1X2X3X4RPSGVX5 (5) (SEQ ID NO: 407)
   wherein,
   X1= L, S, R, or E
   X2= D, K, or N
   X3= S or N
   X4= E, N, Q, or K
   X5= P or R
YCX1X2X3X4X5X6X7X8X9X10VF (6) (SEQ ID NO: 408)
   wherein,
   X1= Q, A, or S
   X2= S or A
   X3= Y or W
   X4= D or A
   X5= S, D, or G
   X6= S, N, or T
   X7= S, L, N, or K
   X8= V, S, N, or G
   X9= G, L, V, or not present
   X10 = P or not present

In a specific embodiment, the anti-CD300c antibody or antigen-binding fragment may include sequences having 80% or more, preferably 90% or more, more preferably 95% or more, and most preferably 98% or more sequence identity to the CDR sequences or the sequences shown in Tables 4, 5, and 6.

In a specific embodiment, amino acid sequence variants of the antibodies of the present disclosure are contemplated. For example, it may be desirable to improve the binding affinity and/or other biological properties of the antibody. Amino acid sequence variants of the antibody may be prepared by introducing appropriate modifications into the nucleotide sequence encoding the molecules, or by peptide synthesis. Such modifications include, for example, deletions from, and/or insertions into and/or substitutions of residues within the amino acid sequence of the antibody. Any combination of deletion, insertion, and substitution can be made to arrive at the final construct, provided that the final construct possesses the desired characteristics, for example, antigen-binding. Sites of interest for substitutional mutagenesis include heavy chain variable regions (HVRs) and framework regions (FRs). Conservative substitutions are provided in Table 1 under the heading "Preferred Substitutions" and further described below in reference to amino acid side chain classes (1) to (6). Amino acid substitutions may be introduced into the molecule of interest and the products screened for a desired activity, for example, retained/improved antigen binding, decreased immunogenicity, or improved ADCC or CDC.

**Table 1]**

| Original residue | Exemplary substitutions | Preferred substitutions |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Ile |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

As used herein, the term "amino acid sequence variant" includes substantial variants wherein there are amino acid substitutions in one or more hypervariable region residues of a parent antigen binding molecule (for example, a humanized or human antibody). In general, the resulting variant(s) selected for further study will have modifications (for example, improvements) in certain biological properties (for example, increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, for example, using phage display-based affinity maturation techniques known in the art. Briefly, one or more HVR residues are mutated and the variant antigen binding molecules displayed on phage and screened for a particular biological activity (for example, binding affinity). In a specific embodiment, substitutions, insertions, or deletions may occur within one or more HVRs so long as such alterations do not substantially reduce the ability of the antigen binding molecule to bind antigen. For example, conservative alterations (for example, conservative substitutions as provided herein) that do not substantially reduce binding affinity may be made in HVRs.

Amino acid sequence insertions include amino- and/or carboxyl-terminal fusions ranging in length from one residue to polypeptides containing a hundred or more residues, as well as intra-sequence insertions of single or multiple amino acid residues. Examples of terminal insertions include antibodies with an N-terminal methionyl residue. Other insertional variants of the molecule include the fusion to the N- or C-terminus to a polypeptide which increases the serum half-life of the antibody. In addition, other insertional variants of the molecule may include fusions to the N-terminus or C-terminus of the polypeptide to facilitate passage of the blood-brain barrier (BBB).

In addition, there are provided variants of the antibody or antigen-binding fragment thereof of the present disclosure which have improved affinity for the CD300c antigen. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of *E*. *coli* (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996), and sexual PCR (Crameri et al., Nature, 391, 288-291, 1998). Vaughan et al. (Science, 239, 1534-1536, 1988) discuss these methods of affinity maturation.

In an embodiment, the anti-CD300c monoclonal antibody or antigen-binding fragment thereof may have inter-species cross-reactivity. Specifically, the anti-CD300c monoclonal antibody or antigen-binding fragment thereof may exhibit cross-reactivity to both human and mouse CD300c antigens. Such cross-reactivity is identified in Experimental Examples 3.1 to 3.8.

In another embodiment, the anti-CD300c monoclonal antibody or antigen-binding fragment thereof may include a form of an antibody-drug conjugate, in which the anti-CD300c monoclonal antibody or antigen-binding fragment thereof is bound to another drug, or may be provided in such a form.

As used herein, the term "antibody-drug conjugate" refers to a form in which the drug and the antibody are chemically linked to each other without degrading biological activity of the antibody and the drug. In the present disclosure, the antibody-drug conjugate denotes a form in which the drug is bound to an amino acid residue at the N-terminus of the heavy and/or light chain of the antibody, specifically, a form in which the drug is bound to an α-amine group at the N-terminus of the heavy and/or light chain of the antibody.

The "drug" may mean any substance having a certain biological activity for a cell (for example, a cancer cell), which is a concept including DNA, RNA, or a peptide. The drug may be in a form which contains a reactive group capable of reacting and crosslinking with an α-amine group, and also includes a form which contains a reactive group capable of reacting and crosslinking with an α-amine group and to which a linker is linked.

The reactive group capable of reacting and crosslinking with the α-amine group are not particularly limited in terms of type as long as the reactive group can react and crosslink with an α-amine group at the N-terminus of a heavy chain or light chain of an antibody. The reactive group includes all types of groups known in the art which react with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, but is not limited thereto.

The drug is contained regardless of the type of drug that can treat the disease targeted by the anti-CD300c antibody or antigen-binding fragment thereof according to the present disclosure, but may preferably be an anticancer agent.

The present inventors identified that an anti-CD300c antibody or antigen-binding fragment thereof could exhibit an enhanced anticancer effect in combination with at least one other anticancer agent. Therefore, the anti-CD300c antibody or antigen-binding fragment thereof of the present disclosure can be used in the prevention or treatment of cancer in combination with at least one other anticancer agent. In an embodiment, the anti-CD300c antibody or antigen-binding fragment thereof can be used in combination with at least one other immunotherapeutic agent and/or at least one chemotherapeutic agent. In another embodiment, the anti-CD300c antibody or antigen-binding fragment thereof can be used in combination with at least one other immunotherapeutic agent and at least one chemotherapeutic agent.

### Combined use with Immunotherapeutic agents

Immunotherapeutic agents have a novel mechanism by which immune cells in the body are activated to kill cancer cells, and thus are advantageous in that they can be widely used for most cancers without specific genetic mutations. In addition, the immunotherapeutic agents have fewer adverse effects in that they treat cancer by strengthening the patient's own immune system, and have effects of improving the patient's quality of life and significantly extending the survival. These immunotherapeutic agents include immune checkpoint inhibitors, and may be manufactured by known methods or commercially available products. Examples of the immunotherapeutic agent include, but are not limited to, anti-PD-1, anti-PD-L1, anti-CTLA-4, anti-CD47, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT antibodies. In addition, examples of the immunotherapeutic agent include, but are not limited to, durvalumab (Imfinzi), atezolizumab (Tecentriq), avelumab (Bavencio), pembrolizumab (Keytruda), nivolumab (Opdivo), αCD47, cemiplimab (Libtayo), magrolimab (Hu5F9-G4), and ipilimumab (Yervoy).

In an embodiment, the immunotherapeutic agent may include at least one selected from the group consisting of anti-PD-1, anti-PD-L1, anti-CTLA-4, anti-CD47, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT antibodies. In one example, the immunotherapeutic agent may include at least one selected from the group consisting of anti-PD-1, anti-PD-L1, anti-CTLA-4, and anti-CD47 antibodies.

In another embodiment, the immunotherapeutic agent may include at least one selected from the group consisting of durvalumab (Imfinzi), atezolizumab (Tecentriq), pembrolizumab (Keytruda), nivolumab (Opdivo), αCD47, and ipilimumab (Yervoy).

### Combined use with chemotherapeutic agents

Chemotherapeutic agent refers to a cancer therapeutic agent also called a cytotoxic anticancer agent or a chemical drug anticancer agent. These chemotherapeutic agents have side effects, such as vomiting, hair loss, and reduction of white blood cells, by attacking not only cancer cells but also surrounding normal cells. Therefore, the chemotherapeutic agents are not used alone but used in combination with other immunotherapeutic agents showing a synergistic effect when used together. These chemotherapeutic agents include small molecule therapeutic age and may be products that are manufactured or commercially available by known methods. Examples of the chemotherapeutic agents may include microtubule assembly inhibitors, DNA intercalators or replication inhibitors, multikinase inhibitors, angiogenesis inhibitors, antimetabolites, and Taxols, but are not limited thereto.

In an embodiment, the chemotherapeutic agent may include at least one selected from the group consisting of a microtubule assembly inhibitor, a DNA replication inhibitor, a multikinase inhibitor, and an angiogenesis inhibitor.

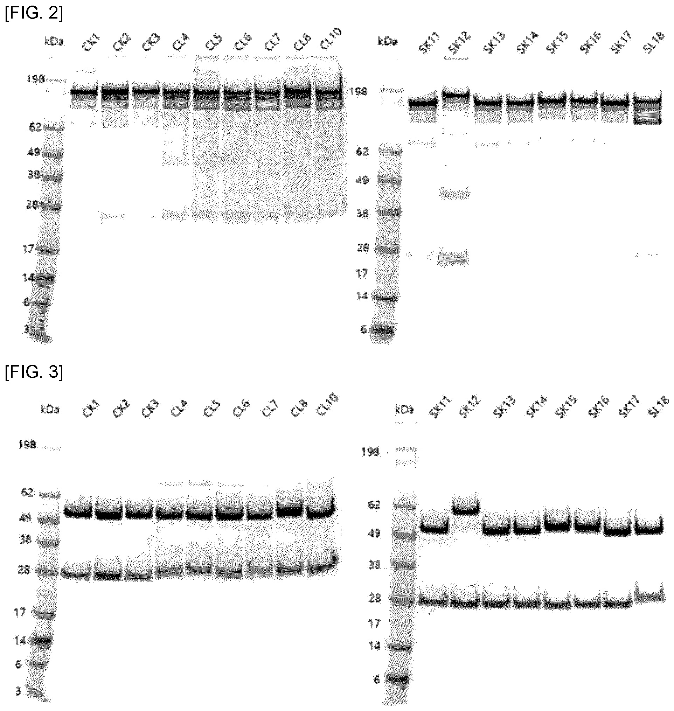

Specific examples of the chemotherapeutic agent are shown in FIG. 2.

**TABLE 2**

| Anticancer agents | Mechanism of Action (MoA) |
|---|---|
| Doxorubicin | Microtubule assembly inhibitors |
| Paclitaxel | |
| Docetaxel | |
| Vinblastine | |
| Vincristine | |
| Vinorelbine | |
| Estramustine Phosphate (EMP) | |
| NAB-Paclitaxel (Abraxane), | |
| Doxorubicin | DNA intercalators or replication inhibitors |
| Cyclophosphamide | |
| Epirubicin | |
| 5-Fluorouracil | |
| Etoposide | |
| Ifosfam ide | |
| Gemcitabine | |
| Deoxyadenosine | |
| Cladribine | |
| Clofarabine | |
| Fludarabine | |
| Pentostatin | |
| Deoxycytidine | |
| Cytosine Arabinoside (Ara-C) | |
| 5-Aza-2'-Deoxycytidine (Decitabine) | |
| Tezacitabine | |
| Capecitabine | |
| Cytarabine | |
| Methotrexate | |
| Pemetrexed | |
| Mercaptopurine | |
| Dactinomycin | |
| Daunorubicin | |
| Mitomycin | |
| Bleomycin | |
| Idarubicin | |
| Mitoxantrone HCL | |
| Mechlorethamine | |
| Melphalan | |
| Chlorambucil | |
| Thiotepa | |
| Altretamine | |
| Procarbazine | |
| Busulfan | |
| Streptozotocin | |
| Carmustine | |
| Lomustine | |
| Dacarbazine (DTI) | |
| Chlorambucil | |
| Topotecan | |
| Irinotecan | |
| Temozolomide | |
| Cisplatin | |
| Carboplatin | |
| Oxaliplatin | |
| Sorafenib | multikinase inhibitors or angiogenesis inhibitors |
| Regorafenib | |
| Vatalanib | |
| Axitinib | |
| Masitinib | |
| Pazopanib | |
| Sunitinib | |
| Toceranib | |
| Cediranib | |
| Lenvatinib | |
| Nintedanib | |
| Semaxanib | |
| Tivozanib | |
| Vandetanib | |
| Revlimid (Lenalidomide) | |

However, the chemotherapeutic agents are not limited to the anticancer agents shown in Table 2, and any chemotherapeutic agent may be used as long as it exerts the same or similar effect through the same or similar mechanisms of action as these chemotherapeutic agents.

In another embodiment, the chemotherapeutic agent may include at least one selected from the group consisting of sorafenib, gemcitabine, and paclitaxel.

### Dosage

The effective amounts or effective non-toxic amounts of the anti-CD300c antibody or antigen-binding fragment thereof and the additional anti-cancer agent (for example, an immunotherapeutic agent or a chemotherapeutic agent) may be determined by routine experiments. For example, the therapeutically active amount of an antibody or an anticancer agent may vary according to factors, such as the stage of a disease, severity of a disease, age, sex, medical complications, and weight of a subject, and the ability of a component to elicit a desired response in the subject, and the dosage of an anticancer agent used together. The dosage and dosing regimen of each of the anti-CD300c antibody or antigen-binding fragment thereof and the additional anticancer agent may be adjusted to provide an optimal therapeutic response. For example, several divided doses may be administered daily, weekly, every other week, every three weeks, every four weeks or the like, and/or the dose may be proportionally reduced as indicated by the exigencies of the therapeutic situation.

### Method for Prevention or Treatment of Cancer

According to an aspect of the present disclosure, there is provided a method for preventing or treating cancer, alleviating or decreasing severity of at least one symptom or sign of cancer, inhibiting metastasis, or inhibiting growth of cancer. As used herein, "preventing or treating cancer" may include inhibiting proliferation, survival, metastasis, recurrence, or anticancer agent resistance of cancer. Such a method includes the combined use of an anti-CD300c antibody or antigen-binding fragment thereof and at least one additional anticancer agent. Specifically, the method includes administering a therapeutically effective amount of an anti-CD300c antibody or antigen-binding fragment thereof and a therapeutically effective amount of at least one additional anticancer agent to a subject in need thereof. The at least additional anticancer agent may include at least one immunotherapeutic agent, at least one chemotherapeutic agent, or at least one immunotherapeutic agent and at least one chemotherapeutic agent.

The term "cancer" refers to a physiological condition that is typically characterized by unregulated cell growth in mammals. The cancer to be prevented or treated in the present disclosure may include, depending on the site of occurrence, colorectal cancer, small intestine cancer, rectal cancer, colon cancer, thyroid cancer, endocrine adenocarcinoma, oral cancer, tongue cancer, pharyngeal cancer, laryngeal cancer, esophageal cancer, cervical cancer, uterine cancer, fallopian tube cancer, ovarian cancer, brain cancer, head and neck cancer, lung cancer, lymph gland cancer, gallbladder cancer, bladder cancer, kidney cancer, liver cancer, pancreatic cancer, prostate cancer, skin cancer (or melanoma), breast cancer, stomach cancer, bone cancer, blood cancer, and the like. However, any cancer can be included therein as long as it expresses a CD300c protein on the surface of cancer cells. In an embodiment, the cancer may include at least one selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer. In another embodiment, the cancer may be a solid cancer.

In an embodiment, the anti-CD300c antibody or antigen-binding fragment thereof and the at least one additional anticancer agent may be administered simultaneously or sequentially.

"Administered sequentially" means that one ingredient is first administered and the other ingredient is administered immediately or at a predetermined interval after the first administration, wherein the ingredients may be administered in any order. That is, the anti-CD300c antibody or antigen-binding fragment thereof may be first administered and at least one additional anticancer agent may be administered immediately or at a predetermined interval after the first administration, or *vice versa.* In addition, any one of the at least one additional anticancer agent may be first administered, followed by the anti-CD300c antibody or antigen-binding fragment thereof, and then the other of the at least one additional anticancer agent.

In an embodiment, the anti-CD300c antibody or antigen-binding fragment thereof may be administered in combination with two or more additional anticancer agents. In one example, it was identified that a highest cancer cell proliferation inhibitory effect was observed when the anti-CD300c antibody or antigen-binding fragment thereof was used in combination with two immunotherapeutic agents (for example, an anti-PD-L1 antibody and an anti-PD-1 antibody, or an anti-PD-1 antibody and an anti-CTLA-4 antibody).

Each of the antibody or antigen-binding fragment thereof according to the present disclosure and at least one additional anticancer agent may be administered in several ways depending on whether local or systemic treatment is desired and on the area to be treated. Methods of administering these ingredients to a subject may vary depending on the purpose of administration, the site of a disease, the condition of a subject, and the like. The route of administration may be oral, parenteral, inhalation, local or topical (for example, intralesional administration). For example, parenteral administration may include, but is not limited to, intravenous, subcutaneous, intraperitoneal, intrapulmonary, intraarterial, intramuscular, rectal, vaginal, intraarticular, intraprostatic, intranasal, intraocular, intravesical, intrathecal, or intraventricular administration (for example, intracerebroventricular administration). In addition, the anti-CD300c antibody and the additional anticancer agent may be administered through the same route or mutually different routes.

In the methods, the effective amount of each of the anti-CD300c antibody or antigen-binding fragment thereof according to the present disclosure and the at least one additional cancer agent may vary depending on the age, sex, and body weight of an individual (patient). In general, administration may be performed in an amount of about 0.01 mg to about 100 mg, or about 5 mg to about 50 mg, per kg of body. The amount may be administrated once a day or several times a day in divided doses. However, the effective amount may be increased or decreased depending on route and period of administration, severity of disease, sex, body weight, age, and the like. Thus, the range of the present disclosure is not limited thereto.

The method according to the present disclosure may include identifying the expression level of a CD300c protein from the subject in advance. Depending on the expression level, determination may be made on whether or not to administer the anti-CD300c antibody or antigen-binding fragment thereof.

In an embodiment, the method may further include identifying the expression level of a CD300c protein on the basis of a biological sample or data of the subject, before the administration of the anti-CD300c antibody or antigen-binding fragment thereof, before the administration of the anti-CD300c antibody or antigen-binding fragment thereof.

In addition, the method may include determining that the subject is a suitable for the treatment using an anti-CD300c antibody or antigen-binding fragment thereof when the expression level of the CD300c protein determined on the basis of a biological sample or data of the subject is statistically significantly higher (for example, at least 10% higher) than that in a control group (for example, the expression level in normal people without cancer or the average expression level in cancer patients). However, the exemplified difference in expression level of the CD300c protein is just an example and may be 20% or more, 30% or more, 40% or more, 50% or more, 60% or more, or 70% or more, but is not limited thereto. Preferably, the control group may show the average expression level in cancer patients of the same type. In another embodiment, the method according to the present disclosure may include selecting an additional immunotherapeutic agent suitable for use in combination with the anti-CD300c antibody or antigen-binding fragment thereof by measuring the change in expression level of a specific marker after the administration of the anti-CD300c antibody or antigen-binding fragment thereof to a subject.

Specifically, the method may further include determining the expression level of at least one marker selected from the following markers on the basis of a biological sample or data from the subject administered the anti-CD300c antibody or antigen-binding fragment thereof:
Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, PD-1, PD-L1, CTLA-4, Lag3, Tim3, Ox40, Gitr, Hvem, CD27, CD28, Cma1, Timd4, Bcl6, Cxcl5, and Ccl21a.

See Table 3 for description of the markers.

**[Table 3]**

| No. | Name | Full name | Brief description |
|---|---|---|---|
| 1 | Bst2 | Bone marrow stromal antigen 2 | This is also known as Tetherin or CD137, and is a lipid raft-associated protein encoded by the BST2 gene. |
| 2 | CD40 | Cluster of differentiation 40 | This is a co-stimulatory protein found in antigen-presenting cells and is required for their activation. The binding of CD154 (CD40L) on TH cells to CD40 activates antigen presenting cells and induces a variety of downstream effects. |
| 3 | CD70 | Cluster of differentiation 70 | This is a protein encoded by the CD70 gene in humans. CD70 is a ligand for CD27. |
| 4 | CD86 | Cluster of differentiation 86 | This is also known as B7-2, and is a protein constitutively expressed in dendritic cells, Langerhans cells, macrophages, B-cells (including memory B-cells), and in other antigen-presenting cells. |
| 5 | Ccl8 | Chemokine (C-C motif) ligand 8 | This is also known as monocyte chemoattractant protein 2 (MCP2) and is a protein encoded by the CCL8 gene in humans. |
| 6 | Xcl1 | X-c motif chemokine ligand 1 | This is a small cytokine belonging to the C chemokine family that is also known as lymphotactin. |
| 7 | Ccr7 | C-C chemokine receptor type 7 | This is a protein encoded by the CCR7 gene in humans. The following two ligands have been identified for this receptor: chemokine (C-C motif) ligand19 (CCL19/ELC) and (C-C motif) ligand 21 (CCL21). |
| 8 | CD80 | Cluster of differentiation 80 | This is a B7, type 1 membrane protein in the immunoglobulin superfamily, with an extracellular immunoglobulin constant-like domain and a variable-like domain required for receptor binding. It is closely related to CD86, another B7 protein (B7-2), and often works in tandem, binding to the same receptors to prime T cells. |
| 9 | CD20 6 | Cluster of differentiation 206 | CD206 is also known as the mannose receptor. This is a 206 C-type lectin primarily present on the surface of macrophages, immature dendritic cells, and liver sinusoidal endothelial cells, but is also expressed on the surface of skin cells such as human dermal fibroblasts and keratinocytes. |
| 10 | Msr1 | Macrophage scavenger receptor 1 | This is a protein encoded by the scavenger MSR1 gene in humans. MSR1 is receptor 1 also called cluster of differentiation 204 (CD204). |
| 11 | Arg1 | Arginase 1 | The human ARG1 gene encodes a protein arginase. |
| 12 | Vegfa | Vascular endothelial growth factor A | This is a protein encoded by the VEGFA gene in humans. |
| 13 | Pdgfrb | Platelet-derived growth factor receptor beta | This is a protein encoded by the PDGFRB gene in humans. |
| 14 | Col4a 1 | Collagen, type IV, alpha 1 | This is a protein encoded by the COL4A1 gene on chromosome in humans. |
| 15 | Hif1a | Hypoxia-inducible factor 1-alpha | This is also known as HIF-1-alpha and is a subunit of a hetero-dimeric transcription factor hypoxia-inducible factor 1 (HIF-1) encoded by the HIF1A gene. |
| 16 | Vcam 1 | Vascular cell adhesion protein 1 | This is also known as vascular cell adhesion molecule 1 (VCAM-1) protein 1 or cluster of differentiation 106 (CD106) and is a protein encoded by the VCAM1 gene in humans. |
| 17 | Icam1 | Intercellular adhesion molecule 1 | This is also known as CD54 Adhesion (Cluster of Differentiation 54) and is a protein encoded by the ICAM1 gene in humans. |
| 18 | Gzma | Granzyme A | This is encoded by the Gzma gene and is present in cytotoxic T lymphocyte granules. |
| 19 | Gzmb | Granzyme B | This is encoded by the Gzmb gene and is expressed by cytotoxic T lymphocytes (CTL) and natural killer (NK) cells. |
| 20 | Icos | Inducible T-cell co-stimulator | This is an immune checkpoint protein encoded by the ICOS gene in humans. |
| 21 | Cd69 | Cluster of Differentiation 69 | This is a human transmembrane C-Type lectin protein encoded by the CD69 gene, and is an early activation marker expressed in hematopoietic stem cells, T cells, and the like. |
| 22 | Ifng | Interferon gamma | This is a dimerized soluble that is the only member of the type II class of interferons. |
| 23 | Tnf | Tumor necrosis factor | This is encoded by the Tnf gene and is a multifunctional pro-inflammatory cytokine that belongs to the tumor necrosis factor (TNF) superfamily and is secreted primarily by macrophages. |
| 24 | Cd1d1 | CD1d1 antigen | This enables T cell receptor binding activity and endogenous lipid antigen binding activity, and is involved in differentiation of natural killer T (NKT) cells and regulation of immature T cell proliferation. |
| 25 | Cd1d2 | CD1d2 antigen | This encodes an MHC class I-like molecule involved in presentation of lipid antigens to T cells, and is involved in activation of natural killer T cells. |
| 26 | Cd38 | Cluster of Differentiation 38 | This is a glycoprotein found on the surface of many immune cells (white blood cells), including CD4+, CD8+, B lymphocytes, and natural killer cells. |
| 27 | Cxcr6 | C-X-C chemokine receptor type 6 | This is also called CD 186 and has been identified as an entry coreceptor used by HIV-1 and SIV to enter target cells, in conjunction with CD4. |
| 28 | Xcr1 | X-C motif chemokine receptor 1 | This is a chemokine receptor belonging to the G protein-coupled receptor superfamily. |
| 29 | Tbx21 | T-box transcription factor TBX21 | This is also called T-box transcription expressed in T cells (T-bet) and is a protein encoded by the TBX21 gene in humans. |
| 30 | Stat1 | Signal transducer and activator of transcription 1 | This is a transcription factor encoded by the STAT1 gene in humans. It is a member of the STAT protein family. |
| 31 | Stat4 | Signal transducer and activator of transcription 4 | This is a transcription factor belonging to the STAT protein family. |
| 32 | Cxcr3 | C-x-c motif chemokine receptor 3 | This is a Gαᵢ protein-coupled receptor in the CXC chemokine receptor family. |
| 33 | IL-12b | Subunit beta of interleukin 12 | This is also known as IL-12B, natural killer cell stimulatory factor 2, cytotoxic lymphocyte maturation factor p40, or interleukin-12 subunit p40, and is a protein encoded by the IL12B gene in humans. |
| 34 | IL4 | Interleukin 4 | This is a cytokine that induces differentiation of naive helper T cells (Th0 cells) to Th2 cells. Upon activation by IL-4, Th2 cells subsequently produce additional IL-4 in a positive feedback loop. |
| 35 | IL6 | Interleukin 6 | This is an interleukin that acts as both a pro-inflammatory cytokine and an anti-inflammatory myokine. In humans, it is encoded by the IL6 gene. |
| 36 | IL13 | Interleukin 13 | This is a protein encoded by the IL13 gene in humans. |
| 37 | PD-1 | Programmed cell death protein 1 | This is also known as cluster of differentiation 279 (CD279), and is a protein that has a role in regulating the immune system's response to the cells of the human body by down-regulating the immune system and promoting self-tolerance by suppressing T cell inflammatory activity. It prevents autoimmune diseases, but it can also prevent the immune system from killing cancer cells. |
| 38 | PD-L1 | Programmed death-ligand 1 | This is also known as cluster of death- differentiation 274 (CD274) or B7 homolog 1 (B7-H1), and is a protein encoded by the CD274 gene in humans. |
| 39 | CTLA-4 | Cytotoxic T-lymphocyte-associated protein 4 | This is also known as cluster of differentiation 152 (CD152), and is a protein receptor that functions as an immune checkpoint and downregulates immune responses. |
| 40 | Lag3 | Lymphocyte-activation gene 3 | This is a protein encoded by the LAG3 gene in humans. |
| 41 | Tim3 | Immunoglobulin and mucin-domain containing-3 | This is also known as hepatitis A virus cellular receptor 2 (HAVCR2), and is a protein encoded by the HAVCR2 gene in humans. |
| 42 | Ox40 | (Cd134, tnfrsf4) | This is a member of the TNFR-superfamily of receptors which is not constitutively expressed on resting naive T cells, unlike CD28. |
| 43 | Gitr | Glucocorticoid-induced tumor necrosis factor receptor | This is a protein encoded by the TNFRSF18 gene in humans. |
| 44 | Hvem | Herpesvirus entry mediator | This is a human cell surface receptor of the TNF-receptor superfamily. |
| 45 | CD27 | Cluster of differentiation 27 | This is a member of the tumor necrosis factor receptor superfamily. |
| 46 | CD28 | Cluster of differentiation 28 | This is one of the proteins expressed on T cells that provide co-stimulatory signals required for T cell activation and survival. |
| 47 | Cma1 | Chymase 1 | This is an enzyme encoded by the CMA1 gene in humans. |
| 48 | Timd4 | T-cell immunoglobulin and mucin domain containing 4 | This is also known as T-cell membrane protein 4 (TIM-4) and is a protein encoded by the TIMD4 gene in humans. |
| 49 | Bcl6 | B-cell lymphoma 6 protein | This is a protein encoded by the BCL6 gene in humans. Like BCL2, BCL3, BCL5, BCL7A, BCL9, and BCL10, it has clinical significance in lymphoma. |
| 50 | Cxcl5 | C-x-c motif chemokine ligand 5 | This is a protein encoded by the CXCL5 gene in humans. |
| 51 | Ccl21a | Chemokine (C-C motif) ligand 21 | This is a small cytokine belonging to the CC chemokine family. |

In still another embodiment, the method may further include selecting an additional anticancer agent on the basis of the determined expression level of the marker. Specifically, the marker may include, but is not limited to, PD-1, PD-L1, CTLA-4, Lag3, Tim3, Icos, Ox40, Gitr, Hvem, CD27, and CD28. In still another embodiment, the marker may include at least one selected from the group consisting of PD-1, PD-L1, CTLA-4, Lag3, Tim3, Icos, Ox40, Gitr, Hvem, CD27, and CD28. In still another embodiment, the marker may include at least one selected from the group consisting of PD-1, PD-L1, CTLA-4, Lag3, and Tim3. In still another embodiment, the marker may include at least one selected from the group consisting of Icos, Ox40, Gitr, Hvem, CD27, and CD28.

The changes in expression levels of these markers mean changes in tumor/immune-related markers, which affect tumor suppressive effects, observed when an anti-CD300c antibodies or antigen-binding fragment thereof of the present disclosure is administered to an individual. For example, the changes in expression levels of the markers may include changes in expression pattern of protein markers or immune checkpoint protein markers related to activity of immune cells (for example, dendritic cells, macrophages, T cells, NKT cells), tumor microenvironment (TME) protein markers that affect tumor proliferation, and markers related to Th1 and Th2 responses. As for specific examples of the markers, reference is made to the description as shown above. Through these changes in expression pattern, it is possible to predict the probability that a patient will respond to a drug or to select the anticancer agent, including another immune checkpoint inhibitor, which can maximize an anticancer effect. In addition, utilization of the markers makes it possible to determine whether a patient can be treated with the antibody. In addition, it is possible to monitor therapeutic effects of a drug. In addition, it is possible to provide information for treatment methods, including drug doses, usages, combination therapies, and the like.

According to an embodiment of the present disclosure, it was identified that an enhanced antitumor effect was exhibited when the anti-CD300c antibody or antigen-binding fragment thereof of the present disclosure was used in combination with another immune checkpoint inhibitor (at least one of durvalumab (Imfinzi) that is an anti-PD-L1 antibody, nivolumab (Opdivo) that is an anti-PD-1 antibody, pembrolizumab (Keytruda) that is an anti-PD-1 antibody, an anti-CTLA-4 antibody, and an anti-CD47 antibody (αCD47)) which is selected by changes in expression levels of the markers in an individual observed after administration of the anti-CD300c antibody or antigen-binding fragment thereof.

In still another embodiment, the method may further include determining the therapeutic responsiveness of the anti-CD300c antibody or antigen-binding fragment thereof on the basis of the determined expression level of the marker. The marker may include, but is not limited to, vegfa, pdgfrb, Col4a1, Hif1a, Bst2, CCL8, Xcl1, CCR7, CD80, Tbx21, Stat1, Stat4, Ifng, Cxcr3, IL-6, Gzma, Icos, Cd69, Cd1d1, Cd38, Cxcr6, Ox40, Gitr, CD27, and CD28. Preferably, the marker may include at least one selected from the group consisting of vegfa, pdgfrb, Col4a1, Hif1a, Bst2, CCL8, Xcl1, CCR7, CD80, Tbx21, Stat1, Stat4, Ifng, Cxcr3, IL-6, Gzma, Icos, Cd69, Cd1d1, Cd38, and Cxcr6. In addition, the marker may include at least one selected from the group consisting of vegfa, pdgfrb, Col4a1, and Hif1a. Additionally, the marker may include at least one selected from the group consisting of Bst2, CCL8, and Xcl1. In addition, the marker may include CCR7, CD80, or a combination thereof. In still another embodiment, the marker may include at least one selected from the group consisting of Tbx21, Stat1, Stat4, Ifng, Cxcr3, and IL-6.

In still another embodiment, the method may further include determining that the therapeutic responsiveness of the anti-CD300c antibody or antigen-binding fragment thereof is good or excellent when the expression level of at least one among the markers is reduced compared with a subject not having been administered the anti-CD300c antibody or antigen-binding fragment thereof. For example, according to the method, it may be determined that the therapeutic responsiveness of the anti-CD300c antibody or antigen-binding fragment thereof is good or excellent when the expression level of at least one marker selected from the group consisting of vegfa, pdgfrb, Col4a1, Hif1a, and IL-6 is reduced compared with a subject not having been administered the anti-CD300c antibody or antigen-binding fragment thereof. Specifically, the reduction in expression level means a statistically significant reduction. A reduction rate in expression level may include, but is not limited to, about 10% or higher, about 20% or higher, about 30% or higher, about 40% or higher, about 50% or higher, about 60% or higher, about 70% or higher, or about 100% or higher.

In addition, the method may further include determining that the therapeutic responsiveness of the anti-CD300c antibody or antigen-binding fragment thereof is good or excellent when the expression level of at least one of the markers is increased compared with a subject not having been administered the anti-CD300c antibody or antigen-binding fragment thereof. For example, according to the method, it may be determined that the therapeutic responsiveness of the anti-CD300c antibody or antigen-binding fragment thereof is good or excellent when the expression level of at least one marker selected from the group consisting of Bst2, CCL8, Xcl1, CCR7, CD80, Tbx21, Stat1, Stat4, Ifng, Cxcr3, Gzma, Icos, Cd69, Cd1d1, Cd38, Cxcr6, Ox40, Gitr, Cd27, and Cd28 is increased compared with a subject administered the anti-CD300c antibody or antigen-binding fragment thereof. Specifically, the increase in expression level means a statistically significant increase. An increase rate in expression level may include, but is not limited to, about 10% or higher, about 20% or higher, about 30% or higher, about 40% or higher, about 50% or higher, about 60% or higher, about 70% or higher, or about 100% or higher.

### Pharmaceutical Composition

According to still another aspect of the present disclosure, there is provided a pharmaceutical composition for preventing or treating cancer, the pharmaceutical composition including, as active ingredients, an anti-CD300c antibody or antigen-binding fragment thereof and at least one additional anticancer agent.

Specifically, the anti-CD300c antibody or antigen-binding fragment thereof and the at least one additional anticancer agent may be contained at a prophylactically or therapeutically effective amount in the composition. In an embodiment, the additional anticancer agent may include an immunotherapeutic agent, a chemotherapeutic agent, or a combination thereof. The pharmaceutical composition may be administered to a subject to inhibit the proliferation, survival, metastasis, recurrence, or anticancer agent resistance of cancer.

In an embodiment, the anti-CD300c antibody or antigen-binding fragment thereof and the at least one additional anticancer agent may be each formulated and separately administered simultaneously or sequentially.

In an embodiment, the antibody or antigen-binding fragment thereof and the at least one additional anticancer agent may be contained in the same composition or separately contained in individual compositions. In another embodiment, the antibody or antigen-binding fragment thereof and the at least one additional anticancer agent may be separately contained in individual compositions.

To prepare the pharmaceutical composition of the present disclosure, the antibody or antigen-binding fragment thereof and/or the additional anticancer agent may be mixed with a pharmaceutically acceptable carrier and/or excipient. The pharmaceutical composition may be prepared in the form of a lyophilized preparation or an aqueous solution. For example, see literature [Remington's Pharmaceutical Sciences and U.S. Pharmacopeia: National Formulary, Mack Publishing Company, Easton, PA (1984)].

Acceptable carriers and/or excipients (including stabilizers) are non-toxic to the subject at the dosages and concentrations employed, and examples thereof may include, but are not limited to, buffers (such as phosphate, citrate, or organic acids); antioxidants (such as ascorbic acid or methionine); preservatives (such as octadecyldimethylbenzyl ammonium chloride; hexamethonium chloride; benzalkonium chloride, benzethonium chloride; phenol, butyl or benzyl alcohol; alkyl paraben, for example, methyl or propyl paraben; catechol; resorcinol; cyclohexanol; 3-pentanol; and m-cresol); low-molecular weight (less than about 10 residues) polypeptides; proteins (such as serum albumin, gelatin, or immunoglobulins); hydrophilic polymers (such as polyvinylpyrrolidone); amino acids (such as glycine, glutamine, asparagine, histidine, arginine, or lysine); monosaccharides, disaccharides, and other carbohydrates, for example, glucose, mannose, and dextrins; chelating agents (such as EDTA); sugars (such as sucrose, mannitol, trehalose, or sorbitol); salt-forming counter-ions (such as sodium); metal complexes (such as Zn-protein complexes); and (or) non-ionic surfactants (such as TWEEN^{™}, PLURONICS^{™}, or polyethylene glycol (PEG)).

The pharmaceutical composition of the present disclosure may be formulated in a suitable form known in the art depending on the route of administration.

As used herein, the term "prophylactically or therapeutically effective amount" or "effective amount" refers to an amount of an active ingredient in a composition which is effective for preventing or treating cancer in a subject. This amount is also sufficient for preventing or treating cancer at a reasonable benefit/risk ratio applicable to medical treatment and does not cause adverse effects. The level of the effective amount may be determined depending on the patient's health status, type of disease, severity of disease, activity of the drug, sensitivity to the drug, method of administration, frequency of administration, route of administration and rate of excretion, duration of treatment, drugs used in combination or coincidentally therewith, and other factors well known in the medical field. Specifically, it is important to administer a minimum amount that allows the maximum effect to be achieved with minimal or no adverse effects in consideration of all of the above factors, which can be easily determined by those skilled in the art.

Specifically, the effective amount of each active ingredient in the pharmaceutical composition of the present disclosure may vary depending on the age, sex, and body weight of an individual (patient). In general, about 0.01 mg to about 100 mg, or about 5 mg to about 50 mg, per kg of body, may be administered once a day or several times a day in divided doses. However, the scope of the present disclosure is not limited thereto since the effective amount may be increased or decreased depending on the route and duration of administration, severity of disease, sex, body weight, age, and the like.

### Kit for preventing or treating cancer

According to still another aspect of the present disclosure, there is provided a kit for preventing or treating cancer, the kit including the anti-CD300c antibody or antigen-binding fragment thereof according to the present disclosure as a first active ingredient and the additional anticancer agent as a second active ingredient.

In an embodiment, the first active ingredient and the second active ingredient may be mixed and formulated and then placed in the same container. In another embodiment, the first active ingredient and the second active ingredient may be individually formulated and then placed in the same container or in separate containers, and may be administered simultaneously or administered sequentially at time intervals regardless of the order.

In an embodiment, the kit may further include instructions containing medication or administration instructions of the active ingredients. Optionally, instruments or devices required to administer each active ingredient may be included in the kit.

There is also provided a kit for preventing or treating cancer, the kit including: (i) a composition containing the anti-CD300c antibody or antigen-binding fragment thereof according to the present disclosure; and (ii) instructions directing the combined use of the antibody or antigen-binding fragment thereof and at least one additional anticancer agent. Specifically, the composition may contain a prophylactically or therapeutically effective amount of the anti-CD300c antibody or antigen-binding fragment.

### Method and kit for predicting therapeutic responsiveness

According to still another aspect of the present disclosure, there is provided a method for providing information needed for predicting the therapeutic responsiveness of an anti-CD300c antibody or antigen-binding fragment thereof. The method may include determining the expression level of a marker for predicting the therapeutic responsiveness on the basis of a biological sample or data obtained from a subject. The marker may include at least one selected from the group consisting of Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, PD-1, PD-L1, CTLA-4, Lag3, Tim3, Ox40, Gitr, Hvem, CD27, CD28, Cma1, Timd4, Bcl6, Cxcl5, and Ccl21a, but is not limited thereto. In an embodiment, the marker may include at least one selected from the group consisting of Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, Pd-1, Pd-l1, Ctla-4, Lag3, Tim3, Ox40, Gitr, Hvem, Cd27, Cd28, Cma1, Timd4, Bcl6, Cxcl5, and Ccl21a. In another embodiment, the marker may include at least one selected from the group consisting of vegfa, pdgfrb, Col4a1, Hif1a, Bst2, CCL8, Xcl1, CCR7, CD80, Tbx21, Stat1, Stat4, Ifng, Cxcr3, IL-6, Gzma, Icos, Cd69, Cd1d1, Cd38, Cxcr6, Ox40, Gitr, Cd27, and Cd28.

In addition, the method may further include determining that the therapeutic responsiveness of the anti-CD300c antibody or antigen-binding fragment thereof is good or excellent when the expression level of at least one marker among the above-described markers is reduced compared with a control group, for example, a subject not having been administered the anti-CD300c antibody or antigen-binding fragment thereof. In an embodiment, according to the method, the therapeutic responsiveness of the anti-CD300c antibody or antigen-binding fragment thereof may be determined to be good or excellent when the expression level of at least one marker selected from the group consisting of vegfa, pdgfrb, Col4a1, Hif1a, and IL-6 is reduced compared with a control group, for example, a subject not having been administered the anti-CD300c antibody or antigen-binding fragment thereof. Specifically, the reduction in expression level means a statistically significant reduction, and the expression level reduction rate may include, but is not limited to, at least about 10%, at least about 20%, at least about 30%, at least about 40%, at least about 50%, at least about 60%, at least about 70%, and at least about 100%.

In addition, the method may further include determining that the therapeutic responsiveness of the anti-CD300c antibody or antigen-binding fragment thereof is good or excellent when the expression level of at least one marker among the above-described markers is increased compared with a control group, for example, a subject not having been administered the anti-CD300c antibody or antigen-binding fragment thereof. In an embodiment, according to the method, the therapeutic responsiveness of the anti-CD300c antibody or antigen-binding fragment thereof may be determined to be good or excellent when the expression level of at least one marker selected from the group consisting of Bst2, CCL8, Xcl1, CCR7, CD80, Tbx21, Stat1, Stat4, Ifng, Cxcr3, Gzma, Icos, Cd69, Cd1d1, Cd38, Cxcr6, Ox40, Gitr, Cd27, and Cd28 is increased compared with a subject not having been administered the anti-CD300c antibody or antigen-binding fragment thereof. Specifically, the increase in expression level means a statistically significant increase, and the expression level increase rate may include, but is not limited to, at least about 10%, at least about 20%, at least about 30%, at least about 40%, at least about 50%, at least about 60%, at least about 70%, and at least about 100%.

According to still another aspect of the present disclosure, there is provided a kit including a substance for measuring the expression level of the marker for predicting the therapeutic responsiveness of the anti-CD300c antibody or antigen-binding fragment thereof. The kit may include one or more types of constituent compositions, solutions, or devices, which are suitable for analysis methods. The kit may be a kit for measuring the expression level of a protein marker, for example, an enzyme-linked immunosorbent assay (ELISA) kit. The kit may include other reagents known in the art to be necessary for the immunological detection of the antibody.

### Method and kit for providing information for the prevention or treatment of cancer.

According to still another aspect of the present disclosure, there is provided a method for providing information for the prevention or treatment of cancer, the method including determining the expression level of a CD300c protein on the basis of a biological sample or data obtained from a subject in need of the prevention or treatment of cancer.

In an embodiment, the method may further include comparing the measured expression level of the CD300c protein with that in a control group (the expression level in normal people or the average expression level in cancer patients). Specifically, the subject may be determined to be suitable for the prevention or treatment of cancer using an anti-CD300c antibody or antigen-binding fragment thereof when the expression level of the CD300c protein is statistically significantly higher compared with the control group, for example, by 1.1-fold or more, 1.2-fold or more, 1.3-fold or more, 1.4-fold or more, or 1.5-fold or more. However, the difference in expression level is not limited thereto.

The information for the prevention or treatment of cancer may include information about at least one of the therapeutic responsiveness of a therapeutic agent (for example, an anti-CD300c antibody or antigen-binding fragment thereof) associated with the CD300c protein, the selection of a therapeutic agent, the selection of a subject to be treated, the prognosis of a subject, and the survival period of a subject. Preferably, the information for the prevention or treatment of cancer may include cancer therapeutic responsiveness of the anti-CD300c antibody or antigen-binding fragment thereof, survival period of the subject, or both thereof.

According to still another aspect of the present disclosure, there is provided a kit for providing information for the prevention or treatment of cancer, the kit including a substance for measuring the expression level of a CD300c protein by using a biological sample or data obtained from a subject in need of the prevention or treatment of cancer. The kit may include one or more types of constituent compositions, solutions, or devices, which are suitable for analysis methods. The kit may be a kit for measuring the expression level of a protein marker, for example, an enzyme-linked immunosorbent assay (ELISA) kit. The kit may include other reagents known in the art to be necessary for the immunological detection of the antibody.

### [DETAILED DESCRIPTION OF THE INVENTION]

Hereinafter, the present disclosure will be described in more detail by way of examples. However, the following examples are only for illustrating the present disclosure, and the scope of the present disclosure is not limited thereto.

### I. Monoclonal antibodies specifically binding to CD300c

### Example 1. Production of Anti-CD300c Monoclonal Antibodies

### Example 1.1. Construction of Anti-CD300c Monoclonal Antibody Library

In order to select anti-CD300c monoclonal antibodies, biopanning was performed using a lambda phage library, a kappa phage library, a VH3VL1 phage library, and an OPALTL phage library. More specifically, a CD300c antigen was added at a concentration of 5 µg/mL to each immunotube, followed by incubation for 1 hour, to allow the antigen to be adsorbed on the surface of the immunotube. Thereafter, 3% skim milk was added to suppress non-specific reactions. Then, 10¹² PFU of the antibody phage library dispersed in 3% skim milk was added to each immunotube for antigen binding. Thereafter, non-specifically bound phages were removed by washing three times using Tris buffered saline-Tween 20 (TBST) solution, and then single-chain variable fragment (scFv) phage antibodies specifically binding to the CD300c antigen were eluted using 100 mM triethylamine solution. The eluted phages were neutralized using 1.0 M Tris-HCl buffer (pH 7.8), and then *E*. *coli* ER2537 was infected with the eluted phages at 37°C for 1 hour. The infected *E*. *coli* was applied onto LB agar medium containing carbenicillin and cultured at 37°C for 16 hours. Then, the formed *E*. *coli* colonies were suspended using 3 mL of a super broth (SB)-carbenicillin culture. Some of the suspension was stored at -80°C with the addition of 15% glycerol until use, and the remaining portion was re-inoculated into SB-carbenicillin-2% glucose solution and cultured at 37°C. Then, the obtained culture was centrifuged, and biopanning was again repeated three times using the supernatant containing phage particles to obtain and concentrate antigen-specific antibodies.

After repeating the biopanning three times, *E. coli* containing the antibody gene was applied onto LB agar medium containing carbenicillin and cultured at 37°C for 16 hours. The formed *E*. *coli* colonies were inoculated again into SB-carbenicillin-2% glucose solution and cultured at 37°C until the absorbance (at OD 600 nm) reached 0.5. Then, IPTG was added and further cultured at 30°C for 16 hours. Thereafter, periplasmic extraction was performed. From the results, a library pool of antibodies specifically binding to the CD300c antigen was primarily obtained.

### Example 1.2. Selection of Anti-CD300c Monoclonal Antibodies

In order to select anti-CD300c monoclonal antibodies specifically binding, with high binding affinity, to a CD300c antigen, ELISA was performed using the library pool obtained by the same method as in Example 1.1. More specifically, each of a CD300c antigen and a CD300a antigen in a coating buffer (0.1 M sodium carbonate, pH 9.0) was dispensed into an ELISA plate at a concentration of 5 µg/mL per well, followed by incubation at room temperature for 3 hours, to allow the antigen to bind to the plate. The unbound antigens were removed by washing three times using phosphate buffered saline-Tween 20 (PBST), and then 350 µL of PBST supplemented with 2% bovine serum albumin (BSA) was added to each well, followed by incubation at room temperature for 1 hour and then washing again using PBST. Then, 25 µg of a periplasmic extract containing scFv obtained by the same method as in Example 1.1 was added thereto, followed by incubation for 1 hour at room temperature for antigen binding. After 1 hour, the unbound scFv was removed by washing three times using PBST, and then 4 µg/mL of an antibody for detection was added, followed by incubation again at room temperature for 1 hour. Subsequently, the unbound antibodies for detection were removed using PBST, and then HRP-conjugated anti-rabbit IgG was added, followed by incubation at room temperature for 1 hour. The unbound antibodies were removed again using PBST. Subsequently, 3,3',5,5'-tetramethylbenzidine (TMB) solution was added, followed by incubation for 10 minutes for development. Then, 2 N sulfuric acid solution was added to terminate the development reaction, and the absorbance was measured at 450 nm to identify the antibodies specifically binding to the CD300c antigen.

### Example 1.3. Identification of Anti-CD300c Monoclonal Antibody

### Sequences

The nucleotide sequences of the anti-CD300c monoclonal antibodies selected using the same method as in Example 1.2 were identified. More specifically, for each of the selected antibody clones, plasmid DNA was extracted therefrom using a plasmid miniprep kit. Then, DNA sequencing was performed to analyze complementarity-determining region (CDR) sequences. As a result, 25 types of anti-CD300c monoclonal antibodies having different amino acid sequences were obtained. The heavy chain and light chain variable regions of these 25 types of anti-CD300c monoclonal antibodies are shown in Tables 4 and 5 below.

**[Table 4]**

| Antibody name | Source (phage library) | Heavy chain variable region (nucleic acid) | Light chain variable region (nucleic acid) | Heavy chain variable region (amino acid) | Light chain variable region (amino acid) |
|---|---|---|---|---|---|
| CK1 | Kappa | FIG. 1aa (SEQ ID: 301) | FIG. 1ab (SEQ ID: 302) | FIG. 1ac (SEQ ID: 303) | FIG. 1ad (SEQ ID: 304) |
| CK2 | Kappa | FIG. 1ba (SEQ ID: 305) | FIG. 1bb (SEQ ID: 306) | FIG. 1bc (SEQ ID: 307) | FIG. 1bd (SEQ ID: 308) |
| CK3 | Kappa | FIG. 1ca (SEQ ID: 309) | FIG. 1cb (SEQ ID: 310) | FIG. 1cc (SEQ ID: 311) | FIG. 1cd (SEQ ID: 312) |
| CL4 | Lambda | FIG. 1da (SEQ ID: 313) | FIG. 1db (SEQ ID: 314) | FIG. 1dc (SEQ ID: 315) | FIG. 1dd (SEQ ID: 316) |
| CL5 | Lambda | FIG. 1ea (SEQ ID: 317) | FIG. 1eb (SEQ ID: 318) | FIG. 1ec (SEQ ID: 319) | FIG. 1ed (SEQ ID: 320) |
| CL6 | VH3VL1 | FIG. 1fa (SEQ ID: 321) | FIG. 1fb (SEQ ID: 322) | FIG. 1fc (SEQ ID: 323) | FIG. 1fd (SEQ ID: 324) |
| CL7 | VH3VL1 | FIG. 1ga (SEQ ID: 325) | FIG. 1gb (SEQ ID: 326) | FIG. 1gc (SEQ ID: 327) | FIG. 1gd (SEQ ID: 328) |
| CL8 | VH3VL1 | FIG. 1ha (SEQ ID: 329) | FIG. 1hb (SEQ ID: 330) | FIG. 1hc (SEQ ID: 331) | FIG. 1hd (SEQ ID: 332) |
| CL9 | VH3VL1 | FIG. 1ia (SEQ ID: 333) | FIG. 1ib (SEQ ID: 334) | FIG. 1 ic (SEQ ID: 335) | FIG. 1id (SEQ ID: 336) |
| CL10 | VH3VL1 | FIG. 1ja (SEQ ID: 337) | FIG. 1jb (SEQ ID: | FIG. 1jc (SEQ ID: | FIG. 1jd (SEQ ID: |
| | | | 338) | 339) | 340) |
| SK11 | Kappa | FIG. 1ka (SEQ ID: 341) | FIG. 1kb (SEQ ID: 342) | FIG. 1kc (SEQ ID: 343) | FIG. 1kd (SEQ ID: 344) |
| SK12 | Kappa | FIG. 1la (SEQ ID: 345) | FIG. 1lb (SEQ ID: 346) | FIG. 1lc (SEQ ID: 347) | FIG. 1ld (SEQ ID: 348) |
| SK13 | Kappa | FIG. 1ma (SEQ ID: 349) | FIG. 1mb (SEQ ID: 350) | FIG. 1mc (SEQ ID: 351) | FIG. 1md (SEQ ID: 352) |
| SK14 | Kappa | FIG. 1na (SEQ ID: 353) | FIG. 1nb (SEQ ID: 354) | FIG. 1nc (SEQ ID: 355) | FIG. 1nd (SEQ ID: 356) |
| SK15 | Kappa | FIG. 1oa (SEQ ID: 357) | FIG. 1ob (SEQ ID: 358) | FIG. 1oc (SEQ ID: 359) | FIG. 1od (SEQ ID: 360) |
| SK16 | Kappa | FIG. 1pa (SEQ ID: 361) | FIG. 1pb (SEQ ID: 362) | FIG. 1pc (SEQ ID: 363) | FIG. 1pd (SEQ ID: 364) |
| SK17 | Kappa | FIG. 1qa (SEQ ID: 365) | FIG. 1qb (SEQ ID: 366) | FIG. 1qc (SEQ ID: 367) | FIG. 1qd (SEQ ID: 368) |

**Table 5]**

| Antibody name | Source (phage library) | Heavy chain variable region (nucleic acid) | Light chain variable region (nucleic acid) | Heavy chain variable region (amino acid) | Light chain variable region (amino acid) |
|---|---|---|---|---|---|
| SL18 | Lambda | FIG. 1ra (SEQ ID: 369) | FIG. 1rb (SEQ ID: 370) | FIG. 1rc (SEQ ID: 371) | FIG. 1rd (SEQ ID: 372) |
| CB301_H3L1_A10 | VH3VL1 | FIG. 1sa (SEQ ID: 373) | FIG. 1sb (SEQ ID: 374) | FIG. 1sc (SEQ ID: 375) | FIG. 1sd (SEQ ID: 376) |
| CB301_H3L1_A12 | VH3VL1 | FIG. 1ta (SEQ ID: 377) | FIG. 1tb (SEQ ID: 378) | FIG. 1tc (SEQ ID: 379) | FIG. 1td (SEQ ID: 380) |
| CB301_H3L1_E6 | VH3VL1 | FIG. 1ua (SEQ ID: 381) | FIG. 1ub (SEQ ID: 382) | FIG. 1uc (SEQ ID: 383) | FIG. 1ud (SEQ ID: 384) |
| CB301_H3L1_F4 | VH3VL1 | FIG. 1va (SEQ ID: 385) | FIG. 1vb (SEQ ID: 386) | FIG. 1vc (SEQ ID: 387) | FIG. 1vd (SEQ ID: 388) |
| CB301_H3L1_G11 | VH3VL1 | FIG. 1wa (SEQ ID: 389) | FIG. 1wb (SEQ ID: 390) | FIG. 1wc (SEQ ID: 391) | FIG. 1wd (SEQ ID: 392) |
| CB301_QPALTL_B5 | OPALTL | FIG. 1xa (SEQ ID: 393) | FIG. 1xb (SEQ ID: 394) | FIG. 1xc (SEQ ID: 395) | FIG. 1xd (SEQ ID: 396) |
| CB301_QPALTL_E6 | OPALTL | FIG. 1ya (SEQ ID: 397) | FIG. 1yb (SEQ ID: 398) | FIG. 1yc (SEQ ID: 399) | FIG. 1yd (SEQ ID: 400) |

In each of the drawings mentioned in Tables 4 and 5 above, the CDR regions (CDR1, CDR2, and CDR3) are underlined and appear sequentially (that is, CDR1 appears, followed by CDR2, and then CDR3). In addition, the CDR regions included in the heavy chain or light chain variable region shown in each drawing are as set forth in SEQ ID Nos as shown in Table 6 below.

**Table 6]**

| Related drawing | Antibod y | Heavy chain/ Light chain | Amino acid/ nucleic acid | CDR1 | CDR2 | CDR3 |
|---|---|---|---|---|---|---|
| FIG. 1aa | CK1 | heavy chain | nucleic acid | SEQ ID: 1 | SEQ ID: 2 | SEQ ID: 3 |
| FIG. 1ab | | light chain | nucleic acid | SEQ ID: 4 | SEQ ID: 5 | SEQ ID: 6 |
| FIG. 1ac | | heavy chain | amino acid | SEQ ID: 7 | SEQ ID: 8 | SEQ ID: 9 |
| FIG. 1ad | | light chain | amino acid | SEQ ID: 10 | SEQ ID: 11 | SEQ ID: 12 |
| FIG. 1ba | CK2 | heavy chain | nucleic acid | SEQ ID: 13 | SEQ ID: 14 | SEQ ID: 15 |
| FIG. 1bb | | light chain | nucleic acid | SEQ ID: 16 | SEQ ID: 17 | SEQ ID: 18 |
| FIG. 1bc | | heavy chain | amino acid | SEQ ID: 19 | SEQ ID: 20 | SEQ ID: 21 |
| FIG. 1bd | | light chain | amino acid | SEQ ID: 22 | SEQ ID: 23 | SEQ ID: 24 |
| FIG. 1ca | CK3 | heavy chain | nucleic acid | SEQ ID: 25 | SEQ ID: 26 | SEQ ID: 27 |
| FIG. 1cb | | light chain | nucleic acid | SEQ ID: 28 | SEQ ID: 29 | SEQ ID: 30 |
| FIG. 1cc | | heavy chain | amino acid | SEQ ID: 31 | SEQ ID: 32 | SEQ ID: 33 |
| FIG. 1cd | | light chain | amino acid | SEQ ID: 34 | SEQ ID: 35 | SEQ ID: 36 |
| FIG. 1da | CL4 | heavy chain | nucleic acid | SEQ ID: 37 | SEQ ID: 38 | SEQ ID: 39 |
| FIG. 1db | | light chain | nucleic acid | SEQ ID: 40 | SEQ ID: 41 | SEQ ID: 42 |
| FIG. 1dc | | heavy chain | amino acid | SEQ ID: 43 | SEQ ID: 44 | SEQ ID: 45 |
| FIG. 1dd | | light chain | amino acid | SEQ ID: 46 | SEQ ID: 47 | SEQ ID: 48 |
| FIG. 1ea | CL5 | heavy chain | nucleic acid | SEQ ID: 49 | SEQ ID: 50 | SEQ ID: 51 |
| FIG. 1eb | | light chain | nucleic acid | SEQ ID: 52 | SEQ ID: 53 | SEQ ID: 54 |
| FIG. 1ec | | heavy chain | amino acid | SEQ ID: 55 | SEQ ID: 56 | SEQ ID: 57 |
| FIG. 1ed | | light chain | amino acid | SEQ ID: 58 | SEQ ID: 59 | SEQ ID: 60 |
| FIG. 1fa | CL6 | heavy chain | nucleic acid | SEQ ID: 61 | SEQ ID: 62 | SEQ ID: 63 |
| FIG. 1fb | | light chain | nucleic acid | SEQ ID: 64 | SEQ ID: 65 | SEQ ID: 66 |
| FIG. 1fc | | heavy chain | amino acid | SEQ ID: 67 | SEQ ID: 68 | SEQ ID: 69 |
| FIG. 1fd | | light chain | amino acid | SEQ ID: 70 | SEQ ID: 71 | SEQ ID: 72 |
| FIG. 1ga | CL7 | heavy chain | nucleic acid | SEQ ID: 73 | SEQ ID: 74 | SEQ ID: 75 |
| FIG. 1gb | | light chain | nucleic acid | SEQ ID: 76 | SEQ ID: 77 | SEQ ID: 78 |
| FIG. 1gc | | heavy chain | amino acid | SEQ ID: 79 | SEQ ID: 80 | SEQ ID: 81 |
| FIG. 1gd | | light chain | amino acid | SEQ ID: 82 | SEQ ID: 83 | SEQ ID: 84 |
| FIG. 1ha | CL8 | heavy chain | nucleic acid | SEQ ID: 85 | SEQ ID: 86 | SEQ ID: 87 |
| FIG. 1hb | | light chain | nucleic acid | SEQ ID: 88 | SEQ ID: 89 | SEQ ID: 90 |
| FIG. 1hc | | heavy chain | amino acid | SEQ ID: 91 | SEQ ID: 92 | SEQ ID: 93 |
| FIG. 1hd | | light chain | amino acid | SEQ ID: 94 | SEQ ID: 95 | SEQ ID: 96 |
| FIG. 1ia | CL9 | heavy chain | nucleic acid | SEQ ID: 97 | SEQ ID: 98 | SEQ ID: 99 |
| FIG. 1ib | | light chain | nucleic acid | SEQ ID: 100 | SEQ ID: 101 | SEQ ID: 102 |
| FIG. 1ic | | heavy chain | amino acid | SEQ ID: 103 | SEQ ID: 104 | SEQ ID: 105 |
| FIG. 1id | | light chain | amino acid | SEQ ID: 106 | SEQ ID: 107 | SEQ ID: 108 |
| FIG. 1ja | CL10 | heavy chain | nucleic acid | SEQ ID: 109 | SEQ ID: 110 | SEQ ID: 111 |
| FIG. 1jb | | light chain | nucleic acid | SEQ ID: 112 | SEQ ID: 113 | SEQ ID: 114 |
| FIG. 1jc | | heavy chain | amino acid | SEQ ID: 115 | SEQ ID: 116 | SEQ ID: 117 |
| FIG. 1jd | | light chain | amino acid | SEQ ID: 118 | SEQ ID: 119 | SEQ ID: 120 |
| FIG. 1ka | SK11 | heavy chain | nucleic acid | SEQ ID: 121 | SEQ ID: 122 | SEQ ID: 123 |
| FIG. 1kb | | light chain | nucleic acid | SEQ ID: 124 | SEQ ID: 125 | SEQ ID: 126 |
| FIG. 1kc | | heavy chain | amino acid | SEQ ID: 127 | SEQ ID: 128 | SEQ ID: 129 |
| FIG. 1kd | | light chain | amino acid | SEQ ID: 130 | SEQ ID: 131 | SEQ ID: 132 |
| FIG. 1la | SK12 | heavy chain | nucleic acid | SEQ ID: 133 | SEQ ID: 134 | SEQ ID: 135 |
| FIG. 1lb | | light chain | nucleic acid | SEQ ID: 136 | SEQ ID: 137 | SEQ ID: 138 |
| FIG. 1lc | | heavy chain | amino acid | SEQ ID: 139 | SEQ ID: 140 | SEQ ID: 141 |
| FIG. 1ld | | light chain | amino acid | SEQ ID: 142 | SEQ ID: 143 | SEQ ID: 144 |
| FIG. 1ma | SK13 | heavy chain | nucleic acid | SEQ ID: 145 | SEQ ID: 146 | SEQ ID: 147 |
| FIG. 1mb | | light chain | nucleic acid | SEQ ID: 148 | SEQ ID: 149 | SEQ ID: 150 |
| FIG. 1mc | | heavy chain | amino acid | SEQ ID: 151 | SEQ ID: 152 | SEQ ID: 153 |
| FIG. 1md | | light chain | amino acid | SEQ ID: 154 | SEQ ID: 155 | SEQ ID: 156 |
| FIG. 1na | SK14 | heavy chain | nucleic acid | SEQ ID: 157 | SEQ ID: 158 | SEQ ID: 159 |
| FIG. 1nb | | light chain | nucleic acid | SEQ ID: 160 | SEQ ID: 161 | SEQ ID: 162 |
| FIG. 1nc | | heavy chain | amino acid | SEQ ID: 163 | SEQ ID: 164 | SEQ ID: 165 |
| FIG. 1nd | | light chain | amino acid | SEQ ID: 166 | SEQ ID: 167 | SEQ ID: 168 |
| FIG. 1oa | SK15 | heavy chain | nucleic acid | SEQ ID: 169 | SEQ ID: 170 | SEQ ID: 171 |
| FIG. 1ob | | light chain | nucleic acid | SEQ ID: 172 | SEQ ID: 173 | SEQ ID: 174 |
| FIG. 1oc | | heavy chain | amino acid | SEQ ID: 175 | SEQ ID: 176 | SEQ ID: 177 |
| FIG. 1od | | light chain | amino acid | SEQ ID: 178 | SEQ ID: 179 | SEQ ID: 180 |
| FIG. 1pa | SK16 | heavy chain | nucleic acid | SEQ ID: 181 | SEQ ID: 182 | SEQ ID: 183 |
| FIG. 1pb | | light chain | nucleic acid | SEQ ID: 184 | SEQ ID: 185 | SEQ ID: 186 |
| FIG. 1pc | | heavy chain | amino acid | SEQ ID: 187 | SEQ ID: 188 | SEQ ID: 189 |
| FIG. 1pd | | light chain | amino acid | SEQ ID: 190 | SEQ ID: 191 | SEQ ID: 192 |
| FIG. 1qa | SK17 | heavy chain | nucleic acid | SEQ ID: 193 | SEQ ID: 194 | SEQ ID: 195 |
| FIG. 1qb | | light chain | nucleic acid | SEQ ID: 196 | SEQ ID: 197 | SEQ ID: 198 |
| FIG. 1qc | | heavy chain | amino acid | SEQ ID: 199 | SEQ ID: 200 | SEQ ID: 201 |
| FIG. 1qd | | light chain | amino acid | SEQ ID: 202 | SEQ ID: 203 | SEQ ID: 204 |
| FIG. 1ra | SL18 | heavy chain | nucleic acid | SEQ ID: 205 | SEQ ID: 206 | SEQ ID: 207 |
| FIG. 1rb | | light chain | nucleic acid | SEQ ID: 208 | SEQ ID: 209 | SEQ ID: 210 |
| FIG. 1rc | | heavy chain | amino acid | SEQ ID: 211 | SEQ ID: 212 | SEQ ID: 213 |
| FIG. 1rd | | light chain | amino acid | SEQ ID: 214 | SEQ ID: 215 | SEQ ID: 216 |
| FIG. 1sa | CB301_ H3L1_A 10 | heavy chain | nucleic acid | SEQ ID: 217 | SEQ ID: 218 | SEQ ID: 219 |
| FIG. 1sb | | light chain | nucleic acid | SEQ ID: 220 | SEQ ID: 221 | SEQ ID: 222 |
| FIG. 1sc | | heavy chain | amino acid | SEQ ID: 223 | SEQ ID: 224 | SEQ ID: 225 |
| FIG. 1sd | | light chain | amino acid | SEQ ID: 226 | SEQ ID: 227 | SEQ ID: 228 |
| FIG. 1ta | CB301_ H3L1_A 12 | heavy chain | nucleic acid | SEQ ID: 229 | SEQ ID: 230 | SEQ ID: 231 |
| FIG. 1tb | | light chain | nucleic acid | SEQ ID: 232 | SEQ ID: 233 | SEQ ID: 234 |
| FIG. 1tc | | heavy chain | amino acid | SEQ ID: 235 | SEQ ID: 236 | SEQ ID: 237 |
| FIG. 1td | | light chain | amino acid | SEQ ID: 238 | SEQ ID: 239 | SEQ ID: 240 |
| FIG. 1ua | CB301_ H3L1_E 6 | heavy chain | nucleic acid | SEQ ID: 241 | SEQ ID: 242 | SEQ ID: 243 |
| FIG. 1ub | | light chain | nucleic acid | SEQ ID: 244 | SEQ ID: 245 | SEQ ID: 246 |
| FIG. 1uc | | heavy chain | amino acid | SEQ ID: 247 | SEQ ID: 248 | SEQ ID: 249 |
| FIG. 1ud | | light chain | amino acid | SEQ ID: 250 | SEQ ID: 251 | SEQ ID: 252 |
| FIG. 1va | CB301_ H3L1_F 4 | heavy chain | nucleic acid | SEQ ID: 253 | SEQ ID: 254 | SEQ ID: 255 |
| FIG. 1vb | | light chain | nucleic acid | SEQ ID: 256 | SEQ ID: 257 | SEQ ID: 258 |
| FIG. 1vc | | heavy chain | amino acid | SEQ ID: 259 | SEQ ID: 260 | SEQ ID: 261 |
| FIG. 1vd | | light chain | amino acid | SEQ ID: 262 | SEQ ID: 263 | SEQ ID: 264 |
| FIG. 1wa | CB301_ H3L1_G 11 | heavy chain | nucleic acid | SEQ ID: 265 | SEQ ID: 266 | SEQ ID: 267 |
| FIG. 1wb | | light chain | nucleic acid | SEQ ID: 268 | SEQ ID: 269 | SEQ ID: 270 |
| FIG. 1wc | | heavy chain | amino acid | SEQ ID: 271 | SEQ ID: 272 | SEQ ID: 273 |
| FIG. 1wd | | light chain | amino acid | SEQ ID: 274 | SEQ ID: 275 | SEQ ID: 276 |
| FIG. 1xa | CB301_ OPALTL _B5 | heavy chain | nucleic acid | SEQ ID: 277 | SEQ ID: 278 | SEQ ID: 279 |
| FIG. 1xb | | light chain | nucleic acid | SEQ ID: 280 | SEQ ID: 281 | SEQ ID: 282 |
| FIG. 1xc | | heavy chain | amino acid | SEQ ID: 283 | SEQ ID: 284 | SEQ ID: 285 |
| FIG. 1xd | | light chain | amino acid | SEQ ID: 286 | SEQ ID: 287 | SEQ ID: 288 |
| FIG. 1ya | CB301_ OPALTL _E6 | heavy chain | nucleic acid | SEQ ID: 289 | SEQ ID: 290 | SEQ ID: 291 |
| FIG. 1yb | | light chain | nucleic acid | SEQ ID: 292 | SEQ ID: 293 | SEQ ID: 294 |
| FIG. 1yc | | heavy chain | amino acid | SEQ ID: 295 | SEQ ID: 296 | SEQ ID: 297 |
| FIG. 1yd | | light chain | amino acid | SEQ ID: 298 | SEQ ID: 299 | SEQ ID: 300 |

As described above, 25 types of anti-CD300c monoclonal antibodies were identified that specifically bind, with high binding affinity, to the CD300c antigen and can be used for the prevention or treatment of cancer.

### Example 1.4. Production and Purification of Anti-CD300c Monoclonal Antibodies

By using each of the nucleotide sequences of the anti-CD300c monoclonal antibodies identified in Example 1.3, expression vector with separated heavy and light chains capable of expressing antibodies were constructed. More specifically, the expression vectors were constructed by inserting genes into the pCIW3.3 vectors using the analyzed CDR sequences so that the vectors can express the heavy and light chains, respectively. The constructed expression vectors for heavy and light chains were mixed with polyethylenimine (PEI) at a mass ratio of 1:1 and transfected into 293T cells to induce antibody expression. Then, on day 8, the culture was centrifuged to remove the cells to obtain the resulting culture. The obtained culture was filtered, and then resuspended using a mixed solution of 0.1 M NaH₂PO₄ and 0.1 M Na₂HPO₄ (pH 7.0). The resuspended solution was purified through affinity chromatography using protein A beads (GE Healthcare), and finally eluted using an elution buffer (Thermofisher).

In order to identify the produced antibody, 5 µg of purified antibody was subjected to the addition of a reducing sample buffer and a non-reducing sample buffer, followed by electrophoresis using pre-made SDS-PAGE (Invitrogen). Then, the proteins were stained using Coomassie Blue. The results obtained under non-reducing conditions are illustrated in FIG. 2, and the results obtained under reducing conditions are illustrated in FIG. 3.

As shown in FIGS. 2 and 3, it was identified that anti-CD300c monoclonal antibodies having a high purity were produced and purified.

### Experimental Example 1. Expression of CD300c in Cancer Cells and Immune Cells

### Experimental Example 1.1. Identification of Expression of CD300c in Cancer Cell Line

In order to evaluate whether CD300c is expressed in various cancer cells, various cancer cell lines, such as MKN45 (human gastric cancer cell line), IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line), were cultured to evaluate the expression of CD300c at the mRNA and protein levels. In addition, the immune cell THP-1 cells (human monocyte cell line) were also evaluated. Specifically, HEK293T (normal cell line) was used as a control group.

Meanwhile, the expression of the protein was identified by Western blot and flow cytometry (FACS) of fluorescently labeled cells. Specifically, each cultured cell line was fixed with 4% formaldehyde and then blocked using 5% normal bovine serum albumin. Then, the cells were stained using 0.5 µg of eFluor660-labeled anti-CD300c antibody (Invitrogen). Subsequently, the fluorescently labeled cells were identified using flow cytometry (FACS).

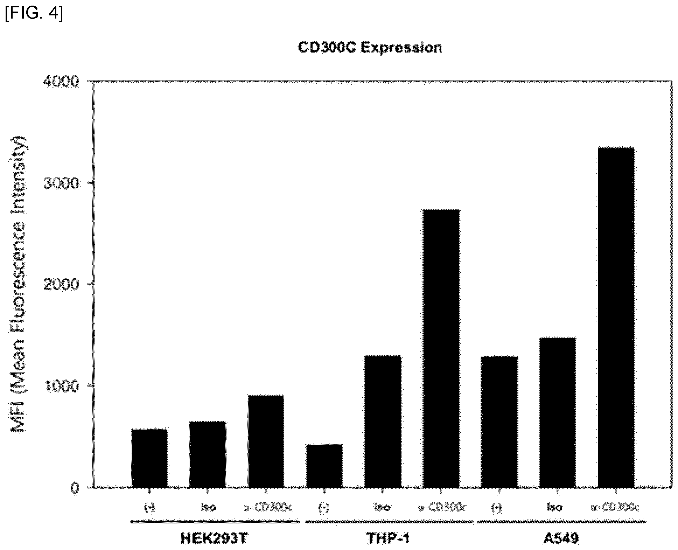

As a result, it was identified that the CD300c antigen was expressed at the mRNA and protein levels in various cancer cells, such as colon cancer, lung cancer, and breast cancer. In addition, as shown in FIG. 4, as a result of analysis using flow cytometry (FACS), it was identified that a significantly high expression of CD300c was observed in the human lung cancer cell line (A549) and the human monocyte cell line (THP-1) compared with the normal cell line (HEK293T).

### Experimental Example 1.2. Identification of Expression of CD300c in Cancer Tissue and Immune Cells (I)

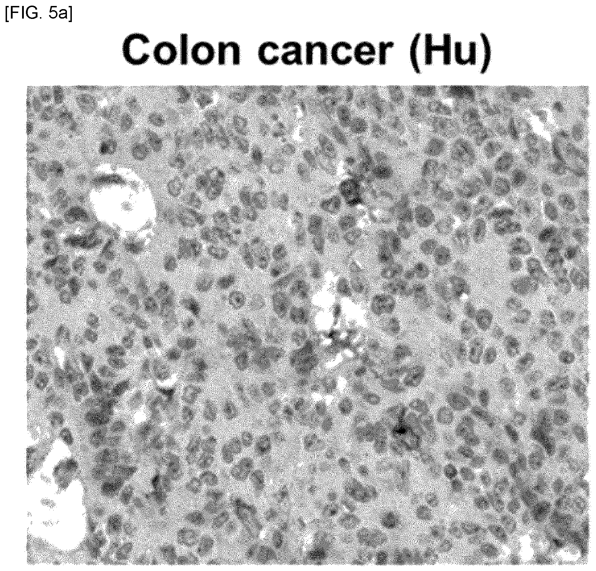

In order to identify the expression of CD300c in the cancer tissue of a patient, tissue microarray was performed as follows. The tissue of a colon cancer patient was fixed with formalin and embedded into a paraffin block. Then, the paraffin block was cut into sections with a diameter of 2.0 mm and a thickness of 3 to 5 um using a microtome. Then, the sections were attached to slides in a certain direction and then dried. The cancer tissue was stained with H&E staining, and then treated with an anti-CD300c antibody (Invitrogen) at 1:500 to stain CD300c. As a result, as shown in FIG. 5a, it was identified that CD300c was expressed in the colon cancer tissue of the patient.

In order to identify whether CD300c is expressed not only in the tissue of the colon cancer patient but also in immune cells within the cancer tissue, 2×10⁵ CT26 cells were transplanted into 8-week-old BALB/c mice by subcutaneous injection. On day 25 (D25) after tumor transplantation, the mice were sacrificed, and tumor tissue was collected from 6 mice of the control group not having been administered the anti-CD300c antibody. The collected tumor tissue was incubated for 1 hour at 37°C in a mixed solution of collagenase D (20 mg/mL) and Dnase I (2 mg/mL). Then, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability (from Invitrogen), and antibodies to the total macrophage markers F4/80 (from Abcam), CD11b (from Abcam), CD11c (from Abcam), CD3 (from Abcam), CD4 (from Thermofisher) and CD8 (from Thermofisher), and a CD300c antibody (from Sino Biological). Then, the data were read with CytoFLEX flow cytometer and analyzed with FlowJo software.

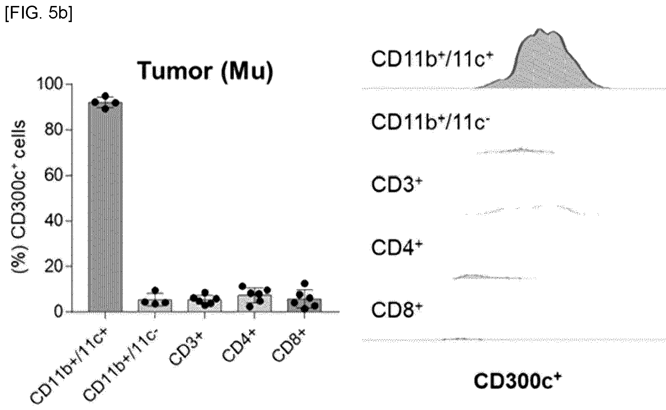

As a result, as shown in FIG. 5b, it was identified that immune cells expressing CD300c were present in the mouse tumor tissue. These were immune cells expressing both CD11b and CD11c markers, and examples thereof include dendritic cells and macrophages. These results identified that CD300c was expressed in both cancer tissue and immune cells.

### Experimental Example 1.3. Identification of Expression of CD300c in Cancer Tissue and Immune Cells (II)

In order to identify the expression of CD300c in human immune tissue and cancer cells, tissue microarray was performed as follows. The normal tonsil tissue and the tissue of a colon cancer patient were fixed with formalin and embedded into a paraffin block. Subsequently, in the normal tonsil tissue and the tissue of the colon cancer patient, the locations for performing tissue microarray were determined, and then each paraffin block was cut into sections with a diameter of 2.0 mm and a thickness of 3 to 5 µm using a microtome. Then, the sections were attached to slides in a certain direction and dried. The cancer tissue was stained with H&E staining and then treated with an anti-CD300c antibody (Invitrogen) at 1:500 to stain CD300c.

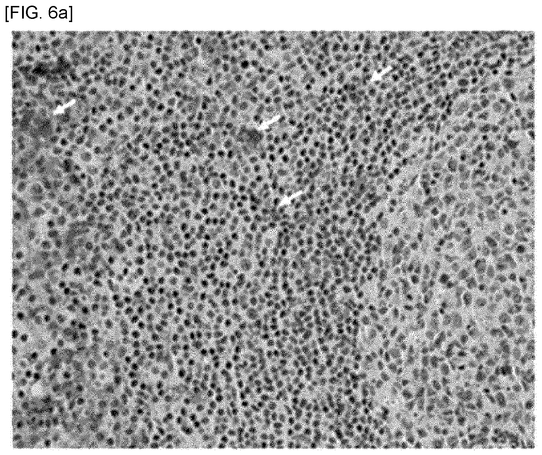

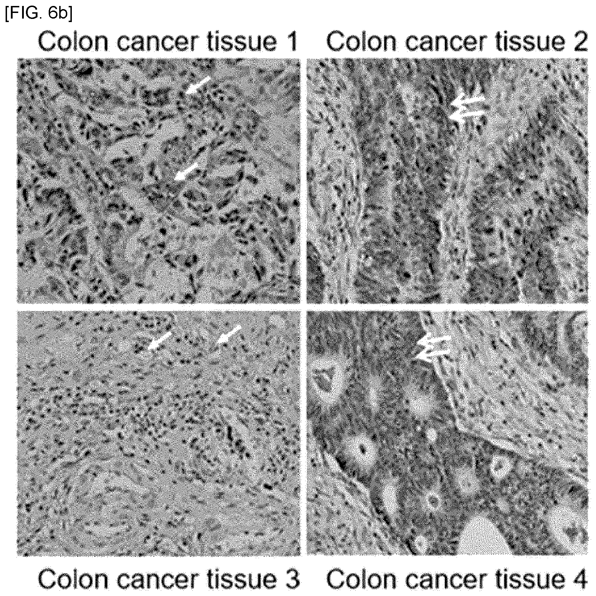

As a result, as shown in FIGS. 6a and 6b, it was identified that CD300c was expressed in both the normal tonsil tissue (FIG. 6a), which is an immune tissue, and the colon cancer tissue (FIG. 6b) of the patient. Since a large number of immune cells such as T cells and monocytes are distributed in the tonsils, the expression of CD300c in the tonsil tissue means that CD300c is expressed in immune cells. As in Experimental Example 1.2, it was identified in this experimental example that CD300c was expressed in the tissue of the colon cancer patient; however, this experimental example is meaningful in that expression of CD300c was observed in tissues of all 4 colon cancer patients, indicating that CD300c was expressed in a large number of colon cancer tissues.

### Experimental Example 2. Identification of Recognition of CD300c Antigen by Anti-CD300c Monoclonal Antibodies and Binding thereof to CD300c Antigen

### Experimental Example 2.1. Identification of Antigen-Binding Affinity of Anti-CD300c Monoclonal Antibodies

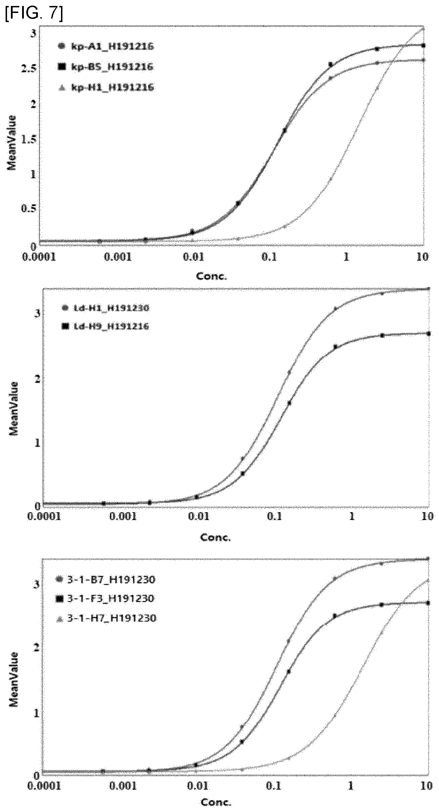

In order to identify the antigen-binding ability of the anti-CD300c monoclonal antibodies produced in Example 1, binding ELISA was performed. Specifically, a CD300c antigen (11832-H08H, Sino Biological) or a CD300a antigen (12449-H08H, Sino Biological) in a coating buffer (0.1 M sodium carbonate, pH 9.0) was dispensed into an ELISA plate at a concentration of 8 µg/mL per well, followed by incubation at room temperature for 3 hours to allow the antigen to be bound to the plate. The unbound antigens were removed by washing three times using phosphate buffered saline-Tween 20 (PBST), and then 300 µL of PBST supplemented with 5% bovine serum albumin (BSA) was added to each well, followed by incubation at room temperature for 1 hour and then washing using PBST. Then, an anti-CD300c monoclonal antibody was diluted in quadruplicate and added thereto, followed by incubation for 1 hour at room temperature for antigen binding. After 1 hour, washing was performed three times using PBST to remove unbound anti-CD300c monoclonal antibody, and then 4 µg/mL of an antibody for detection (HRP conjugated anti-Fc IgG) was added, followed by incubation again at room temperature for 1 hour. Subsequently, the unbound antibody for detection was removed using PBST, and then TMB solution was added, followed by incubation for 10 minutes for development. Then, 2 N sulfuric acid solution was added to terminate the development reaction, and the absorbance was measured at 450 nm to identify the antibodies specifically binding to the CD300c antigen. The results are shown in Table 7 and FIG. 7.

**Table 7]**

| CB301 antibody | EC50 (µg/mL) |
|---|---|
| CK1 | 0.056 |
| CK2 | 0.033 |
| CK3 | 0.793 |
| CL4 | 0.031 |
| CL5 | 0.032 |
| CL6 | 0.148 |
| CL7 | 0.047 |
| CL8 | 49.7 |
| CL9 | 0.094 |
| CL10 | 0.039 |
| SK11 | 0.052 |
| SK12 | 0.067 |
| SK13 | 0.044 |
| SK14 | 0.065 |
| SK15 | 14.74 |
| SK16 | 2.42 |
| SK17 | 0.054 |
| SL18 | 0.17 |

As shown in Table 7, as a result of measuring the EC50 (effective concentration of drug that causes 50% of the maximum response) values of the anti-CD300c monoclonal antibodies, it was identified that the remaining all 14 clones except for 4 clones (CK3, CL8, SK15, SK16) exhibited a binding affinity being as high as 0.2 µg/mL or lower, indicating a high binding affinity. In addition, as shown in FIG. 7, it was identified that the anti-CD300c monoclonal antibodies of the present disclosure were bound to the CD300c antigen with high binding affinity even in the sigmoid curves according to the results of binding ELISA.

### Experimental Example 2.2. Identification of Recognition of Cellular Antigen by Anti-CD300c Monoclonal Antibody

In order to identify whether the anti-CD300c monoclonal antibody (CL7) recognizes a cellular antigen, FACS binding was performed.

CD300c was overexpressed in 293T cells (ATCC) and THP-1 cells (ATCC), and then the respective cells were dispensed into respective microcentrifuge tubes at 2×10⁵ cells per tube. Then, the cells were incubated with the anti-CD300c monoclonal antibody, which was serially diluted 3-fold starting from 10 µg/mL, in a CO₂ incubator for 30 minutes, and washed twice with FACS buffer. Then, the cells were incubated with FITC-conjugated anti-human IgG (H+L), which was diluted 1:100 in FACS buffer, in a CO₂ incubator for 30 minutes, and washed twice with FACS buffer. Subsequently, FITC signals were measured with a CytoFLEX instrument manufactured by Beckman Coulter, Inc., and then the MFI values were obtained using CytExpert program. Using the obtained MFI values, sigmoidal curves were drawn by a sigmaplot program to calculate the EC50 (the effective concentration of drug that causes 50% of the maximum response). As a result, an EC50 of 2.7 nM was obtained for the 293T cells and an EC50 of 2.6 nM was obtained for the THP-1 cells.

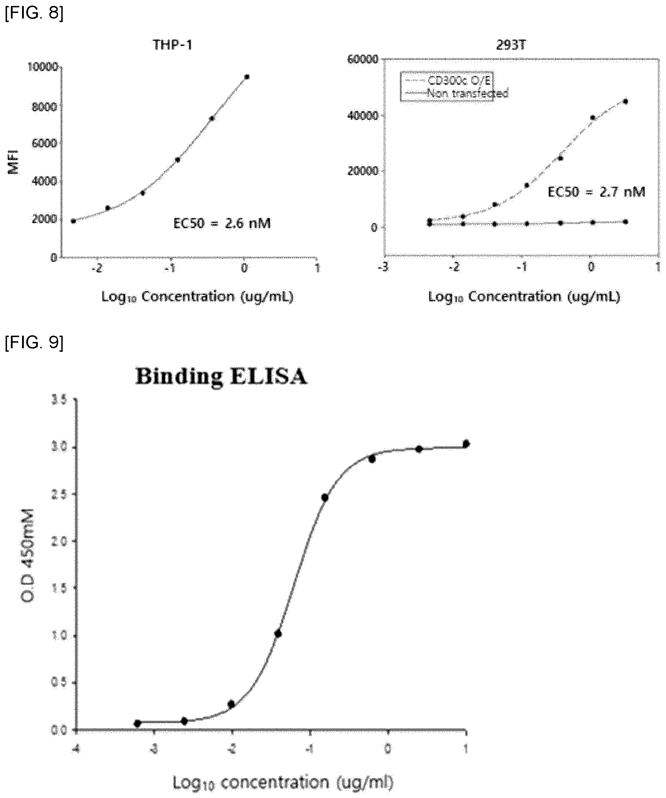

As shown in FIG. 8, in the sigmoidal curves for the result of FACS binding, the anti-CD300c monoclonal antibody produced in Example 1 bound, with strong binding affinity, to CD300c overexpressed on the surface of THP-1 and 293T cells. Therefore, it was identified that the anti-CD300c monoclonal antibody bound to CD300c in an antigen-specific manner.

### Experimental Example 2.3. Identification of Binding Affinity of Anti-CD300c Monoclonal Antibody to CD300c Antigen (I): Binding ELISA

CD300c antigen (250 ug/mL) was diluted to a concentration of 800 ng/mL in coating buffer (0.1 M sodium carbon ate, pH 9.0), placed into a 96-well microplate at 100 µL per well, and incubated at 4°C overnight. The next day, the microplate was washed three times with 200 µL of PBST. Thereafter, 200 µL of a blocking buffer (5% skim milk) was added thereto, followed by blocking at room temperature for 1 hour. The anti-CD300c monoclonal antibody CL7 was diluted to 200 µg/mL in PBS, and the concentration thereof was checked by measurement using Nanodrop (product name: NanoDrop One/One, manufactured by Thermo Fisher Scientific). Subsequently, CL7 was diluted in quadruplicate with PBS starting from 10 µg/mL, and added at 100 µL each, followed by incubation at room temperature for 1 hour. After the incubation, the microplate was washed three times with 200 MI of PBST. A secondary antibody (conjugated anti-Fc IgG) was diluted 1:10,000 in blocking buffer, and added at 100 µL, followed by incubation at room temperature for 1 hour, and then washed three times with 200 µL of PBST. Subsequently, TMB and hydrogen peroxide were mixed 1:1, and added at 100 µL per well, followed by incubation at room temperature for 7 to 9 minutes. Then, 50 µL of 1 N sulfuric acid was added to stop development, and measurement was performed at 450 nm using a microplate reader (product name: Varioskan LUX) to obtain binding affinity results.

As a result, as shown in FIG. 9, it was identified that the anti-CD300c monoclonal antibody bound to CD300c in a concentration-dependent manner, indicating that the anti-CD300c monoclonal antibody has excellent binding affinity and specificity to the antigen CD300c.

### Experimental Example 2.4. Identification of Binding Affinity of Anti-CD300c Monoclonal Antibody to CD300c Antigen (II): Surface Plasmon Resonance (SPR)

In order to identify the binding affinity between the antigen CD300c and the anti-CD300c monoclonal antibody CL7, a surface plasmon resonance experiment was performed.

To immobilize CD300c on a CMS chip, 5 µg/mL of CD300c was diluted in 10 mM acetate buffer (pH 5.5). Then, the flow rates were all set equally to 10 mL/min, and the target RU was respectively set to 300 RU. The antigen was activated by a mixture of 0.2 M EDC and 0.05 M NHS, and blocked with 1 M ethanolamine to perform the immobilization so that the final RU of CD300c became 399.2 RU. Then, CL7 was diluted in PBSP to concentrations of 0, 0.195, 0.39, 0.78, 1.56, 3.125, and 6.25 µg/mL, separately. A kinetics/affinity test was performed with an association time of 240 seconds, a dissociation time of 900 seconds, and a flow rate of 30 µL/min. Subsequently, 50 mM NaOH was allowed to flow at a rate of 30 µL/min for 30 seconds to regenerate the surface.

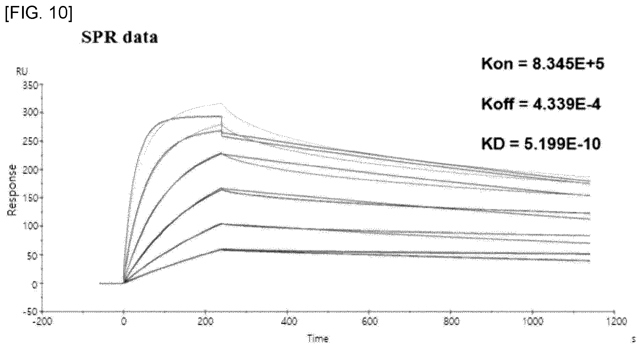

As a result, as shown in FIG. 10, the KD value was analyzed as being 5.199E-10 M, and the binding affinity of the anti-CD300c monoclonal antibody was identified as being 0.52 Nm, corresponding to a subnanomol level. This indicates that the anti-CD300c monoclonal antibody exhibited high binding affinity to the antigen.

### Experimental Example 2.5. Identification of Binding Specificity of Anti-CD300c Monoclonal Antibody to CD300c Antigen (I)

In order to identify that the anti-CD300c monoclonal antibody CL7 specifically binds only to CD300c without binding to other B7 family proteins, binding ELISA was performed. More specifically, each of CD300c antigen, CD300a antigen, or seven B7 family protein antigens (PD-L1 [B7-H1] (Sino Biological), ICOS Ligand[B7-H2] (Sino Biological), CD276[B7-H3] (Sino Biological), B7-H4 (Sino Biological), CD80[B7-1] (Sino Biological), CD86[B7-2] (Sino Biological), CD273[PD-L2] (Sino Biological)) in a coating buffer (0.1 M sodium carbonate, pH 9.0) was coated on an ELISA plate at a concentration of 8 µg/mL per well, and then bound to the plate by incubation at 2°C to 8°C overnight. The unbound antigen was removed by washing with PBST three times, and then 300 mL of blocking buffer (5% skim milk in PBST) was added to each well. Subsequently, blocking was performed at room temperature for 1 hour, followed by washing again with PBST. Thereafter, CL7 was diluted in quadruplicate with PBS, incubated with the antigen at room temperature for 1 hour for antigen binding 1 hour later, and washed three times with PBST. Then, a secondary antibody (HRP-conjugated anti-Fc IgG) diluted to 4 µg/mL in blocking buffer was added thereto, followed by incubation at room temperature for 1 hour. Subsequently, the unbound antibody for detection was removed using PBST, and then a TMB solution was added thereto, followed by incubation for 10 minutes for development. Then, 2 N sulfuric acid solution was added to terminate the development reaction, and the absorbance was measured at 450 nm to identify the antibody specifically binding to the CD300c antigen.

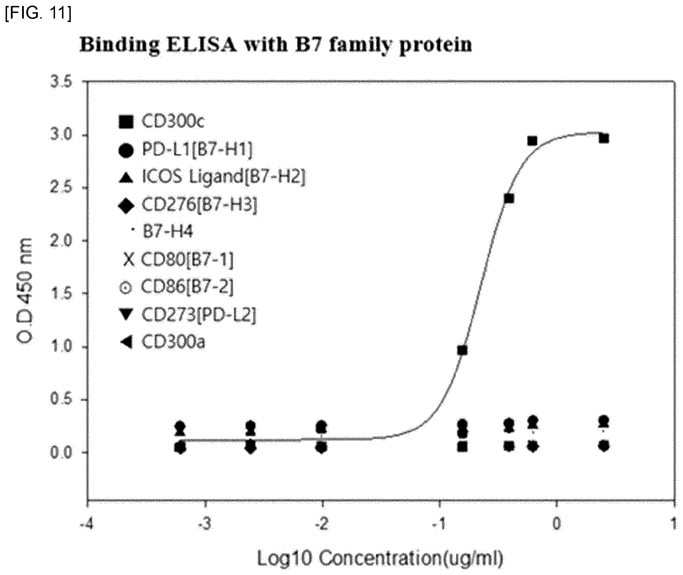

As a result, as shown in FIG. 11, it was identified that the anti-CD300c monoclonal antibody specifically recognized only CD300c without binding to other similar proteins.

### Experimental Example 2.6. Identification of Binding Specificity of Anti-CD300c Monoclonal Antibody to CD300c Antigen (II)

In order to identify specificity of the anti-CD300c monoclonal antibody CL7 to the CD300c antigen, it was further identified whether CL7 exhibits cross-reactivity to the CD300a antigen that has been known to antagonize the CD300c antigen and has a similar protein sequence thereto. More specifically, the CD300a antigen (from Sino Biological) was used to treat at concentrations of 0.039 µg/mL, 0.63 µg/mL, and 10 µg/mL, and then binding ELISA was performed by the same method as in Experimental Example 2.1.

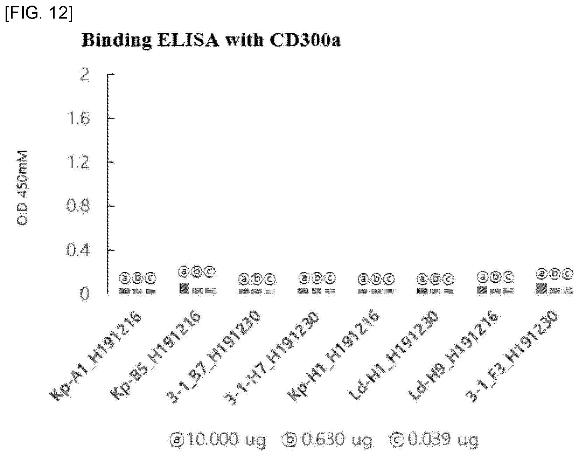

As a result, as shown in FIG. 12, it was identified that the anti-CD300c monoclonal antibody did not bind to antigens other than CD300c and showed high binding specificity only to the CD300c antigen.

### Experimental Example 3. Identification of Anticancer Effects by Administration of Anti-CD300c Monoclonal Antibodies

### Experimental Example 3.1. Identification of Increase in Differentiation Ability into M1 Macrophages in mice

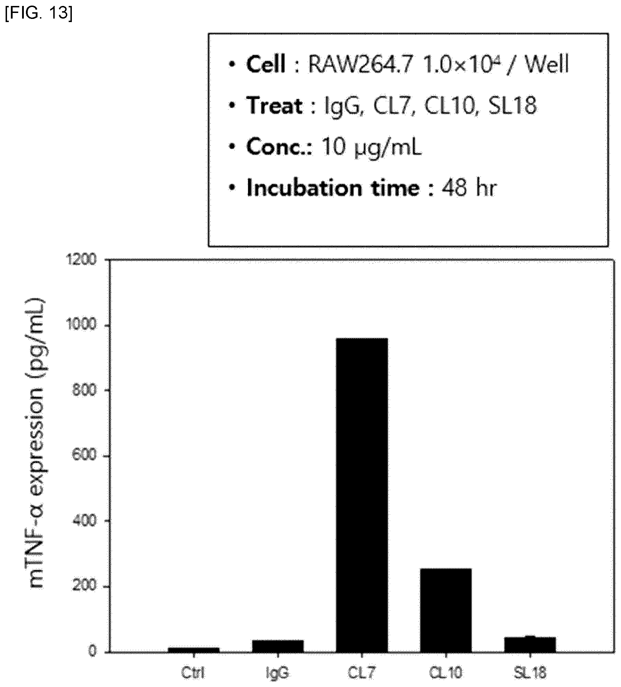

In order to identify whether anti-CD300c monoclonal antibodies can promote the differentiation from mouse macrophages into M1 macrophages, mouse macrophages (Raw264.7) were dispensed into a 96-well plate at a concentration of 1×10⁴ cells/well, and then treated with 10 µg/mL CL7, followed by incubation. Thereafter, the production amount of TNF-α was identified by an ELISA kit (Human TNF-α Quantikine kit, R&D Systems). The results are shown in FIG. 13.

As shown in FIG. 13, it was identified that the production level of TNF-α increased in the experimental groups treated with the anti-CD300c monoclonal antibodies, indicating that the anti-CD300c monoclonal antibodies promoted the differentiation into M1 macrophages in mice.

Referring to Experimental Example 5.2 to be later described, the anti-CD300c monoclonal antibodies also promoted the differentiation into M1 macrophages in humans. It can be therefore seen that the anti-CD300c monoclonal antibodies acted equally in mice as well as humans, and thus had cross-reactivity of promoting the differentiation into M1 macrophages.

### Experimental Example 3.2. Identification of Increase in Tumor-Associated Macrophages (TAMs)

In order to identify effects of the anti-CD300c monoclonal antibody CL7 on tumor-associated macrophages under *in vivo* conditions, a colon cancer cell line (CT26) at 2×10⁵ cells was transplanted by subcutaneous injection into 8-week-old BALB/c mice to prepare syngeneic mouse tumor models. Animal breeding and experiments were all conducted in a specific pathogen free (SPF) facility. In addition, 12 days after transplantation of the colon cancer cell line, the mice with a tumor size of 50 mm³ to 100 mm³ were separately administered the anti-CD300c monoclonal antibody, and for a control group, the mice were injected with the same amount of phosphate buffered saline (PBS). The mice were intraperitoneally injected with 25 mg/kg of each substance twice a week for two weeks over a total of 4 times. On day 25 after the injection, the mice were sacrificed, and the tumor tissue was collected from each of six mice in the CL7 25 mg/kg administration group showing a highest antitumor effect compared with the control group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability (from Invitrogen), and antibodies (from Abcam) to the total macrophage marker F4/80 and the M1 macrophage marker iNOS. Then, the data were read with CytoFLEX flow cytometer and analyzed with FlowJo software.

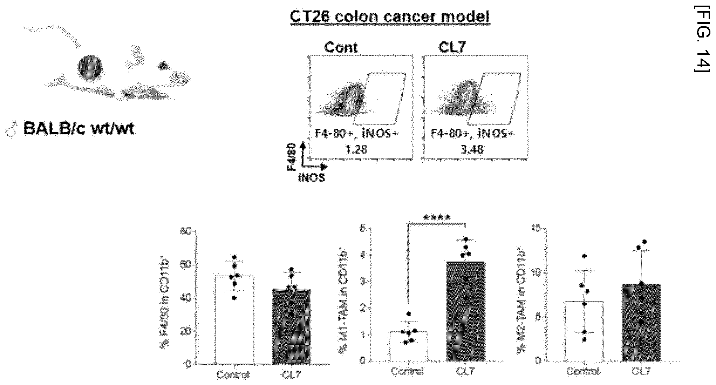

As a result, as shown in FIG. 14, it was identified that as for the treatment with the anti-CD300c monoclonal antibody alone, the expression level of the M1 type tumor-associated macrophages increased in the mouse cancer tissues. This means that the administration of the anti-CD300c monoclonal antibody increased tumor-associated macrophages in cancer tissue, thereby inhibiting cancer growth.

### Experimental Example 3. Identification of Increase in Cytotoxic T Cells

In order to identify effects of the anti-CD300c monoclonal antibody CL7 on CD8+ T cells under *in vivo* conditions, syngeneic mouse tumor models were prepared as in Experimental Example 3.2 and administered the anti-CD300c monoclonal antibody at the same concentration.

On day 25 after the injection, the mice were sacrificed, and the tumor tissue was collected from each of six mice in the CL7 25 mg/kg administration group showing a highest antitumor effect compared with the control group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability and a CD8+ antibody (from Abcam) and a CD4+ antibody (from Abcam). Thereafter, the data were read with CytoFLEX flow cytometer and analyzed with FlowJo software.

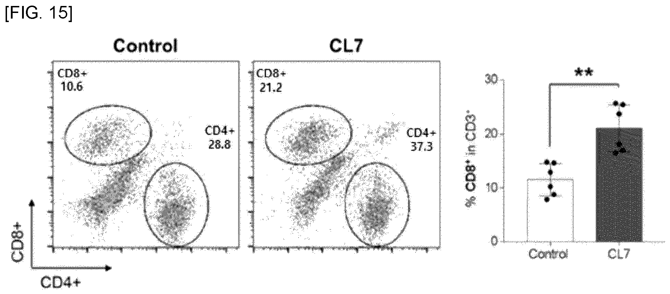

As a result, as shown in FIG. 15, it was identified that as for the treatment with the anti-CD300c monoclonal antibody alone, the number of T cells in tumor increased. This means that the administration of the anti-CD300c monoclonal antibody increased intratumoral cytotoxic T cells, thereby exhibiting a cancer therapeutic effect.

### Experimental Example 3.4. Identification of Tumor-Specific Increase of Cytotoxic T Cells

In order to identify whether the anti-CD300c monoclonal antibody CL7 increases the number of CD8+ T cells in a tumor-specific manner, syngeneic mouse tumor models were prepared as in Experimental Example 3.2 and administered the anti-CD300c monoclonal antibody at the same concentration. On day 25 after the injection, the mice were sacrificed, and the tumor tissue was collected from each of six mice in the CL7 25 mg/kg administration group showing a highest antitumor effect compared with the control group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability and AH1 tetramer antibody (from Abcam). Thereafter, the data were read with CytoFLEX flow cytometer and analyzed with FlowJo software.

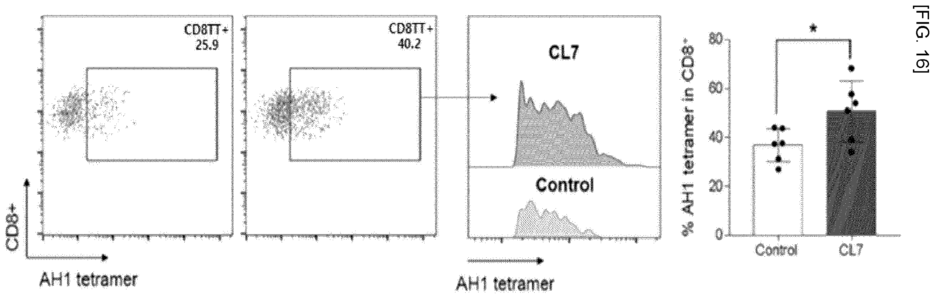

As a result, as shown in FIG. 16, it was identified that as for the treatment with the anti-CD300c monoclonal antibody alone, the expression of AH1 tetramer, which is a CT26 tumor marker factor, increased in CD8+ T cells and thus the number of CD8+ T cells increased in a tumor (CT26)-specific manner. This indicates that the administration of anti-CD300c monoclonal antibody increased CD8+ T cells to target and inhibit CT26 cancer cells.

### Experimental Example 3.5. Identification of Increase in Activity of Cytotoxic T Cells

In order to identify effects of the anti-CD300c monoclonal antibody CL7 on CD8+ T cells under *in vivo* conditions, syngeneic mouse tumor models were prepared as in Experimental Example 3.2 and administered the anti-CD300c monoclonal antibody at the same concentration. On day 25 after injection, the mice were sacrificed, and spleen were collected from each of six mice in the CL7 25 mg/kg administration group showing a highest antitumor effect compared with the control group. Subsequently, the results were identified by measuring IFN-g through ELISPOT assay. Specifically, the Mouse IFN-g ELISpot kit was purchased from R&D Systems (#EL485), and IFN-g was measured according to the protocol for the kit.

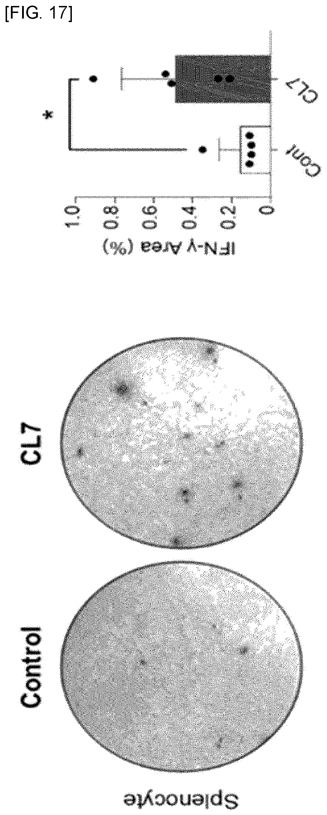

As a result, as shown in FIG. 17, it was identified that as for the treatment with the anti-CD300C monoclonal antibody alone, the expression of IFN-g increased. This indicates that the administration of the anti-CD300C monoclonal antibody alone led to not only an increase in number of CD8+ T cells (see FIG. 15) but also an increase in activity of CD8+ T cells, thereby inhibiting the cancer growth in various ways to exhibit a cancer therapeutic effect.

### Experimental Example 3.6. Identification of Increase in Cytotoxic T Cells Compared with Regulatory T Cells

In order to identify effects of the anti-CD300c monoclonal antibody CL7 on an increase in cytotoxic T cells compared with regulatory T cells under *in vivo* conditions, syngeneic mouse tumor models were prepared as in Experimental Example 3.2 and administered the anti-CD300c monoclonal antibody at the same concentration. On day 25 after the injection, the mice were sacrificed, and the tumor tissue was collected from each of six mice in the CL7 25 mg/kg administration group showing a highest antitumor effect compared with the control group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability, antibodies to the Treg marker proteins CD25 (from Sino Biological) and Foxp3 (from Abcam), CD3+ antibody, and CD8+ antibody. Then, the data were read with CytoFLEX flow cytometer and analyzed with FlowJo software.

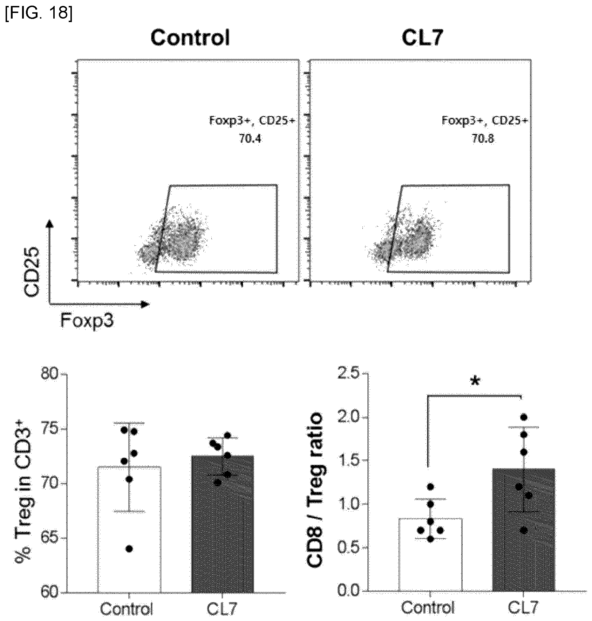

As a result, as shown in FIG. 18, it was identified that as for the treatment with the anti-CD300C monoclonal antibody alone, CD8+ T cells increased compared with Treg T cells. This means that an increased number of CD8+ T cells due to the administration of the anti-CD300C monoclonal antibody further inhibited cancer growth.

### Experimental Example 3.7. Identification of Effects on Cytotoxic T Cells, Regulatory T Cells, and Tumor-Associated Macrophages

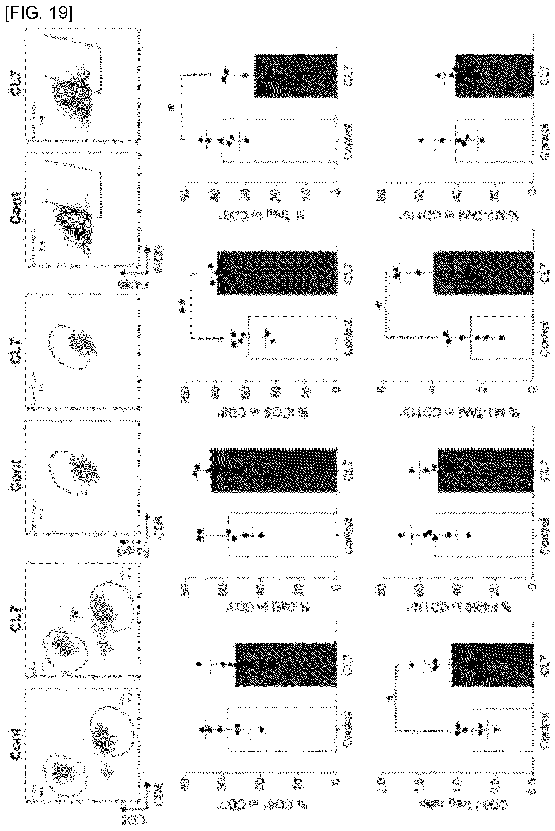

In order to identify effects of the anti-CD300c monoclonal antibody CL7 on cytotoxic T cells, regulatory T cells, and tumor-associated macrophages, the following experiment was performed. Syngeneic mouse tumor models were prepared as in Experimental Example 3.2. In addition, 12 days after transplantation of the colon cancer cell line, the mice with a tumor size of 50 mm³ to 100 mm³ were administered the anti-CD300c monoclonal antibody, and for a control group, the mice were injected with the same amount of phosphate buffered saline (PBS). The mice were intraperitoneally injected with 25 mg/kg twice a week for two weeks over a total of 4 times. On day 25 after the injection, the mice were sacrificed, and the tumor tissue was collected from each of six mice in the CL7 25 mg/kg administration group showing a highest antitumor effect compared with the control group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability, and CD8+ antibody and CD4+ antibody, which are CD8+ T cell markers, Foxp3 antibody and CD4+ antibody, which are Treg cell markers, or antibodies (from Abcam) to the total macrophage marker F4/80 and the M1 macrophage marker iNOS. Then, the data were read with CytoFLEX flow cytometer and analyzed with FlowJo software. The results are shown in FIG. 19.

As shown in FIG. 19, it was identified that the anti-CD300c monoclonal antibody (CL7) significantly increased activated CD8+ T cells, inhibited regulatory T cells, and repolarized tumor-associated macrophages towards M1 phenotype.

### Experimental Example 3.8. Identification of Cancer Cell Growth inhibitory Effect in Mice

In order to identify whether the anti-CD300c monoclonal antibodies CL7, CL10, and SL18 exhibit an anticancer effect, CT26 (mouse colon cancer cell line) was dispensed into a 96-well plate at a concentration of 1×10⁴ cells/well and treated with 10 ug/mL of the monoclonal antibodies, followed by incubation for 5 days. Then, cell proliferation assay was performed through CCK-8 detection.

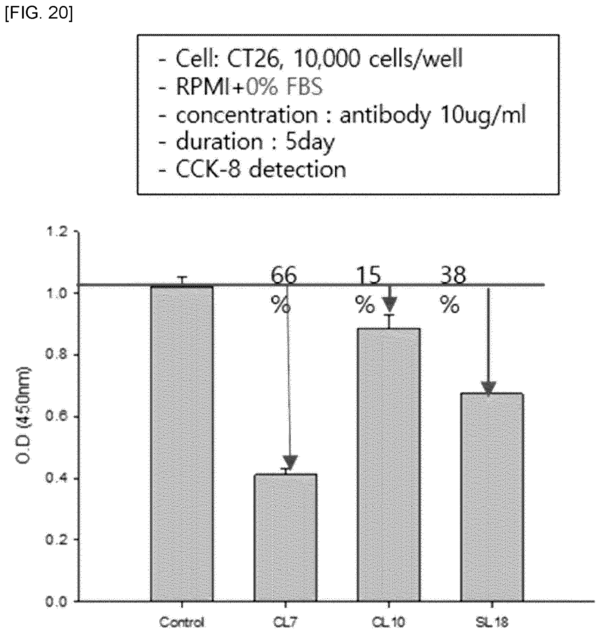

As shown in FIG. 20, it was identified that the anti-CD300c monoclonal antibodies exerted cancer cell proliferation inhibitory effects of 66% (CL7), 15% (CL10), and 38% (SL18), respectively, indicating that the anti-CD300c monoclonal antibodies exhibited a cancer therapeutic effect in mice.

Referring to Experimental Example 6.2 to be later described, it can be seen that the anti-CD300c monoclonal antibodies also exhibited an anticancer effect in humans and thus have cross-reactivity of acting on both of human and mouse CD300c.

### Experimental Example 3.9. Identification of In vivo Cancer Growth inhibitory Effects

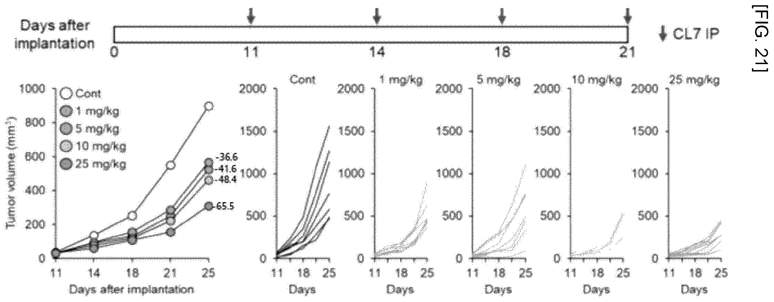

In order to identify anticancer effects of the anti-CD300c monoclonal antibody CL7 under *in vivo* conditions, a colon cancer cell line (CT26) at 2×10⁵ cells was transplanted by subcutaneous injection into 8-week-old BALB/c mice to prepare syngeneic mouse tumor models. Animal breeding and experiments were all conducted in a SPF facility. On day 11 (D11) after the transplantation of the colon cancer cell line, the mice with a tumor size of 50 mm³ to 100 mm³ were administered the anti-CD300c monoclonal antibody 1 mg/kg, 5 mg/kg, 10 mg/kg, or 25 mg/kg, respectively, and for a control group, the mice were administered the same amount of phosphate buffered saline (PBS). Specifically, the mice were intraperitoneally injected with each dosage twice a week for two weeks (a total of 4 times on D11, D14, D18, and D21). The tumor volume was measured for 25 days. The results are shown in FIG. 21.

As illustrated in FIG. 21, it was identified that the anti-CD300c monoclonal antibody CL7 delayed the growth of CT26 colon cancer in a dose-dependent manner.

### Experimental Example 4. Comparison of Overall Survival Periods of Various Cancer Patients According to Expression Level of CD300c

The comparison of the overall survival period according to the expression level of CD300c was made by using data on patients with kidney cancer (530 cases), pancreatic cancer (177 cases), and liver cancer (370 cases) obtained from the US TCGA (The Cancer Genome Atlas) database. First, the respective cancer patients were classified according to high and low CD300c expression levels. These high and low levels are classified by comparison with the average of CD300c expression levels for each cancer type. In the kidney cancer patients, 394 cases had low CD300c expression levels, and 136 cases had high CD300c expression levels. In the pancreatic cancer patients, 57 cases had low CD300c expression levels, and 120 cases had high CD300c expression levels. In the liver cancer patients, 192 cases had low CD300c expression levels, and 178 cases had high CD300c expression levels. The overall survival period according to the CD300c expression level for each cancer patient was analyzed using the Kaplan-Meier method. Then, the survival period was compared between patients with high and low CD300c expression levels was compared by using the log-rank test.

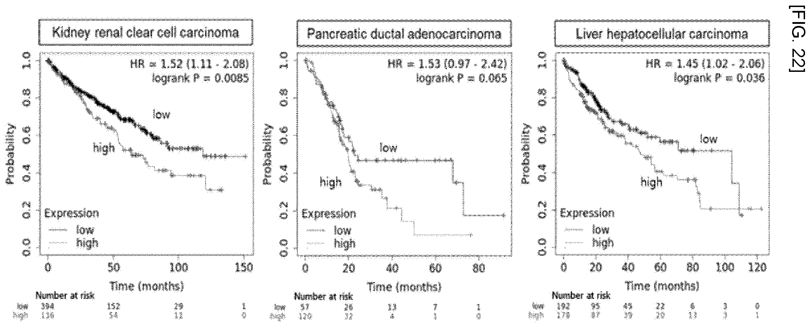

As a result, as shown in FIG. 22, it was identified that the patients with high CD300c expression levels had a shorter survival period than patients with low CD300c expression levels, indicating a significant result in consideration of P value. These results mean not only that the expression of CD300c was highly correlated with the survival period of cancer patients, but also that the inhibition of the expression or activity of CD300c could be expected to have a cancer therapeutic effect or a survival period increasing effect.

### II. Changes in Expression of Biomarkers by Administration of Anti-CD300c Monoclonal Antibody

### Example 2. Changes in Expression of Immune Cell-Related Markers and Tumor Microenvironment-Related Markers by Administration of Anti-CD300c Monoclonal Antibody

### Example 2.1. Nanostring Immune Profiling

In order to identify changes in expression of immune cell- and tumor microenvironment-related markers when solid cancer models are administered the anti-CD300c monoclonal antibody (CL7) produced in Example 1, a colon cancer cell line (CT26) at 2×10⁵ cells was transplanted by subcutaneous injection into 8-week-old BALB/c mice to prepare syngeneic mouse tumor models. Animal breeding and experiments were all conducted in a specific pathogen free (SPF) facility. In addition, 12 days after transplantation of the colon cancer cell line, the mice with a tumor size of 50 mm³ to 100 mm³ were separately administered the anti-CD300c monoclonal antibody, and for a control group, the mice were injected with the same amount of phosphate buffered saline (PBS). The mice were intraperitoneally injected with 25 mg/kg twice a week for two weeks over a total of 4 times. On day 25 after the injection, the mice were euthanized, and the tumor tissue was prepared. RNA was extracted and purified therefrom, and changes in dendritic cell markers, macrophage markers, tumor microenvironment (TME) markers, Th1 response markers, or Th2 response markers were identified through Nanostring Immune profiling.

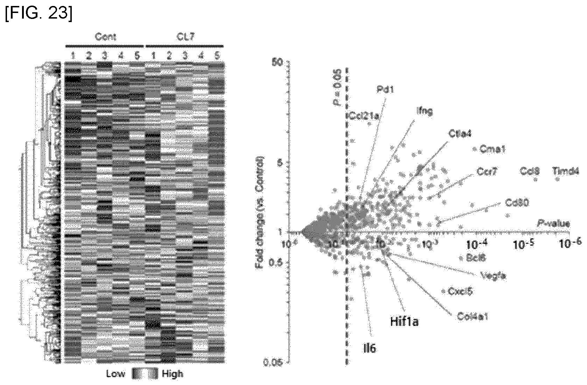

The Nanostring Immune profiling results are shown in FIG. 23. The results identified that the administration of the anti-CD300c monoclonal antibody extensively reprogrammed the tumor immune microenvironment.

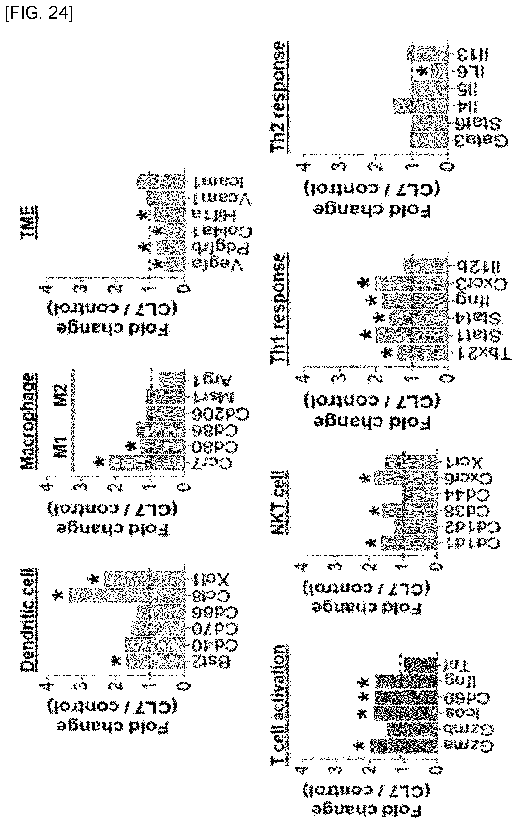

In addition, FIG. 24 illustrates the results obtained by observing, compared with the control group, changes in dendritic cell markers, macrophage markers, tumor microenvironment markers, Th1 response markers, or Th2 response markers by the administration of the anti-CD300c monoclonal antibody. As shown in FIG. 24, it was identified that the administration of the anti-CD300c monoclonal antibody led to a significantly increase in expression of Bst2, CCL8, and Xcl1, which are dendritic cell markers; a significantly increase in expression of CCR7 and CD80, which are M1 macrophage markers; a reduction in expression of vegfa, pdgfrb, Col4a1, and Hif1a, which help cancer growth in the tumor microenvironment; and an increase in expression of Tbx21, Stat1, Stat4, Ifn-g, and Cxcr3, which are markers that can identify the Th1 response.

### Example 2.2. Changes in Expression of Immune Checkpoint Markers

On the basis of the Nanostring Immune profiling results obtained in Example 2.1, it was identified which immune checkpoint markers show a significant difference in expression compared with the control group when syngeneic mouse tumor models are administered the anti-CD300c monoclonal antibody.

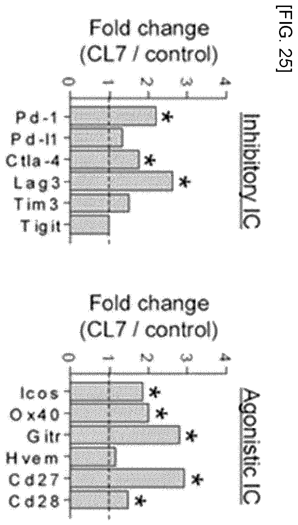

The results are shown in FIG. 25. it was identified that the administration of the anti-CD300c monoclonal antibody led to increases in expression of PD-1, CTLA-4, and Lag3, which are inhibitory immune checkpoints (ICs), and increases in expression of ICOS, OX40, Gitr, Cd27, and Cd28, which are agonistic ICs.

These results are of considerable significance in that the results could provide useful information about which immune checkpoint-related immunotherapeutic agent needed to be selected when the anti-CD300c monoclonal antibody and an additional immunotherapeutic agent were administered in combination to obtain further enhanced anticancer efficacy.

### III. Combined Use of Anti-CD300c Monoclonal Antibody and Immunotherapeutic Agent

### Example 3. Combined administration of Anti-CD300c Monoclonal Antibody (CL7) and Immunotherapeutic Agent

The anti-CD300c monoclonal antibody (CL7) produced in Example 1 was used in combination with another immunotherapeutic agent, for example, the anti-PD-L1 antibodies Imfinzi^{®} and Opdivo^{®}, and the anti-PD-1 antibody Keytruda, an anti-CD47 antibody (αCD47), or an anti-CTLA-4 antibody, and the results were obtained.

The respective immunotherapeutic agents were accessible from: Imfinzi (AstraZeneca); Opdivo and the anti-CTLA-4 antibody (Bristol Myers Squibb Company); Keytruda (Merck Sharp & Dohme); and the anti-CD47 antibody (Abcam).

### Experimental Example 5. Identification of Synergistic Increase in Macrophage Activity by Combined Use

### Experimental Example 5.1. Identification of Increase in M1 Macrophages

In order to identify the pattern of differentiation into M1 macrophages through cell morphology when monocytes are treated with the anti-CD300c monoclonal antibody CL7 produced in Example 1 and an immunotherapeutic agent, such as Imfinzi as an anti-PD-L1 antibody, Keytruda as an anti-PD-1 antibody, and an anti-CD47 antibody (αCD47) alone or in combination, THP-1 (human monocyte cell line) was treated with the anti-CD300c monoclonal antibody and the immunotherapeutic agent, respectively, at 10 µg/mL alone or in combination. The cells were incubated for 48 hours, and then the cell morphology was observed under a microscope.

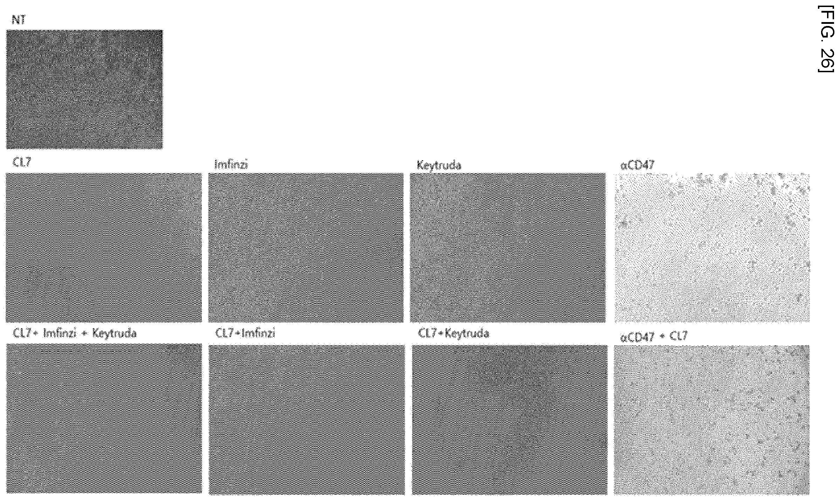

As a result, as shown in FIG. 26, it was identified that the morphology of THP-1 cells changed from suspension cells to circular adherent cells, which are in the form of M1 macrophages, in the combined treatment with the anti-CD300c monoclonal antibody and the immunotherapeutic agent compared with the treatment with the immunotherapeutic agent alone. These results identified that the differentiation of monocytes into M1 macrophages was further promoted by combined treatment with the anti-CD300c monoclonal antibody and the immunotherapeutic agent.

### Experimental Example 5.2. Identification of Increase in M1 Macrophage Markers

In order to identify whether the induction of differentiation of monocytes into M1 macrophages increases when the cells are treated with the anti-CD300c monoclonal antibody CL7 produced in Example 1 and an immunotherapeutic agent, such as Imfinzi as an anti-PD-L1 antibody, Opdivo as an anti-PD-1 antibody, Keytruda as an anti-PD-1 antibody, an anti-CD47 antibody (αCD47), and an anti-CTLA-4 antibody, alone in combination, THP-1 was dispensed into a 96-well plate at 1.5×10⁴ cells/well and treated with the anti-CD300c monoclonal antibody and the immunotherapeutic agent, respectively, at 10 µg/mL alone or in combination, followed by incubation for 48 hours, and the production levels of tumor necrosis factor-α (TNF-α), IL-1b, and IL-8, which are differentiation markers of M1 macrophages, were measured using an ELISA kit (Human TNF-α Quantikine kit, R&D Systems).

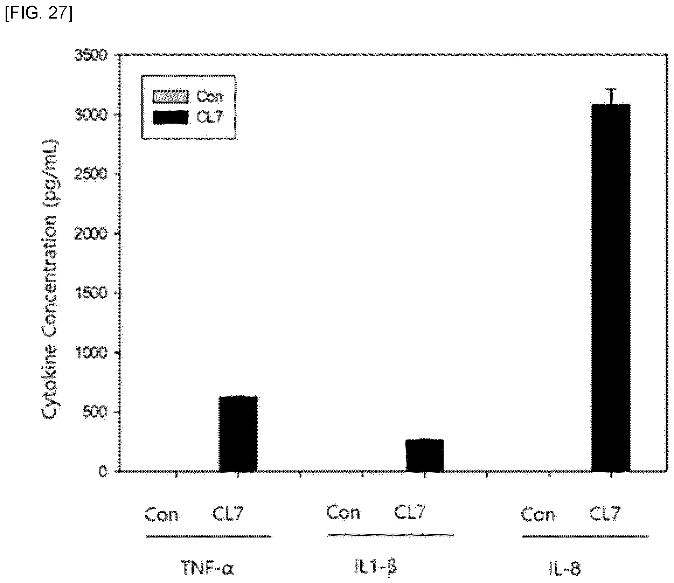

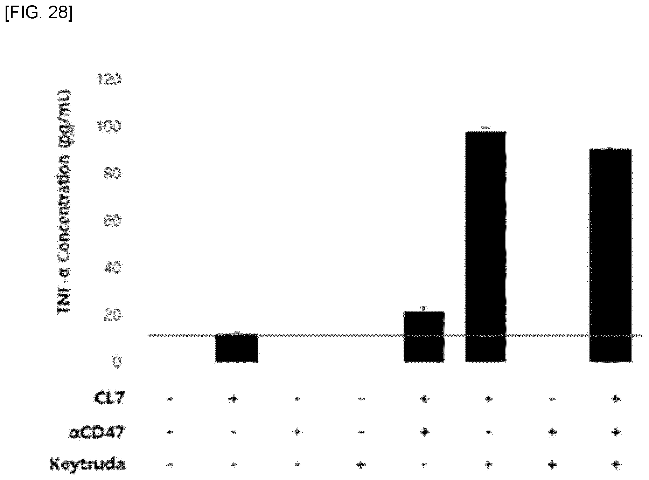

As a result, as shown in FIG. 27, it was identified that the production levels of all three differentiation markers increased when the cells were treated with the anti-CD300c monoclonal antibody alone, and especially, the production level of IL-8 significantly increased. As shown in FIG. 28, it was identified that the production levels of TNF-α, which is a differentiation marker of M1 macrophages, further increased when the cells were treated with the anti-CD300c monoclonal antibody in combination with at least one of Imfinzi, Opdivo, Keytruda, and Acd47, compared with when the cells were treated with the anti-CD300c monoclonal antibody alone. These results identified that more monocytes were differentiated into M1 macrophages when the cells were treated with the anti-CD300c monoclonal antibody and the anti-PD-1 antibody and/or the anti-CD47 antibody than when the cells were treated with the anti-CD300c monoclonal antibody alone.

### Experimental Example 5.3. Identification of Decrease in M2 Macrophage Markers

In order to identify whether the induction of differentiation of monocytes into M2 macrophages decreases when the cells are treated with the anti-CD300c monoclonal antibody and an immunotherapeutic agent, such as Imfinzi, Opdivo, Keytruda, anti-CTLA-4, or αCD47, alone or in combination, MP-1 was dispensed into a 96-well plate at 1.5×10⁴ cells/well and pre-treated with 320 nM PMA for 6 hours. Then, the cells were treated with the anti-CD300c monoclonal antibody and the immunotherapeutic agent at 10 µg/mL alone or in combination, together with interleukin-4 (IL-4) and interleukin-13 (IL-13) at 20 ng/mL, followed by incubation for 48 hours. Then, the production levels of IL-10 and IL-12, which are differentiation markers of M2 macrophages, were measured using an ELISA kit (R&D Systems).

As a result, it was identified that the production levels of IL-10 and IL-12 further decreased by 30% or more in the combined treatment with the anti-CD300c monoclonal antibody and at least one of Imfinzi, Opdivo, Keytruda, and αCD47 compared with the treatment with the anti-CD300c monoclonal antibody alone.

### Experimental Example 5.4. Identification of Increase in Differentiation Ability into M1 Macrophages

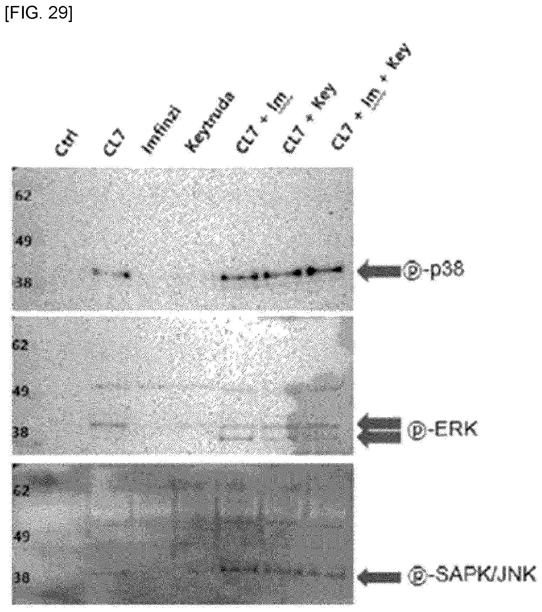

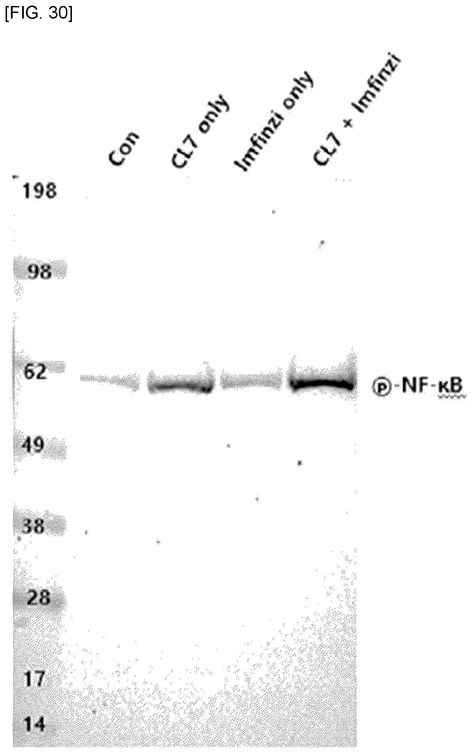

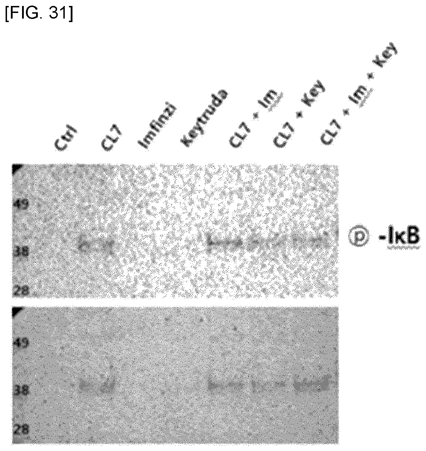

In order to identify whether the differentiation of monocytes into M1 macrophages increases when the cells are treated with the anti-CD300c monoclonal antibody CL7 in combination with an immunotherapeutic agent, such as an anti-PD-1 antibody, an anti-PD-L1 antibody, an anti-CTLA-4 antibody, and an anti-CD47 antibody, the signaling of mitogen-activated protein kinase (MAPK), IκB, and NF-kB, which are representative signals of M1 macrophage differentiation, was checked. Specifically, THP-1 was dispensed into a 6-well plate at 8.8×10⁵ cells/well and treated with 10 µg/mL of the anti-CD300c monoclonal antibody, 10 µg/mL of Imfinzi, and/or 10 µg/mL of Keytruda. For a control group, the cells were treated with the same amount of phosphate buffer solution (PBS). After the cells were incubated for 48 hours, Western blotting was performed to identify phosphorylated SAPK/JNK, phosphorylated ERK, phosphorylated p38 for MAPK signals, phosphorylated NF-kB for NF-kB signals, and phosphorylated IkB for IkB signals. The results are shown in FIGS. 29 to 31.

FIGS. 29, 30, and 31 illustrate the results obtained by identifying the signaling of MAPK, NF-Kb, and IkB, respectively. It was identified that the levels of phosphorylated MAPK, IkB, and NF-kB increased when THP-1 was treated with the anti-CD300c monoclonal antibody in combination with an immunotherapeutic agent, such as an anti-PD-1 antibody, an anti-PD-L1 antibody, an anti-CTLA-4 antibody, and an anti-CD47 antibody compared with when THP-1 was treated with the anti-CD300c monoclonal antibody alone. These results identified that cell signaling showing the differentiation into M1 macrophages increased when THP-1 was treated with the anti-CD300c monoclonal antibody in combination with an immunotherapeutic agent such as an anti-PD-1 antibody, an anti-PD-L1 antibody, an anti-CTLA-4 antibody, and an anti-CD47 antibody compared with when THP-1 was treated with the anti-CD300c monoclonal antibody alone.

### Experimental Example 6. Identification of (Synergistic) Increase in Cancer Cell Inhibitory Effect by Combined Use (In vitro)

### Experimental Example 6.1. Identification of Apoptosis Signals

It was identified whether apoptosis signals increase by combined treatment with the anti-CD300c monoclonal antibody CL7 and an immunotherapeutic agent, such as an anti-PD-1 antibody, an anti-PD-L1 antibody, an anti-CTLA-4 antibody, or an anti-CD47 antibody. Specifically, A549 was dispensed at 8×10⁵ cells/well into a 6-well plate, and treated with 10 µg/mL of the anti-CD300c monoclonal antibody, and 10 µg/mL of Imfinzi, Keytruda, Opdivo, or an anti-CD47 antibody alone or in combination. After the cells were incubated for 48 hours, apoptosis signals or cell cycle signals were identified by Western blotting. Cleaved caspase-9, caspase-3, caspase-2, and caspase-8 were identified as markers for the apoptosis signals, and cyclin D1, CDK2, p27kip1, CDK6, cyclin D3, P21 Waf1, Cip1, and the like were identified as markers for the cell cycle signals.

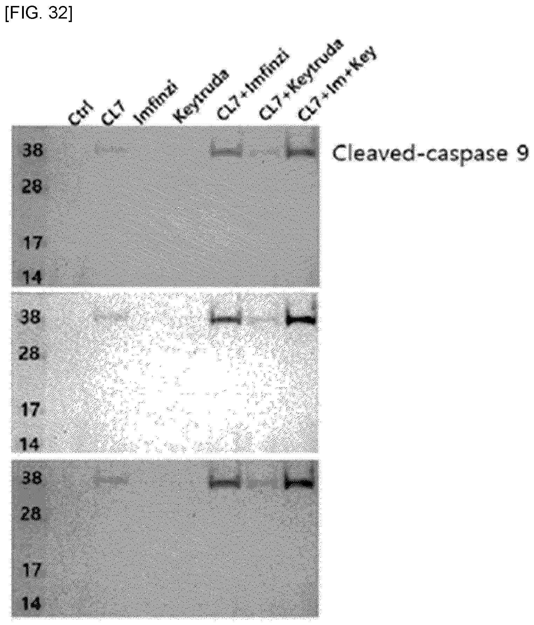

As shown in FIG. 32, the apoptosis signals increased in the combined treatment with the anti-CD300c monoclonal antibody and Imfinzi as an anti-PD-1 antibody compared with the treatment with the anti-CD300c monoclonal antibody alone; and the levels of cleaved-caspase9 and p21 increased and the level of cyclin D1 decreased in the combined treatment with the anti-CD300c monoclonal antibody and an immunotherapeutic agent, such as an anti-PD-1 antibody, an anti-PD-L1 antibody, an anti-CTLA-4 antibody, or an anti-CD47 antibody. These results identified that apoptosis of cancer cells was better induced in the combined treatment with the anti-CD300c monoclonal antibody and an immunotherapeutic agent, such as an anti-PD-1 antibody, an anti-PD-L1 antibody, an anti-CTLA-4 antibody, or an anti-CD47 antibody compared with the treatment with the anti-CD300c monoclonal antibody alone.

### Experimental Example 6.2. Identification of Cancer Cell Line Growth Inhibitory Effects

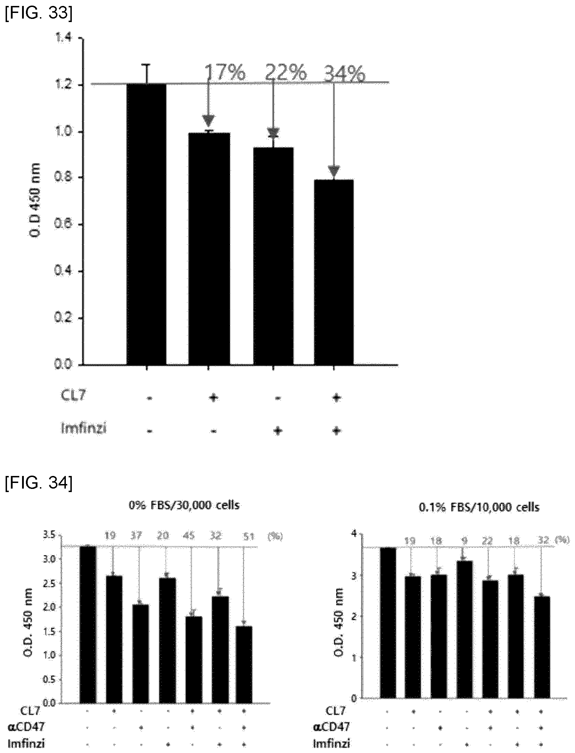

In order to identify cancer cell growth inhibitory effects by combined administration of the anti-CD300c monoclonal antibody CL7 and an immunotherapeutic agent, the cancer cell growth inhibitory effects were compared using A549 (human lung cancer cell line) and MDA-MB-231 (human breast cancer cell line). Specifically, the cells were dispensed into a 96-well plate at 2×10⁴ cells (A549) or 3×10⁴ cells (MDA-MB-231) under 0% fetal bovine serum (FBS) conditions, and at 6×10³ cells (A549) or 1×10⁴ cells (MDA-MB-231) under 0.1% fetal bovine serum conditions. Subsequently, the cells were treated with the anti-CD300c monoclonal antibody and Imfinzi at 10 µg/mL alone or in combination, followed by incubation for 5 days. For a control group, the cells were treated with the same amount of phosphate buffer solution (PBS). Then, the cells were treated with CCK-8 (DOJINDO), and the absorbance was measured at OD 450 nm. The results are shown in FIG. 33 (A549) and FIG. 34 (MDA-MB-231).

As shown in FIG. 33, it was identified that as for the A549 cell line, under 0% FBS conditions, the cell growth inhibitory effect was as high as 17% in the treatment with the anti-CD300c monoclonal antibody alone and as high as 34% in the combined treatment with the anti-CD300c monoclonal antibody and Imfinzi, compared with the control group.

As shown in FIG. 34, it was observed that as for the MDA-MB-231 cell line, under 0.1% FBS conditions, the cancer cell growth inhibitory effect was as high as 19% in the treatment with the anti-CD300c monoclonal antibody alone, as high as 45% in the combined treatment with the anti-CD300c monoclonal antibody and the anti-CD47 antibody, and as high as 51% in the combined treatment with the anti-CD300c monoclonal antibody, the anti-CD47 antibody, and Imfinzi together, compared with the control group. It was observed that under 0.1% FBS conditions, the cancer cell growth inhibitory effect was as high as 19% in the treatment with the anti-CD300c monoclonal antibody alone, as high as 22% in the combined treatment with the anti-CD300c monoclonal antibody and the anti-CD47 antibody, and as high as 32% in the combined treatment with the anti-CD300c monoclonal antibody, the anti-CD47 antibody, and Imfinzi together, compared with the control group.

These results identified that the growth of cancer cells was further inhibited in the combined treatment with the anti-CD300c monoclonal antibody and an immunotherapeutic agent, such as an anti-PD-1 antibody, an anti-PD-L1 antibody, an anti-CTLA-4 antibody, or an anti-CD47 antibody compared with the treatment with the anti-CD300c monoclonal antibody alone.

### Experimental Example 7. Identification of (Synergistic) Increase in In vivo Anticancer Effect by Combined Use (Colon Cancer Mouse Models)

### Experimental Example 7.1. Identification of In Vivo Cancer Growth inhibitory Effects

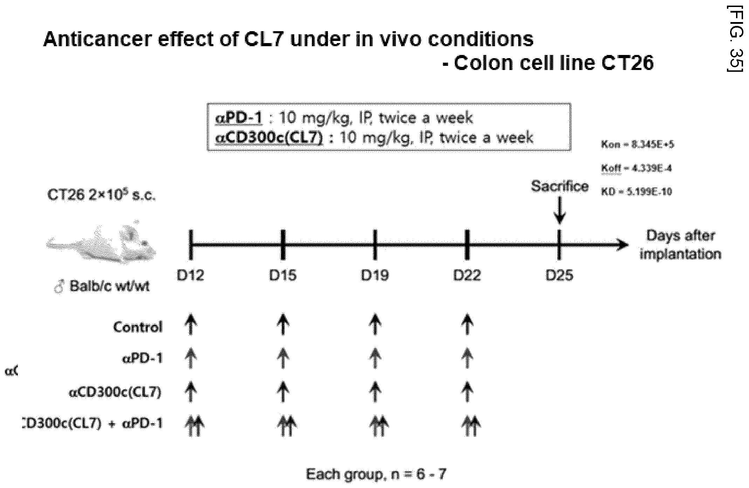

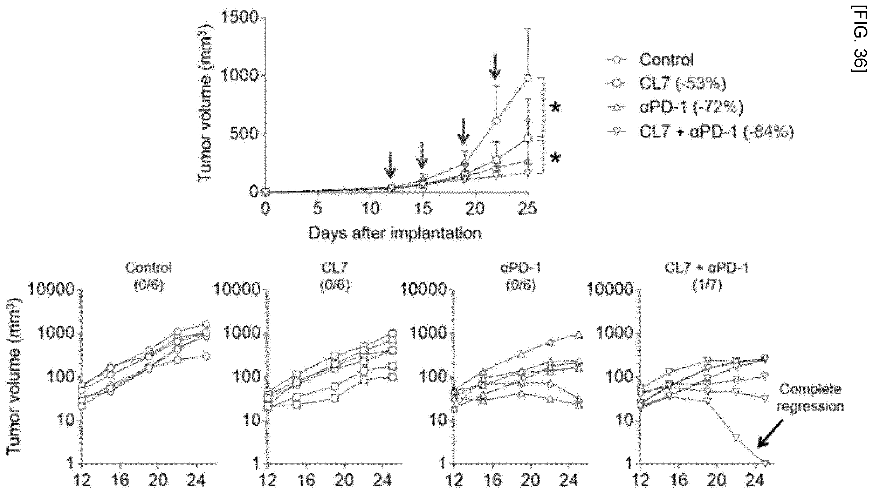

In order to identify anticancer effects of the anti-CD300c monoclonal antibody CL7 under *in vivo* conditions, a colon cancer cell line (CT26) at 2×10⁵ cells was transplanted by subcutaneous injection into 8-week-old BALB/c mice to prepare syngeneic mouse tumor models. Animal breeding and experiments were all conducted in a SPF facility. On day 12 (D12) after transplantation of the colon cancer cell line, the mice with a tumor size of 50 mm³ to 100 mm³ were administered the anti-CD300c monoclonal antibody and an anti-PD-1 antibody purchased from BioXcell alone or in combination, and for a control group, the mice were administered the same amount of phosphate buffered saline (PBS). A schematic experimental method is shown in FIG. 35. Specifically, the mice were intraperitoneally injected with the respective antibodies (CL7: 10 mg/kg; and anti-PD-1 antibody: 10 mg/kg) alone or in combination, twice a week for two weeks (a total of 4 times on D12, D15, D19, and D22). The tumor volume was measured for 25 days. The results are shown in FIG. 36.

As can be seen from FIG. 36, it was identified that although the cancer growth was inhibited compared with the control group even in the experimental group administered the anti-CD300c monoclonal antibody alone, the cancer growth was more effectively inhibited in the combined treatment with the anti-CD300c monoclonal antibody and an immunotherapeutic agent, such as an anti-PD-1 antibody compared with the treatment with the anti-CD300c monoclonal antibody alone.

### Experimental Example 7.2. Identification of Increase in Tumor-Infiltrating Lymphocytes Under In vivo Tumor Microenvironment

In order to identify effects of the anti-CD300c monoclonal antibody on tumor-infiltrating lymphocytes (TIL) in a tumor microenvironment (TME), on day 25 of an experiment conducted by the same method as in Experimental Example 7.1, the mice were euthanized, and injected intravascularly with 1% paraformaldehyde (PFA) to perform perfusion, and then the cancer tissue was obtained. The obtained cancer tissue was fixed using 1% PFA, and sequentially dehydrated using 10%, 20%, and 30% sucrose solutions. The dehydrated cancer tissue was frozen in OCT compound (optimal cutting temperature compound), and then the cancer tissue was sectioned to a thickness of 50 µm using a cryotome. The tissue was incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh for making single cells. To suppress non-specific reactions in the single cell suspension, the cells were incubated with a CD16/32 antibody (Invitrogen) for 1 hour, and then cell viability was identified, and CD8+ T cells and CD31+ cancer blood vessel cells, which are tumor-infiltrating lymphocyte markers, were stained.

As a result, it was identified that CD8+ T cells increased in the experimental group administered the anti-CD300c monoclonal antibody in combination with the anti-PD-1 antibody, the anti-PD-L1 antibody, the anti-CTLA-4 antibody, or the anti-CD47 antibody, compared with the experimental group administered the anti-CD300c monoclonal antibody alone. These results identified that the number of tumor-infiltrating lymphocytes increased in a tumor microenvironment to exert an anticancer effect in the combined treatment with the anti-CD300c monoclonal antibody and the anti-PD-1 antibody, the anti-PD-L1 antibody, the anti-CTLA-4 antibody, or the anti-CD47 antibody, compared with the treatment with the anti-CD300c monoclonal antibody alone.

### Experimental Example 7.3. Identification of Increase Effect in In vivo M1 Macrophages

In order to identify whether the anti-CD300c monoclonal antibody increases M1 macrophages in the cancer tissue of mouse models, the cancer tissue sections prepared by the same method as in Experimental Example 7.2 were stained with antibodies to the M1 macrophage marker iNOS and the M2 macrophage marker CD206, and analyzed by FACS.

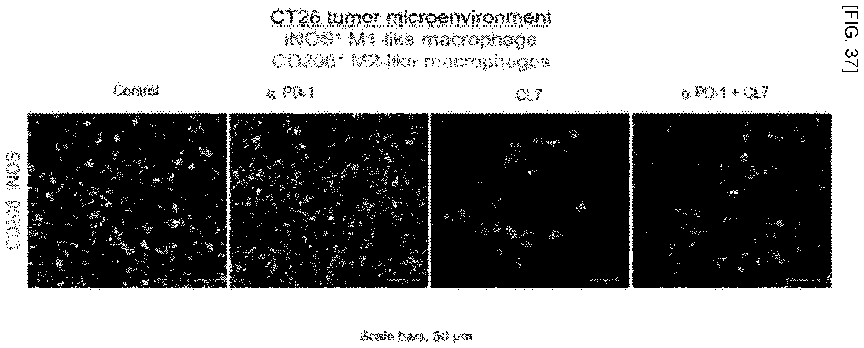

As a result, as shown in FIG. 37, it was identified that compared with the control group, M1 macrophages partially increased in the experimental group treated with the anti-PD-1 antibody, and M1 macrophages remarkably increased but M2 macrophages were hardly observed in the experimental group treated with the anti-CD300c monoclonal antibody. It was also identified that M1 macrophages further increased in the experimental group administered the anti-CD300c monoclonal antibody and the anti-PD-1 antibody in combination. These results identified that the differentiation into M1 macrophages could be effectively promoted by the combined treatment with the anti-CD300c monoclonal antibody and an immunotherapeutic agent, such as an anti-PD-1 antibody, an anti-PD-L1 antibody, an anti-CTLA-4 antibody, or an anti-CD47 antibody, compared with the treatment with the anti-CD300c monoclonal antibody alone.

### Experimental Example 7.4. Identification of In Vivo CD8+ T Cell Immunity Promoting Effect

In order to identify whether the anti-CD300c monoclonal antibody CL7 promotes CD8+ T cell immunity in mouse tumor models, on day 25 of the experiment conducted by the same method as in Experimental Example 7.1, the mice were euthanized, and injected intravascularly with 1% paraformaldehyde (PFA) to perform perfusion, and then the cancer tissue was obtained. The obtained cancer tissue was fixed using 1% PFA, and sequentially dehydrated using 10%, 20%, and 30% sucrose solutions. The dehydrated cancer tissue was frozen in OCT compound, and then the cancer tissue was sectioned to a thickness of 50 µm using a cryotome. Thereafter, the tissue was stained with CD8+ and Inos.

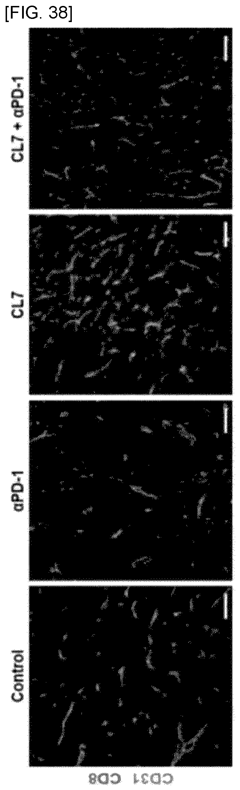

As shown in FIG. 38, it was identified that compared with the control group,

CD8+ T cells partially increased in the experimental group treated with the anti-PD-1 antibody, but CD8+ T cells remarkably increased in the experimental group treated with the anti-CD300c monoclonal antibody. It was also identified that compared with the group administered the anti-PD-1 antibody alone, CD8+ T cells further increased in the experimental group administered the anti-CD300c monoclonal antibody and the anti-PD-1 antibody in combination. These results identified that the anti-CD300c monoclonal antibody more effectively increased the number of CD8+ T cells in the combined use of the anti-CD300c monoclonal and a conventional immunotherapeutic agent.

### Experimental Example 7.5. Identification of Increase Effect in In Vivo Immune Cell Activity

In order to identify whether the combined administration of the anti-CD300c monoclonal antibody and an immunotherapeutic agent increases activity of immune cells, spleens were obtained by the same method as in Experimental Example 7.1 from mice administered the anti-CD300c monoclonal antibody in combination with an anti-PD-1 antibody, an anti-CTLA-4 antibody, an anti-KIR antibody, an anti-LAG3 antibody, an anti-CD137 antibody, an anti-OX40 antibody, an anti-CD276 antibody, an anti-CD27 antibody, an anti-GITR antibody, an anti-TIM3 antibody, an anti-41 BB antibody, an anti-CD226 antibody, an anti-CD40 antibody, an anti-CD70 antibody, an anti-ICOS antibody, an anti-CD40L antibody, an anti-BTLA antibody, an anti-TCR antibody, an anti-TIGIT antibody, or an anti-CD47 antibody. The obtained spleens were FACS-stained with various markers capable of detecting T cell activity and NKT cell activity as described in Experimental Example 7.2, and checked through MFI.

As a result, it was identified that the combined administration of the anti-CD300c monoclonal antibody and the above-mentioned immunotherapeutic agents increased Gzma, Icos, CD69, and Ifng, which are T cell activation markers, and significantly increased Cd11, CD38, and cxcr6, which are NKT cell activation markers. These results identified that T cells and NKT cells were further activated in the combined treatment with the anti-CD300c antibody and the immunotherapeutic agent, compared with the treatment with the anti-CD300c antibody alone.

### Experimental Example 7.6. Identification of In Vivo Treg Cells inhibitory Effect

In order to identify changes in pattern of Treg cells causing immune-suppressing responses when solid cancer models were administered the anti-CD300c monoclonal antibody in combination with an immunotherapeutic agent, such as an anti-PD-1 antibody, an anti-PD-L1 antibody, an anti-CTLA-4 antibody, an anti-KIR antibody, an anti-LAG3 antibody, an anti-CD137 antibody, an anti-OX40 antibody, an anti-CD276 antibody, an anti-CD27 antibody, an anti-GITR antibody, an anti-TIM3 antibody, an anti-41BB antibody, an anti-CD226 antibody, an anti-CD40 antibody, an anti-CD70 antibody, an anti-ICOS antibody, an anti-CD40L antibody, an anti-BTLA antibody, an anti-TCR antibody, an anti-TIGIT antibody, or an anti-CD47 antibody, T cells were extracted from the spleen tissue prepared by the same method as in Experimental Example 7.2, and FACS staining was performed to check the number of Treg cells, which express FOXP3, among CD3+ T cells.

Considering the proportion of Treg cells in CD3+ cells, it was identified that the proportion of Treg cells significantly decreased in the combined administration with the anti-CD300c monoclonal antibody and each of the immunotherapeutic agents compared with the administration with each of the immunotherapeutic agents alone, indicating that the activation of T cells attacking cancer cells was induced.

### Experimental Example 8. Identification of (Synergistic) Increase in In Vivo Anticancer Effect by Combined Use (Melanoma Mouse Models)

### Example 8.1. Identification of In Vivo Cancer Growth inhibitory Effect

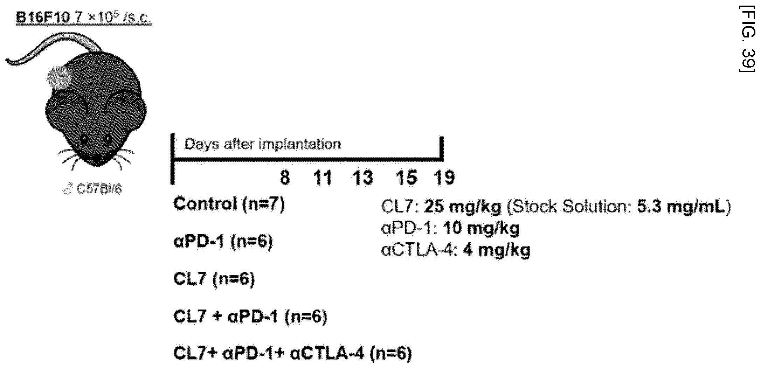

In order to identify whether the anti-CD300c monoclonal antibody CL7 is effective in other carcinomas in addition to a CT26 colon cancer mouse model, an additional experiment was conducted with melanoma mouse models. B16F10 melanoma cells at 7×10⁵ cells were transplanted by subcutaneous injection into 8-week-old male C57BL/6 mice to prepare syngeneic mouse tumor models. Animal breeding and experiments were all conducted in a SPF facility. On day 8 after transplantation of the melanoma cell line, the mice with a tumor size of 50 mm³ to 100 mm³ were intraperitoneally injected with 25 mg/kg of CL7, 10 mg/kg of α-PD-1, and 4 mg/kg of α-CTLA4. A schematic experimental method is shown in FIG. 39. More specifically, the mice were injected, twice a week for 2 weeks (over a total of 4 times), with each of the anti-CD300c monoclonal antibody, the anti-PD-1 antibody, the anti-CD300c monoclonal antibody+the anti-PD-1 antibody (Combo), and the anti-CD300c monoclonal antibody+the anti-PD-1 antibody+the anti-CTLA-4 antibody (Triple), and for a control group, the mice were injected with the same amount of phosphate buffered saline (PBS). The cancer size was measured for 20 days.

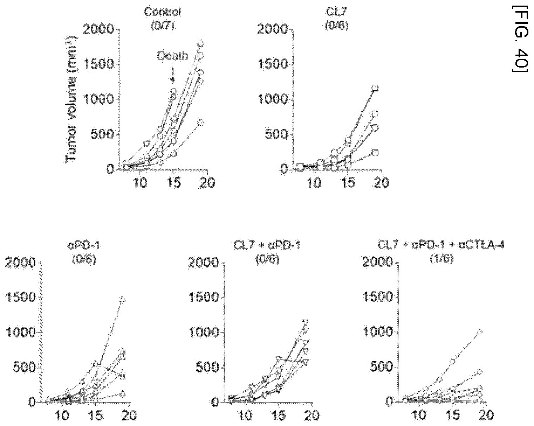

As a result, as shown in FIG. 40, it was identified that although the cancer growth was inhibited even by the administration of the anti-CD300c monoclonal antibody alone, the cancer growth was more effectively inhibited in the group administered the anti-CD300c monoclonal antibody and the anti-PD-1 antibody+the anti-CTLA-4 antibody in combination.

### Experimental Example 8.2. Identification of Increase in Cytotoxic T Cells

In order to identify *in vivo* effect, on CD8+ T cells, of the combined use of the anti-CD300c monoclonal antibody CL7 with an anti-PD-1 antibody and/or an anti-CTLA-4 antibody in a B16F10 melanoma model, mouse tumor models were prepared as in Experimental Example 8.1 and injected with each test substance at the same concentration.

Tumor tissues were collected from six mice in each group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability and CD8+ antibody and CD4+ antibody. Thereafter, the data were read with CytoFLEX flow cytometer and analyzed with FlowJo software.

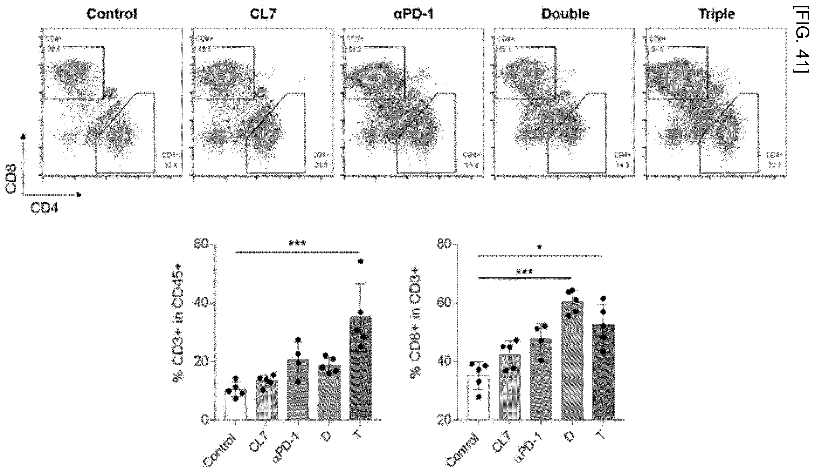

As a result, as shown in FIG. 41, it was identified that like in the CT26 carcinoma models (Experimental Example 3.3), the number of CD8+ T cells increased even in the B16F10 melanoma models by the combined administration of the anti-CD300c monoclonal antibody and the immunotherapeutic agent (groups D and T). Specifically, group D represents the combined use of CL7 and the anti-PD-1 antibody, and group T represents the combined use of CL7, the anti-PD-1 antibody, and the anti-CTLA-4 antibody.

### Experimental Example 8.3. Identification of Increase in Cytotoxic T Cells Compared with Regulatory T Cells

In order to identify *in vivo* effects, on regulatory T cells, of the combined use of the anti-CD300c monoclonal antibody CL7 with an anti-PD-1 antibody and/or an anti-CTLA-4 antibody in B16F10 melanoma models, mouse tumor models was prepared as in Experimental Example 8.1 and injected with each test substance at the same concentration. The experimental groups were as follows: (i) a group administered CL7, (ii) a group administered the anti-PD-1 antibody, (iii) a group administered CL7 and the anti-PD-1 antibody (group D), and (iv) a group administered CL7, the anti-PD-1 antibody, and the anti-CTLA4 antibody (group T).

The tumor tissue was collected from six mice in each group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability, antibodies to the Treg marker proteins CD25 and Foxp3, CD3+ antibody, and CD8+ antibody. Thereafter, the data were read with CytoFLEX flow cytometer and analyzed with FlowJo software.

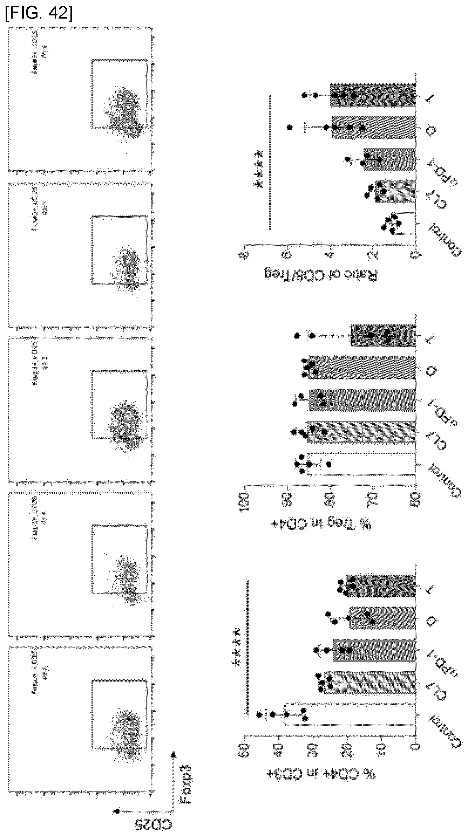

As a result, as shown in FIG. 42, it was identified that like in the CT26 carcinoma models (Experimental Example 4.6), the number of CD8+ T cells increased compared with the regulatory T cells even in the B16F10 melanoma model by the combined administration of the anti-CD300c monoclonal antibody and the immunotherapeutic agents (groups D and T). Specifically, group D represents the combined use of CL7 and an anti-PD-1 antibody, and group T represents the combined use of CL7, the anti-PD-1 antibody, and the anti-CTLA-4 antibody.

### Experimental Example 8.4. Identification of Increase in Tumor-Associated Macrophages (TAMs)

In order to identify *in vivo* effects, on macrophages, of the combined use of the anti-CD300c monoclonal antibody CL7 with an anti-PD-1 antibody and/or an anti-CTLA-4 antibody in B16F10 melanoma models, mouse tumor models were prepared as in Experimental Example 8.1 and injected with each test substance at the same concentration.

The tumor tissue was collected from six mice in each group. The tumor tissue was taken out and then incubated at 37°C for 1 hour in a mixed solution of collagenase D (20 mg/mL) and DNase I (2 mg/mL). Thereafter, the resultant product was filtered through a 70 µm cell strainer, followed by the lysis of red blood cells, and then again filtered through a nylon mesh. Subsequently, the single cell suspension was blocked with CD16/32 antibody (from Invitrogen), and the cells were stained with a staining solution for checking cell viability and antibodies to F4/80 and iNOS. Thereafter, the data were read with CytoFLEX flow cytometer and analyzed with FlowJo software.

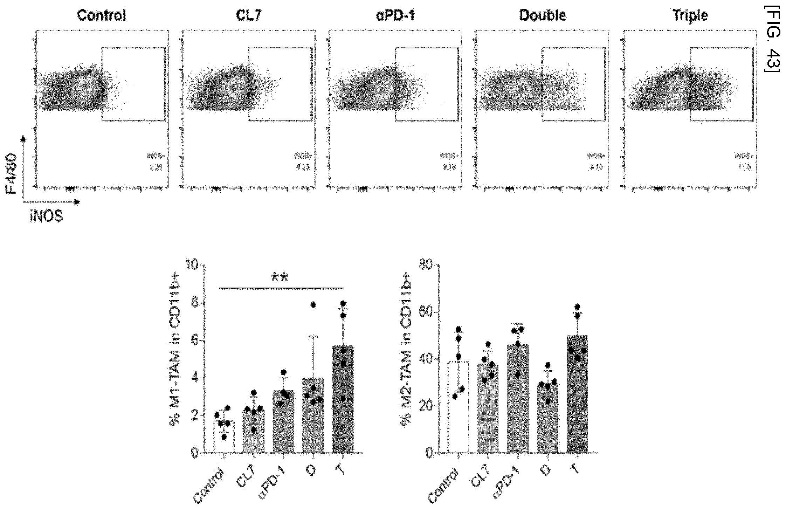

As a result, as shown in FIG. 43, it was identified that like in the CT26 carcinoma models (Experimental Example 3.2), the expression level of tumor-related M1-type macrophages increased even in the B16F10 melanoma models by the combined administration of the anti-CD300c monoclonal antibody and immunotherapeutic agents (groups D and T). Specifically, group D represents the combined use of CL7 and an anti-PD-1 antibody, and group T represents the combined use of CL7, the anti-PD-1 antibody, and the anti-CTLA-4 antibody.

Taken together, the results shown in FIGS. 41, 42, and 43 have the following meanings. the anti-CD300c monoclonal antibody CL7 treated cancer even in the B16F10 melanoma mouse models through the same mechanism as in the CT26 colon cancer mouse models (FIGS. 14, 15, and 18), and thus it is predictable that the combined administration of CL7 and an immunotherapeutic agent could exhibit the same effect in various carcinomas.

### Experimental Example 9. Identification of Complete Remission in Colorectal Cancer Mouse Models by Combined administration

In order to identify *in vivo* anticancer effects by the combined administration of the anti-CD300c monoclonal antibody CL7, and an anti-PD-1 antibody and/or an anti-CTLA-4 antibody, a colon cancer cell line (CT26) at 2×10⁵ cells was transplanted by subcutaneous injection into 8-week-old BALB/c mice to prepare a syngeneic mouse tumor model. Animal breeding and experiments were all conducted in a SPF facility. On day 11 (D11) after transplantation of the colon cancer cell line, the mice with a tumor size of 50 mm³ to 100 mm³ were administered the anti-CD300c monoclonal antibody, and an anti-PD-1 antibody and the anti-CTLA-4 antibody, each of which was purchased from BioXcell, alone or in combination, and for a control group, the mice were injected with the same amount of phosphate buffered saline (PBS). Specifically, on D11, D14 and D18, the mice were injected with each antibody alone or in combination by intraperitoneal injection (CL7: 25 mg/kg; anti-PD-1 antibody: 10 mg/kg; anti-CTLA-4 antibody: 4 mg/kg), and the tumor volume was measured.

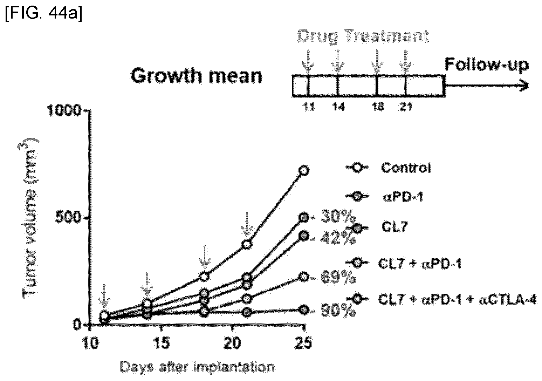

As a result, as shown in FIG. 44a, it was identified that compared with the control group, the growth of cancer was inhibited even in the experimental group administered the anti-CD300c monoclonal antibody alone, and the growth of cancer was further inhibited in the combined treatment with the anti-CD300c monoclonal antibody and an immunotherapeutic agent, such as anti-PD-1 antibody or anti-CTLA-4 antibody compared with the treatment with the anti-CD300c monoclonal antibody alone. In particular, the triple combined administration of CL7+αPD-1+αCTLA-4 decreased the tumor size by 90%.

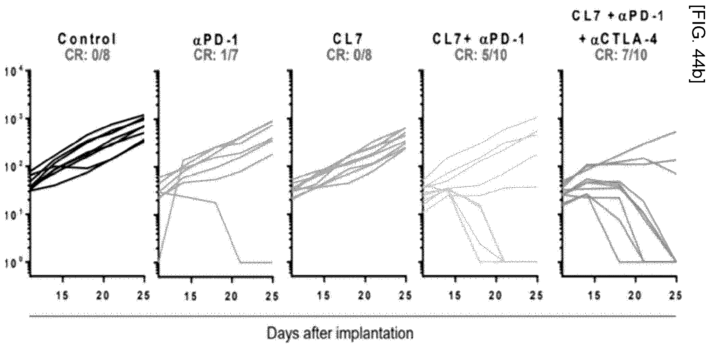

In addition, as shown in FIG. 44b, a complete remission (CR) of 50% was achieved in the combined administration (the anti-CD300c monoclonal antibody and the anti-PD-1 antibody), and a complete remission (CR) of 70% was achieved in the triple combined administration (the anti-CD300c monoclonal antibody+the anti-PD-1 antibody+the anti-CTLA-4 antibody), indicating that the combined administration resulted in an excellent anticancer effect.

### Experimental Example 10. Identification of Improvement in Long-Term Survival Rate by Combined administration

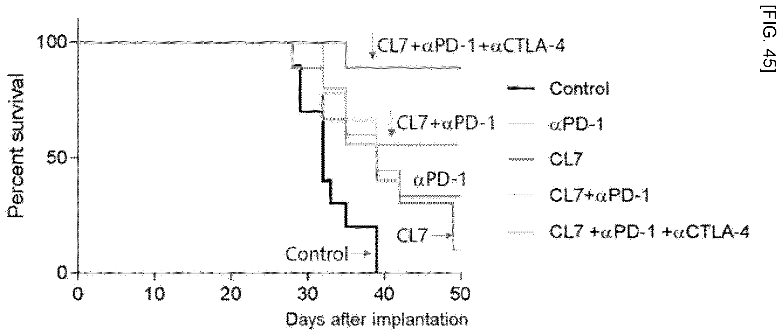

The long-term survival rate was identified on the mice tested in Experimental Example 9. As a result, as shown in FIG. 45, it was identified that the long-term survival rate also increased in the combined treatment with the anti-CD300c monoclonal antibody and an immunotherapeutic agent, such as anti-PD-1 antibody or anti-CTLA-4 antibody, compared with the treatment with the anti-CD300c monoclonal antibody alone.

### Experimental Example 11. Identification of Cancer Recurrence Preventing Effect by Combined administration

In order to identify the *in vivo* cancer recurrence prevention effect by the combined administration of the anti-CD300c monoclonal antibody CL7 and an immunotherapeutic agent, a colon cancer cell line (CT26) at 2×10⁵ cells was transplanted by subcutaneous injection into 8-week-old BALB/c mice to prepare syngeneic mouse tumor models, and then an experiment was conducted as described in Experimental Example 9 to obtain mice that have undergone complete remission. The thus obtained mice were transplanted again with a colon cancer cell line (CT26) at 2×10⁵ cells, and observed for 30 days.

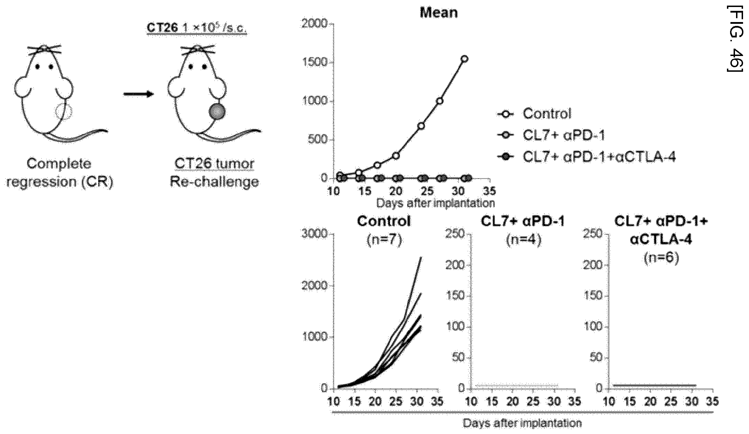

As a result, as shown in FIG. 46, it was identified that cancer recurrence or metastasis did not occur in the group in which complete remission was achieved by the combined administration of the anti-CD300c monoclonal antibody and the immunotherapeutic agent. An individual having undergone complete remission by the combined administration of the anti-CD300c monoclonal antibody and the anti-PD-1 antibody (CL7+αPD-1; Combi) or the triple combined administration of the anti-CD300c monoclonal antibody, the anti-PD-1 antibody, and the anti-CTLA-4 antibody (CL7+αPD-1+αCTLA-4; Triple) had a systemic protective immune response through continuous immune memory, so that the suppression of recurrence or metastasis of cancer was predicted in the individual.

### Experimental Example 12. Identification of Immune Memory Effect by Combined administration

In order to analyze effector memory T cells in the mice having undergone complete remission in Experimental Example 11, the mice were sacrificed and spleens were collected therefrom. Then, splenocytes were obtained therefrom and stained with antibodies (from Invitrogen) to CD44 and CD62L, which are markers related to T cell activity. Thereafter, data were read with CytoFLEX flow cytometer and analyzed with FlowJo software.

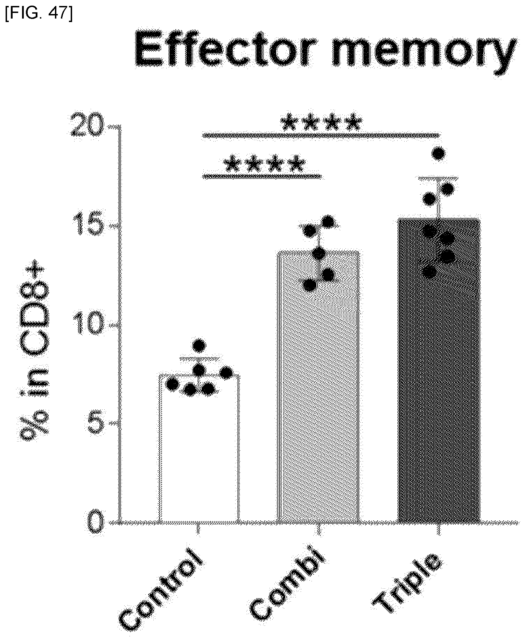

As a result, as shown in FIG. 47, it was identified that the effector memory T cells significantly increased in the combined treatment with the anti-CD300c monoclonal antibody and the immunotherapeutic agent. This indicates that as predicted in Experimental Example 11, the mice having undergone complete remission had the immune memory through increased effector memory T cells by the combined administration (Combi) of CL7 and the anti-PD-1 antibody or triple combined administration (Triple) of CL7, the anti-PD-1 antibody, and the anti-CTLA-4 antibody, and thus can suppress the growth of additional cancer cells even through such cancer cells were formed.

### Example 4. Combined administration of Anti-CD300c Monoclonal Antibodies (CL10 and SL18) and Immunotherapeutic Agents

The anti-CD300c monoclonal antibodies (CL10 and SL18) prepared in Example 1 were used in combination with other immunotherapeutic agents, for example, Imfinzi as an anti-PD-L1 antibody and Keytruda as an anti-PD-1 antibody, and the results were observed.

The respective immunotherapeutic agents were accessible from the following: Imfinzi (AstraZeneca); and Keytruda (Merck Sharp & Dohme).

### Experimental Example 14. Identification of Increase in Differentiation Ability into M1 Macrophages

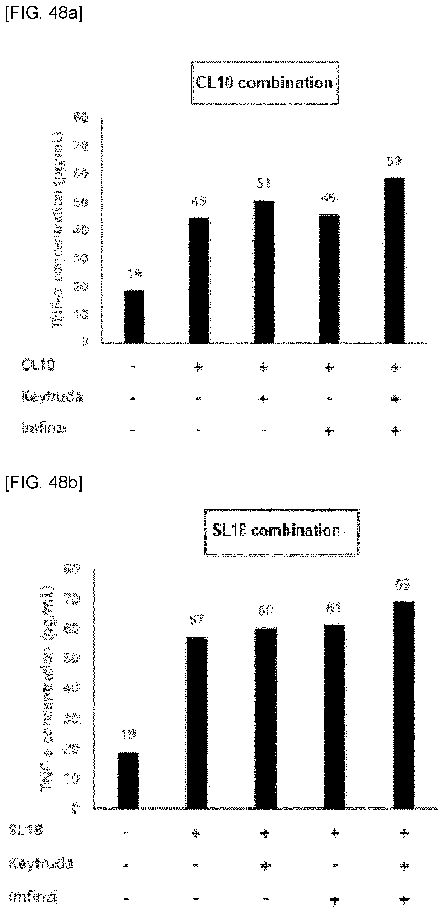

In order to identify whether anti-CD300c monoclonal antibody CL10 or SL18 can promote the differentiation from macrophages into M1 macrophages, THP-1 cells at 1×10⁴ cells were dispensed into a 96-well plate, and then treated with 10 µg/mL of CL10 or SL18. In addition, in order to identify effects according to the combined treatment with CL10 or SL18 and an immunotherapeutic agent, the THP-1 cells were treated with the anti-CD300c monoclonal antibody in combination with Imfinzi and/or Keytruda at 10 µg/mL. Subsequently, the cells were incubated in a CO₂ in incubator for 48 hours, and then the production level of TNF-α, which is a differentiation marker of M1 macrophages, was identified by an ELISA kit (Human TNF-α Quantikine kit, R&D Systems). The results are shown in FIG. 48a (CL10 combination) and FIG. 48b (SL18 combination).

As shown in FIGS. 48a and 48b, it was identified that the expression level of TNF-α increased when the THP-1 cells were treated with CL10 or SL18 in combination with Imfinzi or Keytruda compared with when the THP-1 cells were treated with CL10 or SL18 alone. In particular, it was identified that the expression level of TNF-α was highest in the combined administration of CL10 or SL18 in combination with both the two antibodies Imfinzi and Keytruda.

### Experimental Example 15. Identification of Cancer Cell Growth inhibitory Effect

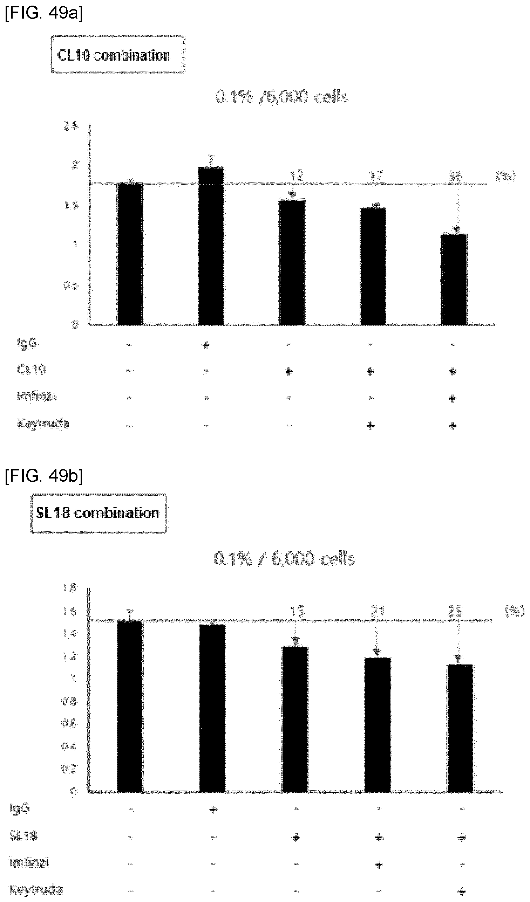

In order to identify the cancer cell growth inhibitory effect by the combined administration of the anti-CD300c monoclonal antibody CL10 or SL18 and an immunotherapeutic agent, the cell growth inhibitory effects were compared using A549 (human lung cancer cell line) cells. Specifically, the cells were dispensed into a 96-well plate at 2×10⁴ cells under 0% FBS conditions and at 6×10³ cells under 0.1% FBS conditions. Then, the cells were treated with CL10 or SL18 alone or treated with CL10 or SL18 in combination with Imfinzi and/or Keytruda at a concentration of 10 µg/mL for each, followed by incubation for 5 days. Subsequently, the cells were treated with CCK-8 (DOJINDO) at 30 µL/well, and the absorbance was measured at OD 450 nm every hour while the cells were incubated in a CO₂ incubator for 4 hours. The results are shown in FIG. 49a (CL10 combination) and FIG. 49b (SL18 combination).

As shown in FIGS. 49a and 49b, it was identified that under 0.1% FBS conditions, the cancer cell proliferation inhibitory effect further increased when the A549 cells were treated with CL10 or SL18 in combination with Imfinzi or Keytruda compared with when the A549 cells were treated with CL10 or SL18 alone, and the cancer cell proliferation inhibitory effect was highest when the A549 cells were treated with CL10 or SL18 in combination with both the two antibodies (Imfinzi and Keytruda).

As can be seen from Experimental Example 14 and this Experimental Example, the other anti-CD300c monoclonal antibodies produced in Example 1, including CL10 and SL18, also exhibited efficacy through the same mechanism of action as in CL7, and the efficacy of the anti-CD300c monoclonal antibodies was increased by combined administration with an immunotherapeutic agent, as in CL7.

### IV. Combined Use of Anti-CD300c Monoclonal Antibody and Chemotherapeutic Agents

### IV. Combined Use of Anti-CD300c Monoclonal Antibody and Chemotherapeutic Agents

### Example 5. Determination of Appropriate Concentration of Each Anticancer Agent for Combined Administration

### Example 5.1. Sorafenib

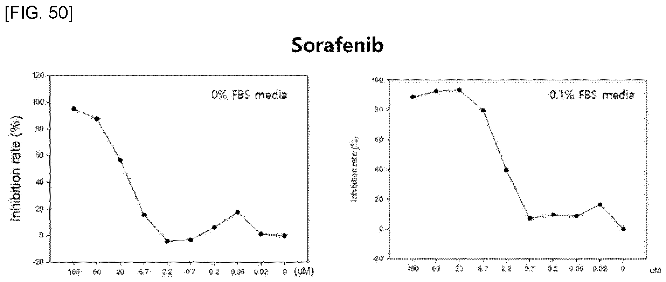

In order to examine whether the combined treatment of anti-CD300c monoclonal antibody and the chemotherapeutic agent sorafenib exhibits a cancer cell growth inhibitory effect, the treatment concentration of sorafenib was first selected. First, sorafenib was dissolved in dimethyl sulfoxide. A549 cells were inoculated into a 96-well plate at 20,000 cells per well under 0% FBS medium conditions and 0.1% FBS medium conditions, and then treated with sorafenib serially diluted to a concentration ranging from as high as 180 µM to as low as 0.02 µM, followed by incubation for 5 days. After the incubation, 10 µL of CCK-8 (DOJINDO) was added to each well, followed by incubation for 4 hours, and the absorbance was measured at 450 nm. The results are shown in FIG. 50.

As shown in FIG. 50, sorafenib showed a concentration-dependent cancer cell growth inhibitory effect, and the optimal treatment concentration for combined treatment of anti-CD300c and sorafenib was selected as 9 µM under 0% FBS medium conditions.

### Example 5.2. Gemcitabine

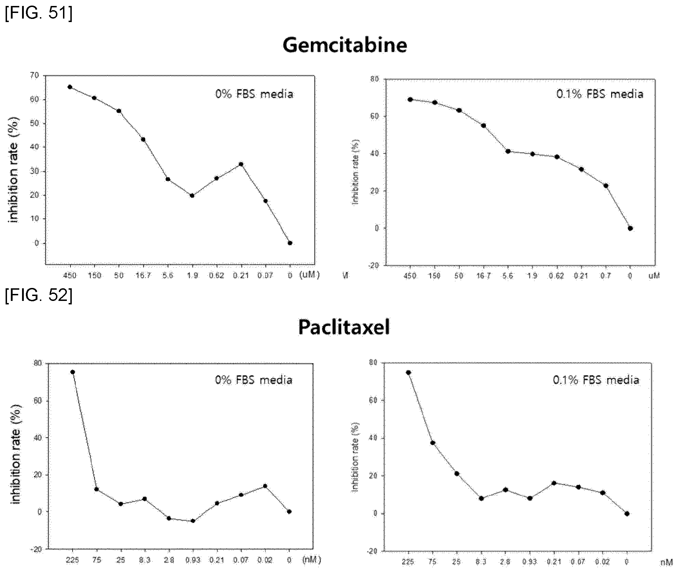

In order to examine whether the combined treatment of anti-CD300c monoclonal antibody and the chemotherapeutic agent gemcitabine exhibits a cancer cell growth inhibitory effect, the treatment concentration of gemcitabine was first selected. First, gemcitabine was dissolved in dimethyl sulfoxide. A549 cells were inoculated into a 96-well plate at 20,000 cells per well under 0% FBS medium conditions and 0.1% FBS medium conditions, and then treated with gemcitabine serially diluted to a concentration ranging from as high as 450 µM to as low as 0.07 µM, followed by incubation for 5 days. After the incubation, 10 µL of CCK-8 (DOJINDO) was added to each well, followed by incubation for 4 hours, and the absorbance was measured at 450 nm. The results are shown in FIG. 51.

As shown in FIG. 51, gemcitabine showed a concentration-dependent cancer cell growth inhibitory effect, and the optimal treatment concentration for combined treatment of anti-CD300c and gemcitabine was selected as 1.9 µM under 0% FBS medium conditions.

### Example 5.3. Paclitaxel

In order to examine whether the combined treatment of anti-CD300c monoclonal antibody and the chemotherapeutic agent paclitaxel exhibits a cancer cell growth inhibitory effect, the treatment concentration of paclitaxel was first selected. First, paclitaxel was dissolved in tertiary distilled water. A549 cells were inoculated into a 96-well plate at 20,000 cells per well under 0% FBS medium conditions and 0.1% FBS medium conditions, and then treated with paclitaxel serially diluted to a concentration ranging from as high as 225 nM to as low as 0.02 nM, followed by incubation for 5 days. After the incubation, 10 µL of CCK-8 (DOJINDO) was added to each well, followed by incubation for 4 hours, and the absorbance was measured at 450 nm. The results are shown in FIG. 52.

As shown in FIG. 52, paclitaxel showed a concentration-dependent cancer cell growth inhibitory effect, and the optimal treatment concentration for combined treatment of anti-CD300c and paclitaxel was selected as 5 nM under 0% FBS medium conditions.

### Experimental Example 16. Anticancer Effect by Combined Use of Anti-CD300c Monoclonal Antibodies and Chemotherapeutic Agent (In vitro)

### Experimental Example 16.1. Identification of Cancer Cell Growth Inhibitory Effect by Combined Treatment with Anti-CD300c Monoclonal Antibody and Sorafenib

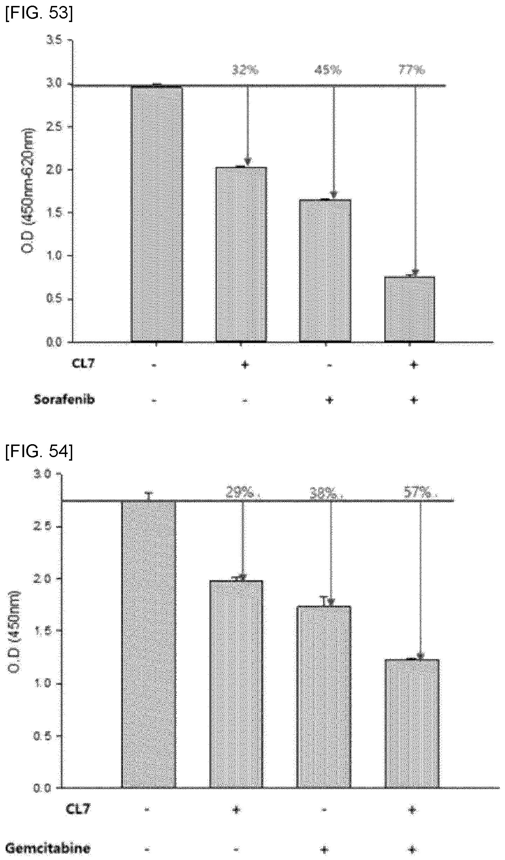

In order to identify the cancer cell growth inhibitory effect by combined treatment with an anti-CD300c monoclonal antibody and sorafenib, cell proliferation analysis was performed using A549 (human lung cancer cell line). Into a 96-well plate, 20,000 cancer cells were inoculated and treated with 10 ug/mL anti-CD300c monoclonal antibody and 9 µM sorafenib, followed by incubation for 5 days. After the incubation, 10 µL of CCK-8 (DOJINDO) was added to each well, followed by incubation for 4 hours, and the absorbance was measured at 450 nm. The results are shown in FIG. 53.

As shown in FIG. 53, the cancer cell growth inhibitory rate was 32% for the treatment with the anti-CD300c monoclonal antibody alone and 45% for the treatment with sorafenib alone, but the combined treatment showed a cancer cell growth inhibitory rate of 77%, higher than the previous two.

### Experimental Example 16.2. Identification of Cancer Cell Growth Inhibitory Effect by Combined Treatment with Anti-CD300c Monoclonal Antibody and Gemcitabine

In order to identify the cancer cell growth inhibitory effect by combined treatment with an anti-CD300c monoclonal antibody and gemcitabine, cell proliferation analysis was performed using A549 (human lung cancer cell line). The cells were treated with the anti-CD300c antibody in combination with 1.9 µM gemcitabine for 5 days, and then 10 µL of CCK-8 (DOJINDO) was added to each well, followed by incubation for 4 hours, and the absorbance was measured at 450 nm. The results are shown in FIG. 54.

As shown in FIG. 54, the cancer cell growth inhibitory rate was 29% for the treatment with the anti-CD300c monoclonal antibody alone and 38% for the treatment with gemcitabine alone, but the combined treatment showed a cancer cell growth inhibitory rate of 57%, higher than the previous two.

### Experimental Example 16.3. Identification of Cancer Cell Growth Inhibitory Effect by Combined Treatment with Anti-CD300c Monoclonal Antibody and Paclitaxel

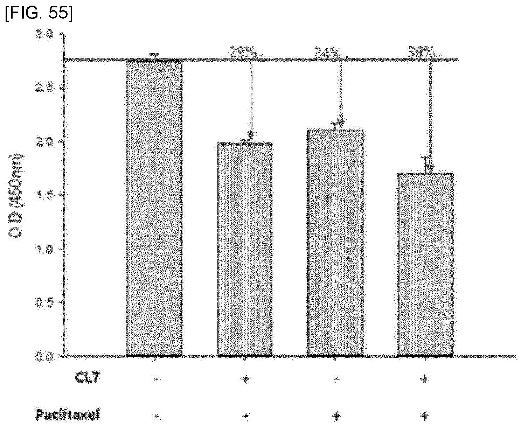

In order to identify the cancer cell growth inhibitory effect by combined treatment with an anti-CD300c monoclonal antibody and paclitaxel, cell proliferation analysis was performed using A549 (human lung cancer cell line). The cells were treated with the anti-CD300c monoclonal antibody in combination with paclitaxel and incubated for 5 days. Thereafter, 10 µL of CCK-8 (DOJINDO) was added to each well, followed by incubation for 4 hours, and the absorbance was measured at 450 nm. The results are shown in FIG. 55.

As shown in FIG. 55, the cancer cell growth inhibitory rate was 29% for the treatment with the anti-CD300c monoclonal antibody alone and 24% for the treatment with paclitaxel alone, but the combined treatment showed a cancer cell growth inhibitory rate of 39%, higher than the previous two.

### Experimental Example 17. Inhibition of Cancer Cell Apoptosis Resistance by Combined Treatment with Anti-CD300c Monoclonal Antibody and Chemotherapeutic Agent

### Experimental Example 17.1. Identification of Cancer Cell Apoptosis Resistance inhibitory Effect by Combined Treatment with Anti-CD300c Monoclonal Antibody and Sorafenib

In order to identify whether the combined treatment with an anti-CD300c monoclonal antibody and a chemotherapeutic agent can inhibit cancer cell apoptosis resistance, MKN45 (human gastric cancer cell line), IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line) were used. Each type of cancer cells were inoculated at 1×10⁴ into a 96-well plate and incubated for 16 hours. The incubated cells were treated with an anti-CD300c monoclonal antibody and a chemotherapeutic agent in combination for 8 hours, and then treated with rapamycin for 48 hours. The degree of survival of cancer cells was identified by fluorescence analysis using Annexin V and propidium iodide (PI).

As a result, compared with the hIgG1 isotype control group, the increase in MFI was 17% for the group treated with anti-CD300c monoclonal antibody alone and 21% for the group treated with sorafenib alone, but the combined treatment group showed 60%, indicating that the combined treatment significantly inhibited cancer cell apoptosis resistance in various cell lines with colon cancer, lung cancer, breast cancer, and the like.

### Experimental Example 17.2. Identification of Apoptosis resistance inhibitory ability by Combined Treatment with Anti-CD300c Monoclonal Antibody and Gemcitabine

In order to identify whether the combined treatment with an anti-CD300c monoclonal antibody and gemcitabine can inhibit cancer cell apoptosis resistance, MKN45 (human gastric cancer cell line), IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line) were used. Each type of cancer cells were inoculated at 1×10⁴ into a 96-well plate and incubated for 16 hours. The incubated cells were treated with an anti-CD300c monoclonal antibody and gemcitabine in combination for 8 hours, and then treated with rapamycin for 48 hours. The degree of survival of cancer cells was identified by fluorescence analysis using Annexin V and propidium iodide (PI). As a result, compared with the hIgG1 isotype control group, the increase in MFI was 14% for the group treated with anti-CD300c monoclonal antibody alone and 21% for the group treated with gemcitabine alone, but the combined treatment group showed 60%, indicating that the combined treatment significantly inhibited cancer cell apoptosis resistance in various cell lines with colon cancer, lung cancer, breast cancer, and the like.

### Experimental Example 17.3. Identification of Cancer Cell Apoptosis Resistance inhibitory Effect by Combined Treatment with Anti-CD300c Monoclonal Antibody and Paclitaxel

In order to identify whether the combined treatment with an anti-CD300c monoclonal antibody and paclitaxel can inhibit cancer cell apoptosis resistance, MKN45 (human gastric cancer cell line), IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line) were used. Each type of cancer cells were inoculated at 1×10⁴ into a 96-well plate and incubated for 16 hours. The incubated cells were treated with an anti-CD300c monoclonal antibody and paclitaxel in combination for 8 hours, and then treated with rapamycin for 48 hours. The degree of survival of cancer cells was identified by fluorescence analysis using Annexin V and propidium iodide (PI).

As a result, compared with the hIgG1 isotype control group, the increase in MFI was 16% for the group treated with anti-CD300c monoclonal antibody alone and 19% for the group treated with paclitaxel alone, but the combined treatment group showed 49%, indicating that the combined treatment significantly inhibited cancer cell apoptosis resistance in various cell lines with colon cancer, lung cancer, breast cancer, and the like.

### Experimental Example 18. Inhibition of Cancer Cell Migration/Infiltration by Combined Treatment with Anti-CD300c Monoclonal Antibody and Chemotherapeutic Agent

### Experimental Example 18.1. Inhibition of Cancer Cell Migration/Infiltration by Combined Treatment with Anti-CD300c Monoclonal Antibody and Sorafenib

In order to identify the cancer cell migration/infiltration inhibitory effect by the combined treatment with an anti-CD300c monoclonal antibody and sorafenib, MKN45 (human gastric cancer cell line), IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line) were used. Into a 6-well plate, 1×10⁵ cancer cells were inoculated and incubated for 16 hours, and then each well was wounded by scratching with a pipette tip. The detached cells were removed by washing with medium, and then treated with an anti-CD300c monoclonal antibody for 8 hours. Thereafter, observation was performed for 24 hours. On the basis of the area of the wound formed by scratching with the pipette tip, the migration distance of cancer cells was measured, and this distance was calculated as a percentage (%) to identify the degree of migration inhibition.

As a result, the group treated with anti-CD300c monoclonal antibody alone showed 27% and the group treated with sorafenib alone showed 32%, but the combined treatment group showed 67%, indicating that the combined treatment significantly inhibited cancer cell migration and infiltration in various cell lines with colon cancer, lung cancer, breast cancer, and the like.

### Experimental Example 18.2. Inhibition of Cancer Cell Migration/Infiltration by Combined Treatment with Anti-CD300c Monoclonal Antibody and Gemcitabine

In order to identify the cancer cell migration/invasion inhibitory effect by the combined treatment with an anti-CD300c monoclonal antibody and gemcitabine, MKN45, IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line) were used. Into a 6-well plate, 1×10⁵ cancer cells were inoculated and incubated for 16 hours, and then each well was wounded by scratching with a pipette tip. The detached cells were removed by washing with medium, and then treated with an anti-CD300c monoclonal antibody for 8 hours. Thereafter, observation was performed for 24 hours. On the basis of the area of the wound formed by scratching with the pipette tip, the migration distance of cancer cells was measured, and this distance was calculated as a percentage (%) to identify the degree of migration inhibition.

As a result, the group treated with anti-CD300c monoclonal antibody alone showed 25% and the group treated with gemcitabine alone showed 28%, but the combined treatment group showed 60%, indicating that the combined treatment significantly inhibited cancer cell migration and infiltration in various cell lines with colon cancer, lung cancer, breast cancer, and the like.

### Experimental Example 18.3. Inhibition of Cancer Cell Migration/Infiltration by Combined Treatment with Anti-CD300c Monoclonal Antibody and Paclitaxel

In order to identify the cancer cell migration/invasion inhibitory effect by the combined treatment with an anti-CD300c monoclonal antibody and paclitaxel, MKN45 (human gastric cancer cell line), IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line) were used. Into a 6-well plate, 1×10⁵ cancer cells were inoculated and incubated for 16 hours, and then each well was wounded by scratching with a pipette tip. The detached cells were removed by washing with medium, and then treated with an anti-CD300c monoclonal antibody for 8 hours. Thereafter, observation was performed for 24 hours. On the basis of the area of the wound formed by scratching with the pipette tip, the migration distance of cancer cells was measured, and this distance was calculated as a percentage (%) to identify the degree of migration inhibition.

As a result, the group treated with anti-CD300c monoclonal antibody alone showed 25% and the group treated with paclitaxel alone showed 20%, but the combined treatment group showed 57%, indicating that the combined treatment significantly inhibited cancer cell migration and infiltration in various cell lines with colon cancer, lung cancer, breast cancer, and the like.

### Experimental Example 19. Identification of Cancer Cell Growth Signaling inhibitory Effect by Combined Treatment with Anti-CD300c Monoclonal Antibody and Chemotherapeutic Agent

In order to identify the influence of the combined treatment with an anti-CD300c monoclonal antibody and each of the chemotherapeutic agents sorafenib, gemcitabine, and paclitaxel on the signaling mechanism that affects the growth of cancer cells, MKN45 (human gastric cancer cell line), IM95 (human gastric cancer cell line), HT-29 (human colon cancer cell line), A549 (human lung cancer cell line), HCT116 (human colon cancer cell line), MDA-MB-231 (human breast cancer cell line), and HepG2 (human liver cancer cell line) were used. Each cancer cell line was treated with an anti-CD300c monoclonal antibody and each of the chemotherapeutic agents in combination, and the phosphorylation of AKT and MAPKs, which is an activation mechanism of downstream signaling, was investigated.

The phosphorylation of AKT and MAPKs proteins was inhibited in cancer cells treated with the anti-CD300c monoclonal antibody and each of chemotherapeutic agents compared with cancer cells treated with the anti-CD300c monoclonal antibody or each therapeutic agent alone, indicating that the combined treatment with an anti-300c monoclonal antibody and each of sorafenib, gemcitabine, and paclitaxel can inhibit the growth of cancer cells by effectively inhibiting the growth signaling mechanism of cancer cells.

### Experimental Example 20. Identification of Anticancer Effect in Mice by Combined Treatment with Anti-CD300c Monoclonal Antibody and Chemotherapeutic Agent

In order to identify the anticancer effect under *in vivo* conditions by the combined treatment with an anti-CD300c monoclonal antibody and a chemotherapeutic agent, human solid tumor xenograft models were prepared by subcutaneous injection of 1×10⁶ CT26 cells (mouse colon cancer cell line) to humanized mouse models (huCD34-NSG mice). From 3 days after the administration of cancer cells, the mice were divided into a group administered 25 mg/kg anti-CD300c monoclonal antibody alone, groups administered the chemotherapeutic agents sorafenib, gemcitabine, and paclitaxel at 10 mg/kg for each, groups administered an anti-CD300c monoclonal antibody and chemotherapeutic agents, and a non-administered group. After the intraperitoneal administration with the anti-CD300c monoclonal antibody and each of the chemotherapeutic agents alone or in combination twice a week, a total of four times, the solid tumor tissue was isolated from the mice to measure the size thereof to identify the therapeutic effect of the anti-CD300c monoclonal antibody alone and the effects of the combined administration with existing chemotherapeutic agents.

As a result, the tumor inhibition rate was 26% for the group administered the anti-CD300c monoclonal antibody alone, 22% for sorafenib, 25% for gemcitabine, and 21% for paclitaxel in the groups administered the chemotherapeutic agents alone, but the combined administration groups showed high tumor inhibition rates of 50%, 52%, and 61%, respectively, indicating excellent cancer cell killing ability by the combined treatment. The p-value of the corresponding results was identified to be 0.05 or less through one-way ANOVA analysis, indicating that the reduction in tumor size in each of the combined treatment groups was significant compared with those in the single treatment groups.

It can be seen from the above results that the anti-CD300c monoclonal antibodies of the present disclosure exhibit advantageous effects when combined with each of the chemotherapeutic agents sorafenib, gemcitabine, and paclitaxel, and such a combined use can be applied to various individuals. In other words, it was identified both *in vivo* and *in vitro* that the combined treatment with an anti-CD300c monoclonal antibody and each chemotherapeutic agent can effectively inhibit the growth, proliferation, metastasis, and the like of cancer cells, indicating that when used in combination with existing chemotherapeutic agents, anti-CD300c monoclonal antibodies have further enhanced therapeutic effects and thus can be effectively used for immunotherapy.

### Statistical Processing

For the results obtained through the experiments, one-way ANOVA followed by Bonferroni's post-hoc test was used to perform comparative analysis between the experimental groups. The difference between the groups was significant when the p-value was 0.05 or lower.

## Claims

1. A pharmaceutical composition for the prevention or treatment of cancer, the pharmaceutical composition comprising, as active ingredient, an anti-CD300c (CD300 antigen-like family member C) antibody or antigen-binding fragment thereof and at least one additional anticancer agent.

2. The pharmaceutical composition of claim 1, wherein the additional anticancer agent includes an immunotherapeutic agent, a chemotherapeutic agent, or a combination thereof.

3. The pharmaceutical composition of claim 1, wherein the anti-CD300c antibody or antigen-binding fragment thereof comprises:
(i) a heavy chain variable region comprising: CDR1 comprising an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295;
CDR2 comprising an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and
CDR3 including an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region comprising: CDR1 comprising of an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298;
CDR2 comprising an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and
CDR3 comprising an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300.

4. The pharmaceutical composition of claim 1, wherein the anti-CD300c antibody or antigen-binding fragment thereof comprises:
(i) a heavy chain variable region comprising: CDR1 comprising an amino acid sequence selected from the group consisting of SEQ ID NO: 79, SEQ ID NO: 115, and SEQ ID NO: 211;
CDR2 comprising an amino acid sequence selected from the group consisting of SEQ ID NO: 80, SEQ ID NO: 116, and SEQ ID NO: 212; and
CDR3 comprising an amino acid sequence selected from the group consisting of SEQ ID NO: 81, SEQ ID NO: 117, and SEQ ID NO: 213; and
(ii) a light chain variable region comprising: CDR1 comprising an amino acid sequence selected from the group consisting of SEQ ID NO: 82, SEQ ID NO: 118, and SEQ ID NO: 214;
CDR2 comprising an amino acid sequence selected from the group consisting of SEQ ID NO: 83, SEQ ID NO: 119, and SEQ ID NO: 215; and
CDR3 comprising an amino acid sequence selected from the group consisting of SEQ ID NO: 84, SEQ ID NO: 120, and SEQ ID NO: 216.

5. The pharmaceutical composition of claim 1, wherein the anti-CD300c antibody or antigen-binding fragment thereof is selected from the group consisting of:
an antibody or antigen-binding fragment thereof comprising: a heavy chain variable region comprising CDR1 comprising the amino acid sequence as set forth in SEQ ID NO: 79, CDR2 comprising the amino acid sequence as set forth in SEQ ID NO: 80, and CDR3 comprising the amino acid sequence as set forth in SEQ ID NO: 81; and a light chain variable region comprising CDR1 comprising the amino acid sequence as set forth in SEQ ID NO: 82, CDR2 comprising the amino acid sequence as set forth in SEQ ID NO: 83, and CDR3 comprising the amino acid sequence as set forth in SEQ ID NO: 84;
an antibody or antigen-binding fragment thereof comprising: a heavy chain variable region comprising CDR1 comprising the amino acid sequence as set forth in SEQ ID NO: 115, CDR2 comprising the amino acid sequence as set forth in SEQ ID NO: 116, and CDR3 comprising the amino acid sequence as set forth in SEQ ID NO: 117; and a light chain variable region comprising CDR1 comprising the amino acid sequence as set forth in SEQ ID NO: 118, CDR2 comprising the amino acid sequence as set forth in SEQ ID NO: 119, and CDR3 comprising the amino acid sequence as set forth in SEQ ID NO: 120; and
an antibody or antigen-binding fragment thereof comprising: a heavy chain variable region comprising CDR1 comprising the amino acid sequence as set forth in SEQ ID NO: 211, CDR2 comprising the amino acid sequence as set forth in SEQ ID NO: 212, and CDR3 comprising the amino acid sequence as set forth in SEQ ID NO: 213; and a light chain variable region comprising CDR1 comprising the amino acid sequence as set forth in SEQ ID NO: 214, CDR2 comprising the amino acid sequence as set forth in SEQ ID NO: 215, and CDR3 comprising the amino acid sequence as set forth in SEQ ID NO: 216.

6. The pharmaceutical composition of claim 1, wherein the anti-CD300c antibody or antigen-binding fragment thereof comprises:
a heavy chain variable region comprising CDR1 comprising the amino acid sequence as set forth in SEQ ID NO: 79, CDR2 comprising the amino acid sequence as set forth in SEQ ID NO: 80, CDR3 comprising the amino acid sequence as set forth in SEQ ID NO: 81; and a light chain variable region comprising CDR1 comprising the amino acid sequence as set forth in SEQ ID NO: 82, CDR2 comprising the amino acid sequence as set forth in SEQ ID NO: 83, and CDR3 comprising the amino acid sequence as set forth in SEQ ID NO: 84.

7. The pharmaceutical composition of claim 1, wherein the anti-CD300c antibody or antigen-binding fragment thereof comprises a heavy chain variable region comprising the amino acid sequence of SEQ ID NO: 327 and a light chain variable region comprising the amino acid sequence of SEQ ID NO: 328.

8. The pharmaceutical composition of claim 1, wherein the anti-CD300c antibody or antigen-binding fragment thereof has inter-species cross-reactivity.

9. The pharmaceutical composition of claim 1, wherein the anti-CD300c antibody or antigen-binding fragment thereof comprises:
a heavy chain variable region comprising CDR1 to CDR3 comprising the amino acid sequences represented by Formulas (1) to (3), respectively; and
a light chain variable region comprising CDR1 to CDR3 comprising the amino acid sequences represented by Formulas (4) to (6), respectively:
FTFX1X2X3X4MX5WVR (1)
wherein,
X1= G or S
X2= S, R, or D
X3= N or Y
X4= Y, A, G, or H
X5= S or H
X1ISX2SGX3X4TYYAX5 (2)
wherein,
X1 = T or A
X2= G or S
X3= T or G
X4= S or Y
X5= D or E
YCAX1X2X3X4X5X6X7X8X9W (3)
wherein,
X1= R or S
X2= G or S
X3= M, S, Y, or I
X4= W, Q, G, or R
X5= G or L
X6= M, I, or P
X7= D, F, or L
X8= V or D
X9= I, Y, or not present
CX1X2X3X4X5X6X7X8X9X10X11VX12W (4)
wherein,
X1=TorS
X2= G or R
X3= K, N, or S
X4= H, N, or S
X5= R, I, or G
X6= H, G, or I
X7= T, I, or S
X8= R, A, K, or not present
X9= R, S, G, or not present
X10= N or not present
X11 = Y or not present
X12= N, H, or Q
X1X2X3X4RPSGVX5 (5)
wherein,
X1= L, S, R, or E
X2= D, K, or N
X3= S or N
X4= E, N, Q, or K
X5= P or R
YCX1X2X3X4X5X6X7X8X9X10VF (6)
wherein,
X1= Q, A, or S
X2= S or A
X3= Y or W
X4= D or A
X5= S, D, or G
X6= S, N, or T
X7= S, L, N, or K
X8= V, S, N, or G
X9= G, L, V, or not present
X10 = P or not present

10. The pharmaceutical composition of claim 2, wherein the immunotherapeutic agent include at least one selected from the group consisting of anti-PD-1, anti-PD-L1, anti-CTLA-4, anti-CD47, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT antibodies.

11. The pharmaceutical composition of claim 2, wherein the immunotherapeutic agent includes at least one selected from the group consisting of anti-PD-1, anti-PD-L1, anti-CTLA-4, and anti-CD47 antibodies.

12. The pharmaceutical composition of claim 2, wherein the immunotherapeutic agent includes at least one selected from the group consisting of durvalumab, pembrolizumab, nivolumab, αCD47, and ipilimumab.

13. The pharmaceutical composition of claim 2, wherein the chemotherapeutic agent includes at least one selected from the group consisting of a microtubule assembly inhibitor, a DNA intercalator or replication inhibitor, a multikinase inhibitor, and an angiogenesis inhibitor.

14. The pharmaceutical composition of claim 2, wherein the chemotherapeutic agent includes at least one selected from the group consisting of doxorubicin, paclitaxel, docetaxel, vinblastine, vincristine, vinorelbine, estramustine phosphate (EMP), nab-paclitaxel (Abraxane), cyclophosphamide, epirubicin, 5-fluorouracil, etoposide, ifosfamide, gemcitabine, deoxyadenosine, cladribine, clofarabine, fludarabine, pentostatin, deoxycytidine, cytosine arabinoside (ara-C), 5-aza-2'-deoxycitidine (decitabine), tezacitabine, capecitabine, cytarabine, methotrexate, pemetrexed, mercaptopurine, dactinomycin, daunorubicin, mitomycin, bleomycin, idarubicin, mitoxantrone HCL, mechlorethamine, melphalan, chlorambucil, thiotepa, altretamine, procarbazine, busulfan, streptozotocin, carmustine, lomustine, dacarbazine (DTI), chlorambucil, topotecan , irinotecan, temozolomide, cisplatin, carboplatin, oxaliplatin, sorafenib, regorafenib, batalanib, axitinib, masitinib, pazopanib, sunitinib, toceranib, cediranib, lenvatinib, nintedanib, semaxanib, tivozanib, vandetanib, and revlimid (lenalidomide).

15. The pharmaceutical composition of claim 2, wherein the chemotherapeutic agent includes at least one selected from the group consisting of sorafenib, gemcitabine, and paclitaxel.

16. The pharmaceutical composition of claim 1, wherein the cancer includes at least one selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer.

17. The pharmaceutical composition of claim 1, wherein the cancer is a solid cancer.

18. The pharmaceutical composition of claim 1, wherein the cancer includes at least one selected from the group consisting of colon cancer, lung cancer, melanoma, and breast cancer.

19. The pharmaceutical composition of claim 1, wherein the pharmaceutical composition inhibits the proliferation, survival, metastasis, recurrence, or cancer agent resistance of cancer.

20. The pharmaceutical composition of claim 1, wherein the anti-CD300c antibody or antigen-binding fragment thereof and the additional anticancer agent are each formulated and separately administered simultaneously or sequentially.

21. A method for preventing or treating cancer, the method comprising administering, to a subject in need of the prevention or treatment of cancer, an anti-CD300c (CD300 antigen-like family member C) antibody or antigen-binding fragment thereof and the at least one additional anticancer agent.

22. The method of claim 21, wherein the anti-CD300c antibody or antigen-binding fragment thereof and the at least one additional anticancer agent are administered simultaneously or sequentially.

23. The method of claim 21, further comprising identifying the expression level of a CD300c protein on the basis of a biological sample or data of the subject, before the administration of the anti-CD300c antibody or antigen-binding fragment thereof.

24. The method of claim 21, further comprising identifying the expression level of at least one marker selected from the following markers on the basis of a biological sample or data of the subject administered the anti-CD300c antibody or antigen-binding fragment thereof:
Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, PD-1, PD-L1, CTLA-4, Lag3, Tim3, Ox40, Gitr, Hvem, CD27, CD28, Cma1, Timd4, Bcl6, Cxcl5, and Ccl21a.

25. The method of claim 24, further comprising selecting the additional anticancer agent on the basis of the identified expression level of the marker.

26. The method of claim 24, further comprising identifying the therapeutic responsiveness of the anti-CD300c antibody or antigen-binding fragment thereof on the basis of the identified expression level of the marker.

27. The method of claim 25, wherein the marker includes at least one selected from the group consisting of PD-1, PD-L1, CTLA-4, Lag3, Tim3, Icos, Ox40, Gitr, Hvem, CD27, and CD28.

28. The method of claim 26, wherein the marker includes at least one selected from the group consisting of vegfa, pdgfrb, Col4a1, Hif1a, Bst2, CCL8, Xcl1, CCR7, CD80, Tbx21, Stat1, Stat4, Ifng, Cxcr3, IL-6, Gzma, Icos, Cd69, Cd1d1, Cd38, Cxcr6, Ox40, Gitr, CD27, and CD28.

29. The method of claim 28, further comprising determining that the therapeutic responsiveness of the anti-CD300c antibody or antigen-binding fragment thereof is good or excellent when the expression level of at least one marker selected from the group consisting of vegfa, pdgfrb, Col4a1, Hif1a, and IL-6 among the markers is statistically significantly reduced compared with a subject not having been administered the anti-CD300c antibody or antigen-binding fragment thereof.

30. The method of claim 28, further comprising determining that the therapeutic responsiveness of the anti-CD300c antibody or antigen-binding fragment thereof is good or excellent when the expression level of at least one marker selected from the group consisting of Icos, Ox40, Gitr, Hvem, CD27, CD28, Bst2, CCL8, Xcl1, CCR7, CD80, Tbx21, Stat1, Stat4, Ifng, Cxcr3, Gzma, Cd69, Cd1d1, Cd38, and Cxcr6 among the markers is statistically significantly increased compared with a subject not having been administered the anti-CD300c antibody or antigen-binding fragment thereof.

31. A kit for preventing or treating cancer, the kit comprising:
a composition comprising an anti-CD300c (CD300 antigen-like family member C) antibody or antigen-binding fragment thereof; and
instructions directing the combined use of the antibody or antigen-binding fragment thereof and at least one additional anticancer agent.

32. A method for providing information needed for the prediction of the therapeutic responsiveness of an anti-CD300c antibody or antigen-binding fragment thereof, the method comprising determining the expression level of a marker for predicting the therapeutic responsiveness by using a biological sample or data obtained from a subject,
wherein the marker includes at least one selected from the group consisting of Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, Pd-1, Pd-I1, Ctla-4, Lag3, Tim3, Ox40, Gitr, Hvem, Cd27, Cd28, Cma1, Timd4, Bcl6, Cxcl5, and CcI21a.

33. The method of claim 32, wherein the marker includes at least one selected from the group consisting of Vegfa, Pdgfrb, Col4a1, Hif1a, Bst2, Ccl8, Xcl1, Ccr7, Cd80, Tbx21, Stat1, Stat4, Ifng, Cxcr3, IL6, Gzma, Icos, Cd69, Cd1d1, Cd38, Cxcr6, Ox40, Gitr, Cd27, and Cd28.

34. The method of claim 32, further comprising determining that the therapeutic responsiveness of the anti-CD300c antibody or antigen-binding fragment thereof is good or excellent when the expression level of at least one marker selected from the group consisting of vegfa, pdgfrb, Col4a1, Hif1a, and IL-6 is statistically significantly reduced compared with a subject not having been administered the anti-CD300c antibody or antigen-binding fragment thereof.

35. The method of claim 32, further comprising determining that the therapeutic responsiveness of the anti-CD300c antibody or antigen-binding fragment thereof is good or excellent when the expression level of at least one marker selected from the group consisting of Bst2, CCL8, Xcl1, CCR7, CD80, Tbx21, Stat1, Stat4, Ifng, Cxcr3, Gzma, Icos, Cd69, Cd1d1, Cd38, Cxcr6, Ox40, Gitr, Cd27, and Cd28 is statistically significantly increased compared with a subject not having been administered the anti-CD300c antibody or antigen-binding fragment thereof.

36. A kit for predicting the therapeutic responsiveness of an anti-CD300c antibody or antigen-binding fragment thereof, the kit comprising a substance for measuring the expression level of a substance for predicting the therapeutic responsiveness of the anti-CD300c antibody or antigen-binding fragment thereof,
wherein the marker includes at least one selected from the group consisting of Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, PD-1, PD-L1, CTLA-4, Lag3, Tim3, Ox40, Gitr, Hvem, CD27, CD28, Cma1, Timd4, Bcl6, Cxcl5, and CcI21a.

37. A method for providing information for the prevention or treatment of cancer, the method comprising measuring the expression level of a CD300c protein by using a biological sample or data obtained from a subject in need of the prevention or treatment of cancer.

38. The method of claim 37, wherein the information for the prevention or treatment of cancer comprises information about at least one of the therapeutic responsiveness of a therapeutic agent (for example, an anti-CD300c antibody or antigen-binding fragment thereof) associated with the CD300c protein, the selection of a therapeutic agent, the selection of a subject to be treated, the prognosis of a subject, and the survival period of a subject.
